# EUROPEAN PATENT SPECIFICATION

(11) **EP 2 152 863 B1**
(45) Date of publication and mention of the grant of the patent: **19.09.2012**
(21) Application number: 08780603.0
(22) Date of filing: 08.05.2008
(51) Int. Cl.: C12N 7/04, C12N 15/86, A61K 39/155

(54) **HUMAN PARAINFLUENZA VIRUSES HAVING SEPARATED P AND C GENES**
MENSCHLICHE PARAINFLUENZAVIREN MIT GETRENNTEN P- UND C-GENEN
VIRUS PARAINFLUENZA HUMAIN AYANT DES GÈNES P ET C SÉPARÉS

(30) Priority: 08.05.2007 US 916799 P
(43) Date of publication of application: 17.02.2010
(73) Proprietor: GOVERNMENT OF THE UNITED STATES OF AMERICA, as represented by THE SECRETARY, DEPARTMENT OF HEALTH AND HUMAN SERVICES, Rockville, MD 20852-3804 (US)
(72) Inventor: COLINS, Peter L., Silver Spring, Maryland 20910 (US); MURPHY, Brian R., Bethesda, Maryland 20816 (US); SKIADOPOULOS, Mario H., Potomac, Maryland 20854 (US); BARLETT, Emmalene J., Chevy Chase, Maryland 20815 (US); SCHMIDT, Alexander C., Bethesda, Maryland 20817 (US); CRUZ, Ann-Marie, M., Bethesda, Maryland 20814 (US)
(74) Representative: Hallybone, Huw George
(86) International application number: PCT/US2008/063033
(87) International publication number: WO 2008/137981

(56) References cited:
- WO-A-03/043587
- BARTLETT ET AL: "Human parainfluenza virus type I (HPIV1) vaccine candidates designed by reverse genetics are attenuated and efficacious in African green monkeys" VACCINE, vol. 23, no. 38, 7 September 2005 (2005-09-07), pages 4631-4646, XP005021726 ISSN: 0264-410X
- BARTLETT ET AL: "Introducing point and deletion mutations into the P/C gene of human parainfluenza virus type 1 (HPIV1) by reverse genetics generates attenuated and efficacious vaccine candidates" VACCINE, vol. 24, no. 14, 24 March 2006 (2006-03-24), pages 2674-2684, XP005309277 ISSN: 0264-410X
- VAN CLEVE ET AL: "Attenuating mutations in the P/C gene of human parainfluenza virus type 1 (HPIV1) vaccine candidates abrogate the inhibition of both induction and signaling of type I interferon (IFN) by wild-type HPIV1" VIROLOGY, vol. 352, no. 1, 15 August 2006 (2006-08-15), pages 61-73, XP005587895 ISSN: 0042-6822

## Description

### BACKGROUND OF THE INVENTION

Human parainfluenza viruses (HPIVs) are members of the *Paramyxovirinae* subfamily of the *Paramyxoviridae* family of viruses. Paramyxoviruses are enveloped viruses that replicate in the cytoplasm and bud at the plasma membrane and have a single-stranded negative-sense non-segmented RNA genome of approximately 13-19 kb. HPIVs are important pathogens in human populations, causing severe lower respiratory tract infections in infants and young children. Human parainfluenza virus type 1 (HPIV1) and type 2 (HPIV2) are the principal etiologic agents of laryngotracheobronchitis (croup) and also cause pneumonia and bronchitis (Chanock et al., 2001, Parainfluenza Viruses 4th Ed., Knipe et al. eds., Lippincott (Philadelphia, PA) 1341-1379). Human parainfluenza virus type 3 (HPIV3) is a leading cause of hospitalization for viral lower respiratory tract disease in infants and young children (Chanock et al., 2001, *supra*). HPIVs are also important causes of respiratory tract disease in adults. Collectively, HPIV1, HPIV2, and HPIV3 have been identified as the etiologic agents responsible for approximately 18% of hospitalizations for pediatric respiratory disease (Murphy et al., 1988, Virus Res., 11:1-15). HPIVs have also been implicated in a significant proportion of cases of virally induced middle ear effusions in children with otitis media (Heikkinen et al., 1999, N. Engl. J. Med., 340:260-264).

Despite considerable efforts, there are currently no parainfluenza virus vaccines available. Attenuated paramyxoviruses suitable for use in vaccines are currently under development. Two live attenuated HPIV3 vaccine candidates, a temperature sensitive (ts) derivative of the wild type HPIV3 JS strain and a bovine PIV3 strain, are currently being tested. (Karron et al, Pediatric Infectious Dis. J., 15:650, 1996; Karron et al, J. Infect. Dis., 171:1107, 1995; Karron et al., J. Infect. Dis., 172:1445, 1995). A chimeric PIV1 vaccine candidate has been generated by replacing the PIV3 HN and F open reading frames with those of PIV in a PIV3 full length cDNA (Tao et al., 2000a). A chimeric HPIV3 bearing the glycoproteins of HPIV2 was also generated previously (Tao et al., 2000b). Attenuated HPIV2 strains have previously been made by introducing mutations into the L protein (WO 04/027037). Recombinant viruses include HPIV3 recombinant viruses having three identified mutations in the L gene. (Skiadopoulos et al, J Virol. 72:1762, 1998; Tao et al, J Virol. 72:2955, 1998; Tao et al, Vaccine, 17:1100, 1999). These live attenuated vaccine candidates can induce protection against HPIV3 infection in some experimental animal models. (Karron et al, J Infect. Dis., 172:1445, 1995b; Skiadopoulos et al, Vaccine 18:503, 1999; Skiadopoulos, Virology, 297: 136, 2002). However immunity to previous HPIV3 infection could limit the use of chimeric HPIV3 vaccines bearing heterologous HPIV1 or HPIV2 glycoproteins. Strategies to develop live viral vaccines are important in the design of safe and stable viral vaccine candidates.

In addition to providing possible vaccine candidates for protection against parainfluenza virus infection and disease, candidate vaccines may also be useful in expressing heterologous viral antigens. Studies demonstrate that foreign genes may be inserted into a paramyxovirus genome and are well expressed. (Bukreyev et al. J. Virol. 80:10293, 2006; Bukereyev et al, J. Virol., 70:6634, 1996; Hassan et al, Virology, 237:249, 1997; Jin et al, Virology 251:206,1998; Schmidt et al., 2001; Skiadopoulos et al., 2002). However, in order to develop vectors for vaccine use, the level of protein expression is not the only influencing factor. Factors to be considered in the design of a vector for delivery of heterologous antigens include viral host range, immunogenicity, and pathogenicity. Some negative strand viruses are undesirable as vectors because of their pathogenicity, such as measles and rabies virus.

Thus, there remains a need to develop effective immunogenic compositions to alleviate health problems associated with HPIVs and other pathogens, and to immunize against multiple HPIV serotypes. There is also a need to develop immunogenic compositions to deliver heterologous antigens.

Each HPIV1 gene encodes a single protein, with the exception of the P/C gene, which contains two open reading frames (ORFs), one coding for the essential P protein and one coding for the non-essential C proteins, i.e., four related accessory proteins referred to as C', C, Y1 and Y2. The P/C genes of HPIV3 are similarly organized, although the P gene of HPIV3 further encodes a D protein accessed by RNA editing and an internal ORF encoding a V protein. (See, e.g. WO 98/53078.) The C ORFs of SeV and HPIV1 are accessed by more than one translational start site to give rise to a nested set of carboxy-coterminal proteins called C', C, Y1 and Y2, listed in the order of appearance of their respective translational start sites. Expression of the Y2 protein, whose existence is predicted based on the nucleotide sequence and by analogy to Sendai virus (SeV, murine PIV1), has not been directly confirmed for HPIV1. C proteins are expressed by viruses of the Respirovirus, Morbillivirus and Henipahvirus genera, but not by viruses of the Rubulavirus and Avulavirus genera. The C proteins are multifunctional, based mainly on studies with SeV. Their most prominent roles are (i) inhibition of the induction of interferon (IFN) α/β by the host cell in response to viral infection and (ii) inhibition of intracellular signaling mediated by IFIV α/β and γ. Other functions of C include down-regulating viral RNA synthesis, facilitating virion budding and release, and inhibiting cellular apoptosis (Karron and Collins, Chapter 42 in Field's Virology, 5th ed., 2007).

The P protein also has several functions. It interacts with the N and L proteins and is essential in forming the active RNA-dependant RNA polymerase (N, P, L). The P protein acts as a chaperone and it might play a role in modulation of the host's immune response.

### DESCRIPTION OF THE INVENTION

The invention provides an infectious self-replicating parainfluenza virus (PIV) comprising a partial or complete PIV1 or PIV3 genome or antigenome comprising
a) a first open reading frame polynucleotide encoding a P protein; and
b) a second open reading frame polynucleotide encoding a C protein
wherein the first and second open reading frame polynucleotides each consist of a single open reading frame and do not overlap.

The invention also provides an infectious self-replicating parainfluenzanvirus (PIV) comprising a partial or complete PIV1 or PIV3 genome or antigenome comprising
a) a first open reading frame polynucleotide encoding a P protein from which expression of C, C', Y1 and Y2 proteins has been ablated or greatly reduced; and
b) a second open reading frame polynucleotide encoding a C protein having at least one nucleotide change that ablates or reduces expression of said C protein or that expresses a truncated C protein or that expresses a C protein with reduced activity compared to a native C protein, and from which expression of a P protein has been ablated or greatly reduced
wherein the first and second open reading frame polynucleotides each consist of a single open reading frame and do not overlap.

The invention further provides an isolated polynucleotide comprising:
a) a first open reading frame polynucleotide encoding a parainfluenza virus (PIV) P protein; and
c) a second open reading frame polynucleotide encoding a parainfluenza virus (PIV) C protein
wherein the first and second open reading frame polynucleotides each consist of a single open reading frame and do not overlap.

The invention further provides an isolated polynucleotide comprising a polynucleotide encoding a partial or complete PIV genome or antigenome comprising the isolated polynucleotide described above.

The invention provides self-replicating, infectious, recombinant parainfluezanviruses (PIV), methods of making the parainfluenzaviruses of the invention, and uses thereof. The PIV of the invention may be a complete virus, or may be a subviral particle comprising at least nucleocapsid (N), phosphoprotein (P) and large polymerase (L) proteins and a genomic or antigenomic RNA comprising all of the cis-acting sequences necessary for replication of the subviral particle and transcription and translation of the proteins encoded by the packaged genome and for packaging of the replicated genome into another viral or sub-viral particle.

The PIV of the invention has a genome or anti genome in which the genes encoding P and C proteins, which normally overlap and are expressed from two ORFs of a common polynucleotide sequence, have been genetically separated into two polynucleotides, each providing only a single "substantial open reading frame" (SSRF) so that mutations can be independently introduced into either or both of the P and C genes. Separation of the two ORFs of the P/C gene allows intentional introduction of mutations and other variations in one ORF without deleterious effects on the other ORF. For example, the IFN-antagonist function or another function of the C proteins can be inhibited by introducing mutations into the C ORF without affecting the P protein. In this manner, the viral RNA dependent RNA polymerase activity can be maintained and virus growth *in vitro* is unaffected by C ORF mutations.

The PIV of the invention can also have one or more amino acid or nucleic acid mutations that confer an attenuated phenotype. In instances where mutations are made by insertions or deletions, the number of nucleotides inserted or deleted is such that the total number of nucleotides in the variant viral genome is divisible by six (known as the "rule of six"). Mutations can be stabilized by at least two changes in the codon specifying the mutation. The PIV of the invention can be human parainfluenza virus (HPIV), such as for example HPIV1 or HYIV3. The "background" genome into which mutations are introduced may be that of HPIV1, the "wild-type" nucleotide sequence of which is represented in SEQ ID NO:1, or HPIV3, the wild-type nucleotide sequence of which is represented by SEQ ID NO: 21.

Furthermore, the PIV of the invention can be a "chimeric" PIV, having a genome in which one or more genes or genome segments of a different PIV are introduced, either as an additional gene beyond the normal complement of genes in the native viral genome (a so-called "supernumerary" gene) or as an exchange, replacing the counterpart of the gene or gene segment from the wild-type genome. The PIV of the invention can also include one or more supernumerary genes or gene segments from another source, such as a gene encoding a protein that affects the host immune response or a gene encoding an epitope from a non-PIV pathogen, such as another virus or a bacterium or parasite.

The PIV of the invention can minimally include a phosphoprotein (P), major nucleocapsid (N) protein, and/or large polymerase (L) protein. The proteins may be variant or naturally occurring. One or more of these proteins may carry one or more mutations that provide for attenuation of replication of a virus or sub-viral particle according to the invention *in vivo* or that provide for favorable properties of the virus during growth *in vitro.*

In some embodiments, the PIV of the invention comprises a partial or complete polyhexameric genome or antigenome comprising a polynucleotide having a single substantial ORF encoding a P protein and a polynucleotide having a single substantial ORF encoding one or more of the C proteins. In this context and throughout this patent application, a "SSRF polynucleotide" is a polynucleotide that has only one substantial reading frame that is transcribed from a genomic (or antigenomic) nucleotide sequence. A SSRF expresses primarily one transcript as a true and exact copy of the genomic or antigenomic nucleotide sequence. Shifted reading frames of the transcript are completely or largely silenced. A SSRF may give rise to one or more gene products that begin from different translational start sites and/or end at different translation stop sites within the SSRF. A polynucleotide that includes two shifted, expressed ORFs, such as for example the naturally occurring C/P gene of HPIV1 or HPIV3, is not a SSRF polynucleotide. However, a SSRF polynucleotide may encompass instances, such as the P/D/V gene complex of HPIV3, wherein additional transcripts are generated by RNA editing.

A "substantial reading frame" ("substantial RF") is an ORF that encodes one or more complete viral proteins. A "complete viral protein" can include minor internal deletions or insertions of one, two or a few amino acids, but translation of the viral protein will end at the native stop codon. An ORF into which stop codons have been introduced so as to terminate translation of a protein in order to ablate expression of that protein is not a substantial open reading frame for that protein.

In some embodiments, the PIV of the invention comprises a partial or complete polyhexameric genome or antigenome comprising a first SSRF polynucleotide expressing one or more C proteins and a second SSRF polynucleotide expressing a P protein. Thus, mutations can be introduced into one or more of the C proteins without at the same time affecting the P protein, and vice versa. The PIV of the invention can comprise a partial or complete polyhexameric genome or antigenome comprising a polynucleotide encoding truncated C proteins and a SSRF polynucleotide encoding a P protein. The PIV of the invention can comprise a partial or complete polyhexameric genome or antigenome in which a SSRF polynucleotide encodes a P protein and any polynucleotide encoding C proteins is deleted.

The polynucleotide encoding a P protein and the polynucleotide encoding a C protein can be separated by a non-coding polynucleotide spacer sequence and, optionally, can be on separate vectors for use in cells for producing stocks of infectious particles. In some embodiments, the non-coding spacer comprises a gene end transcription signal, intergenic transcription signal, and gene start transcription signal. The non-coding spacer can be upstream of the P ORF. The ordering of the polynucleotides encoding the P protein and C proteins can also be changed to P followed by C, and the spacer polynucleotide can be upstream of the polynucleotide encoding the C proteins.

The polynucleotide encoding the C proteins can encode a variant C protein containing a mutation that inhibits the functions of one or more of the C proteins, e.g. ability to interrupt production of IFN in an infected host or signaling though an IFN receptor. The mutation can be amino acid or nucleic acid substitution(s) or deletion(s). In instances of insertions or deletions, the number ofnucleotides inserted or deleted is such that the total number of nucleotides in the variant viral genome is divisible by six. Point mutations can be stabilized by at least two changes in the codon specifying the mutation. In some embodiments, the C protein comprises one or more amino acid substitutions at amino acid residues corresponding to codon positions 83, 84, 85, 169, 170 or 171 or deletions of one or more of these amino acids.

The SSRF polynucleotide encoding the P protein can comprise a P ORF having one or more nucleotide substitutions wherein the substitution introduces one or more stop codons in an overlapping C ORF reading frame but does not alter the amino acid sequence of P protein encoded by the SSRF polynucleotide encoding the P protein. Alternatively, or in addition, the SSRF polynucleotide encoding the P protein can comprise a P ORF having one or more nucleotide substitutions wherein the substitution destroys the start codon in an overlapping C ORF reading frame but does not alter the amino acid sequence of P protein encoded by the SSRF polynucleotide encoding the P protein.

The polynucleotide encoding one or more C proteins can comprise a C ORF having one or more nucleotide substitutions wherein the substitution introduces one or more stop codons in an overlapping P ORF but does not alter the amino acid sequence of C proteins encoded by the polynucleotide encoding the C protein. Alternatively, or in addition, the polynucleotide encoding the C protein can comprise a C ORF having one or more nucleotide substitutions wherein the substitution destroys the start codon in an overlapping P ORF reading frame but does not alter the amino acid sequence of any C protein encoded by the polynucleotide encoding the C proteins. Such polynucleotides encoding the C protein can also include one or more point mutations or deletions that reduce or abolish one or more functions of the C proteins, e.g., the ability of the C proteins to inhibit production of IFN by an infected host cell or IFN signaling as described above.

The polynucleotide encoding one or more C proteins can be a SSRF polynucleotide, or can be a polynucleotide that has at least 85% identity to SEQ ID NO:14 (wild type PIV1 C' protein) or to SEQ ID NO: 23 (PIV3 C' protein) and having at least one nucleotide change that ablates or reduces expression of said C protein or that expresses a truncated C protein.

Due to physical separation of the P and C genes, the mutation(s) introduced into a polynucleotide encoding the C proteins ORF will not alter the P protein. This is important since unwanted mutations of the P proten could have deleterious effects on the biological properties of the recombinant virus, such as restricted growth in vitro.

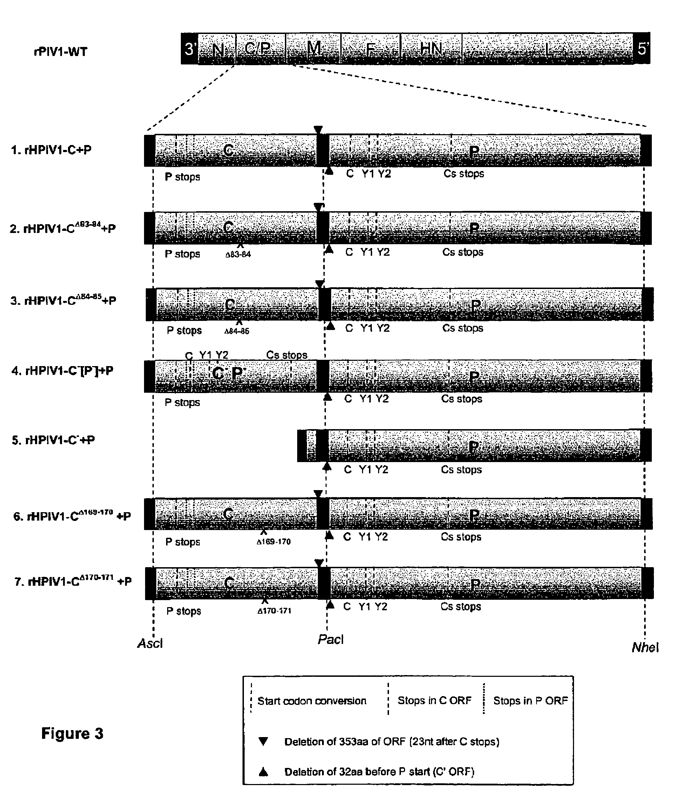

The disclosure also includes polynucleotides and methods of using polynucleotides. In some embodiments, an isolated nucleic acid comprises a polynucleotide having at least 85% sequence identity to a polynucleotide of SEQ ID NO: 14 (wild type HPIV1 C' ORF) or to SEQ ID NO: 23 (wild type HPIV3 C ORF). In other embodiments, an isolated nucleic acid comprises a polynucleotide having a sequence of SEQ ID NO: 15 (mutated C' ORF eliminating P). In further embodiments, an isolated nucleic acid comprises a polynucleotide having a sequence of one of SEQ ID NOs: 2 through 8 (cDNAs for viruses 1-7, Figure 3). The disclosure also includes an isolated nucleic acid comprising a polynucleotide encoding a polypeptide having at least 85% sequence identity to a C polypeptide of SEQ ID NO: 17 (wild-type C' protein of HPIV1) or to SEQ ID NO: 23 (wild type C protein of HPIV3). Other embodiments include an isolated nucleic acid comprising a polynucleotide encoding a polypeptide having at least 85% sequence identity to a polypeptide of SEQ ID NO: 11 (wild-type P protein of HPIV1) or to SEQ ID NO: 22 (wild type P protein of HPIV3). The disclosure also includes vectors including any of the polynucleotides as well as a partial or complete genome or antigenome. Also provided are methods of producing a paramyxovirus particle or sub-viral particle by culturing a host cell comprising any of the polynucleotides described herein.

The invention also encompasses incorporation of any one or more of the many known mutations from negative stranded RNA viruses into homologous positions of the HPIV1 genome that provide for a cold-passage (cp), temperature sensitive (ts), and/or attenuated (att) phenotype. For example, known mutations in the L protein of HPIV1 include mutation at one amino acid residue corresponding to position 456, 942, 992, 1710, or 1711 of an L protein having an amino acid sequence of SEQ ID NO: 13 (wild-type L protein sequence of HPIV1). The L protein may be modified by one or more of the following amino acid substitutions: position 456 is substituted with L, position 942 is substituted with A or T, position 992 is substituted with C or N, or deletion of positions 1710 and 1711. These, and some additional mutations that are useful for attenuating a PIV of the invention, are shown in Table 1 and 2:

**Table 1. Attenuating mutations in the P/C and L genes of HPIV1 and HPIV3**

| **Mutation** | | **Gene** | **Virus** | **ORFs Affected** | **# nt. reversion to wt aa** | **Growth restriction *in vitro*** | **Attenuation *in vivo*** | |
|---|---|---|---|---|---|---|---|---|
| | | | | | | | **Hamsters** | **AGMs** |
| 1 | Δ10-15 | P/C | HPIV1 | P/C'/C/Y1 /Y2 | 18 | no | yes | no |
| 2 | R84G | P/C | HPIV1 | P/C'/C/Y1lY2 | 1 | no | yes | no |
| 3 | Δ83-84 | P/C | HPIV1 | P/C'/C/Y1/Y2 | 6 | yes | - | - |
| 4 | Δ84-85 | P/C | HPIV1 | P/C'/C/Y1/Y2 | 6 | yes | - | - |
| 5 | F170S | P/C | HPIV1 | C'/C/Y1/Y2 | 1 | no | yes | yes |
| 6 | Δ170 | P/C | HPIV1 | P/C'/C/Y1/Y2 | 6 | no | yes | yes |
| 7 | I96T | P/C/D/V | HPIV3 | P/C | 1 | no | yes | - |
| 8 | F164S | P/C/D/V | HPIV3 | P/C | | no | yes | yes |
| 9 | F456L | L | HPIV1 | L | 2 | yes | yes | yes |
| 10 | Y942H | L | HPIV1 | L | 1 | yes | yes | yes |
| 11 | Y942A | L | HPIV1 | L | 3 | yes | yes | yes |
| 12 | Y942T | L | HPIV1 | L | 3 | yes | yes | - |
| 13 | L992F | L | HPIV1 | L | 1 | no | no | yes |
| 14 | L992C | L | HPIV1 | L | 2 | yes | yes | yes |
| 15 | L992N | L | HPIV1 | L | 2 | yes | yes | - |
| 16 | L15581 | L | HPIV1 | L | 2 | yes | yes | yes |
| 17 | Δ1710-11 | L | HPIV1 | L | 6 | yes | yes | yes |
| 18 | 456 | L | HPIV3 | L | 2 | yes | no | - |
| 19 | Y942H | L | HPIV3 | L | 1 | yes | yes | - |
| 20 | L992F | L | HPIV3 | L | 1 | yes | yes | - |
| 21 | T15581 | L | HPIV3 | L | 2 | yes | yes | - |

| | | | | | | | | |
|---|---|---|---|---|---|---|---|---|
| "-" means not tested. | | | | | | | | |

The PIV of the invention comprises one or more attenuating mutations. The attenuating mutations(s) can be temperature sensitive. Replication of PIV of the invention comprising one or more temperature sensitive mutations is attenuated in vitro at about 35°C or greater, as compared to the corresponding wild type PIV. Temperature sensitive mutations can comprise amino acid substitution or deletion of one or more amino acid residues corresponding to position 456, 942, 992, 1710 or 1711 of an L protein having an amino acid sequence of SEQ ID NO: 13 (wild-type L of PIV1). In an embodiment, the substitution comprises F456L. In an embodiment, the substitution comprises Y942A or Y942T. In an embodiment, the substitution comprises L992C or L992N. In an embodiment, amino acid residues at positions 1710 and 1711 are deleted.

The attenuating mutation(s) can be non-temperature sensitive. Non-temperature sensitive mutations include a nucleotide substitution at a position corresponding to amino acid position 170 of the HPIV1 C protein, for example F170S. The polynucleotide encoding C proteins may have a nucleic acid sequence of SEQ ID NO: 16 (nucleotide sequence of F170S C ORF). Non-temperature sensitive mutations can be host range restricted. For example, the PIV of the invention might replicate well in hamsters but might be restricted in replication in African green monkeys.

The PIV of the invention can comprise at least one temperature sensitive mutation and at least one non-temperature sensitive mutation. In an embodiment, at least one of the temperature sensitive mutations comprises an amino acid substitution or deletion of one or more amino acid residues corresponding to position 456, 942, 992, 1710, or 1711, or mixtures thereof in an L protein having an amino acid sequence of SEQ ID NO: 13 (wild-type L of HPIV1) and at least one of the non-temperature sensitive mutations comprising a nucleic acid substitution at a position corresponding to amino acid position 170 of the HPIV1 C protein.

In some embodiments, the PIV of the invention comprises one or more supernumerary heterologous polynucleotides or genome segments encoding one or more antigenic determinants of a heterologous pathogen. The heterologous pathogen can be HPIV1, HPIV2, HPIV3, measles virus, subgroup A or subgroup B respiratory syncytial virus, mumps virus, human papilloma virus, type 1 or type 2 human immunodeficiency virus, herpes simplex virus, cytomegalovirus, rabies virus, Epstein Barr virus, filovirus, bunyavirus, flavivirus, alphavirus, human metapneumovirus, or influenza virus. In an embodiment, the antigen determinant comprises measles HA, HPIV1 HN or F (e.g. for a HPIV3 background), HPIV2 HN or F, HPIV3 HN or F (e.g. for a HPIV1 background) or respiratory syncytial virus (RSV) F or G protein. The antigenic determinant can also be one from an antigen of a bacterial pathogen, or a parasite.

In some embodiments, the supernumerary polynucleotide or genome segment can include an ORF of an immunomodulatory gene, for example an interleukin or a colony stimulating factor or other cytokine or chemokine. Such genes are well-known in the art.

Another aspect of the disclosure includes methods of making the PIV of the invention and polynucleotides according to the invention. In some embodiments, the methods of the disclosure comprise removing a multicistronic polynucleotide encoding P and C proteins from the viral genome or antigenome of a PIV and inserting a polynucleotide encoding one or more C proteins, which is optionally a SSRF polynucleotide, and a SSRF polynucleotide encoding a P protein into a full length or partial genome or antigenome of a PIV. In embodiments used in cell culture, the polynucleotide encoding one or more C proteins and the SSRF polynucleotide encoding a P protein can be on the same vector or separate vectors. In some embodiments, the polynucleotide encoding a P protein and the polynucleotide encoding one or more C proteins are separated by a non-coding polynucleotide spacer sequence comprising a gene end transcription signal, intergenic transcription signal, and gene start transcription signal. The SSRF polynucleotide encoding a P protein may include various attenuating mutations affecting replication of the virus *in vivo* or *in vitro.*

In an embodiment, the removing step comprises introducing unique restriction enzyme recognition sequences into the genome or antigenome such that the recognition sequences flank the polycistronic polynucleotide, and digesting the genome with one or more restriction enzymes that cut the genome at the restriction sites flanking the polycistronic polynucleotide. In an embodiment, the inserting step comprises inserting at the cleaved restriction sites a polynucleotide encoding one or more C proteins and having at least one nucleotide change that ablates or reduces expression of said C protein(s) or that expresses a truncated C protein or that expresses a C protein with reduced activity compared to the corresponding native C protein, and a second, SSRF polynucleotide encoding a P protein and religating the genome or antigenome. In an embodiment, the inserting step comprises inserting a SSRF polynucleotide encoding at least one C protein and a SSRF polynucleotide encoding a P protein at the cleaved restriction sites, and religating the genome or antigenome. The SSRF polynucleotide encoding a P protein may include various attenuating mutations affecting replication of the virus *in vivo* or *in vitro.*

In some embodiments, the methods of the disclosure comprise coexpressing in a cell an expression vector comprising a partial or complete polyhexameric genome or antigenome encoding a PIV of the invention and one or more polynucleotides encoding a N protein, a P protein and a L protein, and incubating the cell under conditions that allow for viral replication. The cells can be, for example, BHK-T7 cells, HEp-2 cells, Vero cells, or LLC-MK2 cells. Methods for obtaining viral or sub-viral particles from such cultures are considered known in the art.

Another aspect of the disclosure is a composition comprising PIV of the invention. The PIVs of the invention are useful, for example, in immunogenic compositions for eliciting an immune response in an animal, including live virus vaccines (which are typically attenuated live virus vaccines) and/or vectors for expressing heterologous antigens. PIV of the invention can be combined with viruses of other PIV serotypes, strains, or genera in a composition to elicit an immune response against multiple genera, serotypes, and strains.

The compositions of the disclosure comprise an immunogenic effective amount of a PIV of the invention and a physiologically acceptable carrier. The compositions of the disclosure can also comprise an adjuvant. In an embodiment, the composition of the disclosure comprises PIV from two or more serotypes. In an embodiment, at least one or more of the serotypes is HPIV1, HPIV2, HPIV3, or HPIV4. The HP1V2 can be strain V94, V98, or Greer. In an embodiment, the composition of the disclosure comprises PIV from two or more genera. In an embodiment, at least one genus is Respirovirus genus.

Another aspect of the disclosure is methods of eliciting an immune response in an animal. The methods generally comprise administering an immunogenic effective amount of a composition of the disclosure the animal. Preferably the immune response produces anti-PIV antibodies that are protective. In an embodiment, the antibodies are IgA. In an embodiment, the immune response produces antibodies that bind one or more antigenic determinants of a heterologous pathogen encoded by a supernumerary gene or genome segment of the PIV of the invention. The heterologous pathogen can be HPIV1 (to a HPIV3 background genome or antigenome), HPIV2, HPIV3 (to a HPIV1 background genome or antigenome), measles virus, subgroup A or subgroup B respiratory syncytial virus, mumps virus, human papilloma virus, type 1 or type 2 human immunodeficiency virus, herpes simplex virus, cytomegalovirus, rabies virus, Epstein Barr virus, filovirus, bunyavirus, flavivirus, alphavirus, human metapneumovirus, or influenza virus. The heterologous pathogen can be a bacterium or a parasite, such as malaria. In an embodiment, the antigenic determinant is from measles HA, HPIV1 HN or F, HPIV2 HN or F, HPIV3 HN or F or RSV G or F.

### BRIEF DESCRIPTION OF THE DRAWINGS

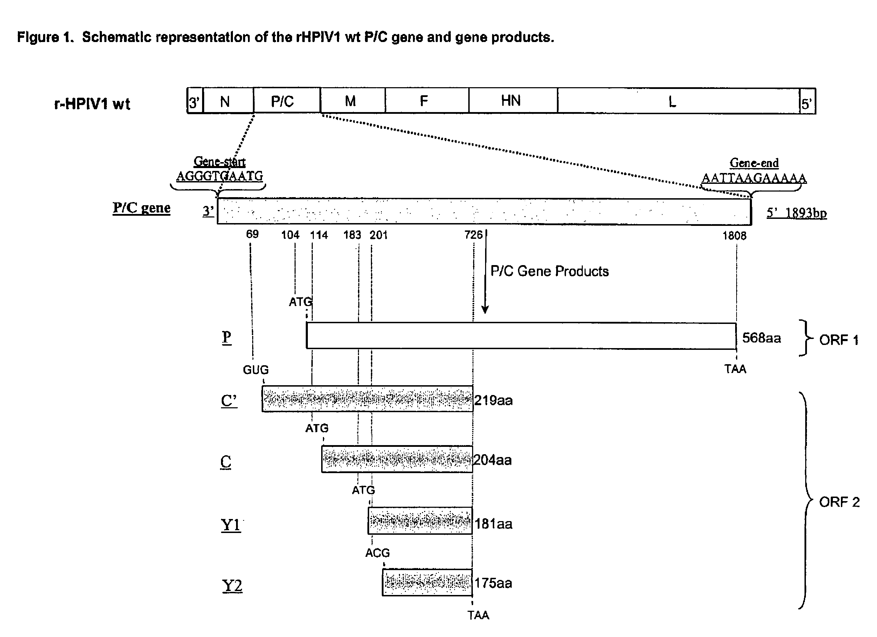

**Figure 1****.** Schematic representation of the rHPIV1 wt P/C gene and gene products. The order of the naturally occurring HPIV1 wt genome is: 3'-N-P/C-M-F-HN-L-5'. The P/C gene is highlighted since it encodes multiple proteins from two overlapping ORFs, one encoding the phosphoprotein (P) and the second encoding a group of non-structural, non-essential, C accessory proteins (C'/C/Y1/Y2). In this setting any mutation introduced into the P/C gene has the potential to affect both the P and C proteins.

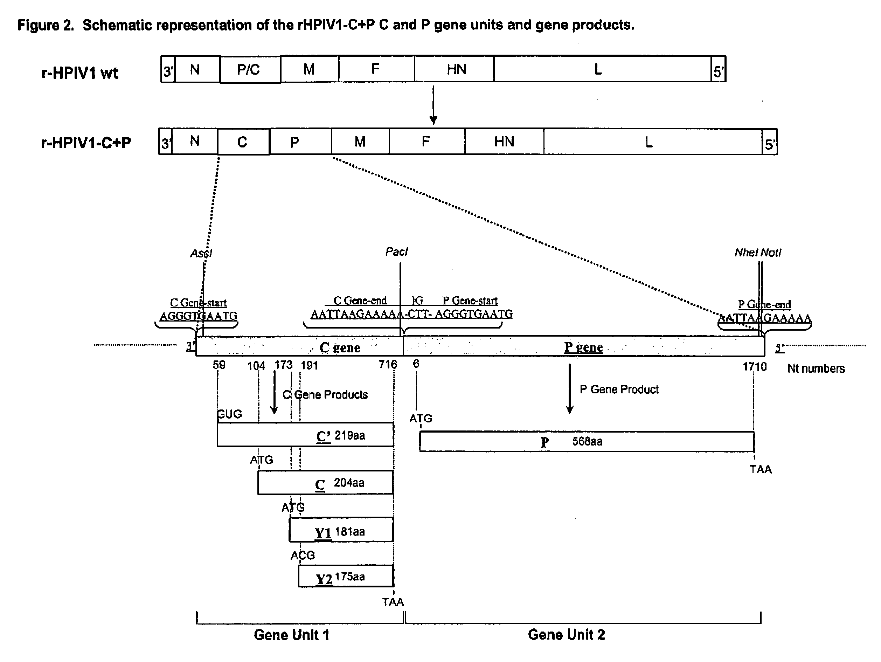

**Figure 2****.** Schematic representation of the rHPIV1-C+P C and P gene units and gene products. The P/C gene of HPIV1 wt has been manipulated such that the C and P ORFs have been separated into individual gene units, gene unit 1 and gene unit 2, respectively. This new viral cDNA has been termed pFLC-rHPIV1-C+P, and the new order of the genome is: 3'-N-C-P-M-F-HN-L-5'. Briefly, using site-directed mutagenesis, the new C gene unit contains nucleotide changes designed to convert the P start codon and to introduce stop codons into the P ORF such that these changes are silent in C and that only the C proteins and not the P protein are expressed from this gene unit. Conversely, the new P gene unit contains nucleotide changes designed to convert the C start codon and to introduce stop codons into the C ORF such that these changes are silent in P and that only the P protein and none of the C proteins are expressed from this gene unit. These gene units are flanked by unique restriction endonuclease digestion sites to facilitate cloning.

**Figure 3****.** Schematic of the rHIPV1-C+P and P/C mutants. A representation of the rHPIV1 wt genome is shown at the top and below is a list of rHPIV1-C+P viruses including the rHPIV1-C+P wt and various C mutants. The C and P gene units of the rHPIV1-C+P viruses are highlighted to show the changes that have been introduced into the C and P gene units to allow the C gene unit to express the C proteins but not the P protein and, similarly, for the P gene unit to express the P protein but not the C proteins, to produce the rHPIV1-C+P virus.

Changes include conversion of start codons (dashed lines), introduction of stop codons into the C ORF (dashed lines), introduction of stop codons into the P ORF (dotted lines) and truncation of non-coding regions (black triangles), for more detail refer to "Example 1". Mutations that have been introduced into the C gene unit are highlighted (crosses) and include C^{ΔH3-84} (rHPIV1-C^{Δ83-84}+P, virus #2, Figure 3; Group 2, Table 1), C^{Δ84-85} (rHPIV1-C^{Δ84-85}+P, virus #3, Figure 3; Group 3, Table 1), C^{Δ169-170} (rHPIV1-C^{Δ169-170}+P, virus #6, Figure 3; Group 6, Table 1), C^{Δ170-171} (rHPIV1-C^{Δ170-171}+P, virus #7, Figure 3; Group 7, Table 1).

In addition, rHPIV1-C+P variants that do not express any wild type C proteins have been recovered, and these include rHPIV1-C⁻(P⁻)+P (virus #4, Figure 3) and rHPIV1-C⁻+P (virus #5, Figure 3). The rHPIV1-C⁻(P⁻)+P mutant has been designed to include the C and P gene units of the rHPIV1-C+P, however, the C gene unit incorporates the same changes that were introduced into the P gene unit such that this gene unit
(C⁻(P⁻)) expresses neither C nor P proteins. This virus is the same length as the rHPIV1-C+P. The rHPIV1-C⁻+P has been designed such that the C gene unit has been removed and the virus contains the P gene unit of rHPIV1-C+P such that this virus will not express C proteins. This virus is similar in length to rHPIV1wt. "Cs stops" indicates the native stop codon that is carboxy co-terminal for all C proteins.

**Figure 4****.** Kinetics of replication of rHPIV1 wt and rHPN1-C+P viruses including the C^{Δ83-84} and C^{Δ84-85} deletion mutations. Viruses including rHPIV1 wt, rHPIV1-C+P, rHPIV1-P/C^{Δ83-84}HN^{T553A}, rHPIV1-P/C^{Δ84-85}HN^{T553A}, rHPIV1-C^{Δ83-84}+P HN^{T553A}, and rHPIV1-C^{Δ84-85}+P HN^{T553A} were compared in a multi-cycle growth curve on LLC-MK2 cells. LLC-MK2 cell cultures were infected at an MOI=0.01 and samples were harvested for virus titration at days 0, 2, 4, 6, 8 and 9 post-infection.

**Figure 5****.** Map of HPIV3, organization of the HPIV3 P/C/V/D gene, and summary of modifications to construct separate C and P/V/D genes in HPIV3-C+P.

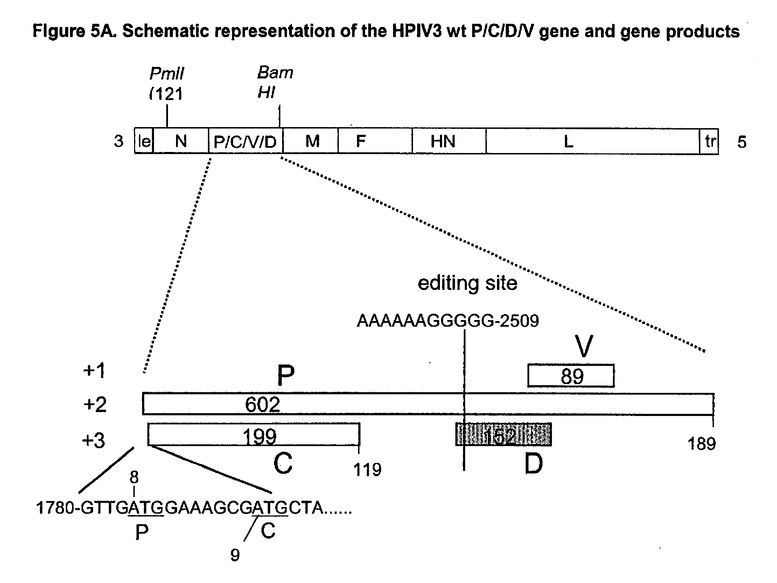

Figure 5A shows the arrangement of the P, C, V and D ORFs in wt HPIV3, positioned according to their locations in the three reading frames (+1, +2, +3), with the codon length indicated. The P ORF is expressed by translational initiation at the ATG at positions 80-82 in the gene. C is expressed by initiation at positions 90-92. The D ORF is expressed by RNA editing at the site shown (positions 2499-2509, numbered according to the complete wt genome sequence). This shifts the reading frame and fuses the N-terminal 242 amino acids of P to 131 amino acids encoded by the D ORF, resulting in a 373 amino acid protein. The mechanism of expression of the V ORF is not known, although there is genetic evidence that it encodes a functional protein. Note that the D and V ORFs are downstream of the C ORF and are thought to be expressed independently of C. The *Pme*I and *Bam*HI sites (numbered according to the complete antigenome sequence) demarcate a fragment of the complete antigenome cDNA that was subcloned and engineered by the insertion of additional restriction sites and an additional gene junction as described by Skiadopoulos et al, Virology 297:136-152, 2002. The resulting cloned DNA, pUC(GE/GS)_{P-M}, provides a convenient substrate for the engineering described here.

Figure 5B. Map of HPIV3-C+P, in which the C ORF is expressed from one SORF and the P protein is expressed from a second SORF. The D and V ORFs co-segregate with the P SSRF and would be expressed as usual from that polynucleotide.

Figure 5C. Mutations that silence expression of the HPIV3 C ORF without affecting the P/V/D ORFs.

Figures 5D and 5E. Mutations that silence expression of the HPIV3 P ORF, delete the V and D ORFs, and provide *Mlu*I sites for cloning into pUC(GE/GS)_{P-M}.

**Figure 6** illustrates the kinetics of replication of rHPIV1-P(C-). Figure 6A shows replication in LLC-MK2 cells. Figure 6B shows replication in Vero cells. Legend is the same as for Figure 6A.

**Figure 7** illustrates the effect of infection with rHPIV1 wild-type or mutants on type I interferon signaling.

**Figure 8** shows the efficacy of rHPIV1 wt and mutants in protecting African green monkeys (AGMs) against challenge with wild-type HPIV1. Figure 8A shows results from the upper respiratory tract (nasopharyngeal swab). Figure 8B shows results from the lower respiratory tract (tracheal lavage).

### DESCRIPTION OF THE INVENTION

"Paramyxovirus" as used herein refers to a paramyxovirus of the *Paramyxovirinae* subfamily of the *Paramyxoviridae* family. Paramyxoviruses are enveloped viruses that have a single strand of negative sense RNA of approximately 13 to 19 kb as a genome. Examples of paramyxoviruses include, but are not limited to, human parainfluenza virus (HPIV) including types 1, 2, 3, 4A, and 4B (HPIV1, HPIV2, HPIV3, HPIV4A, and HPIV4B, respectively), mouse parainfluenza type 1 (Sendai virus, MPIV1), bovine parainfluenza virus type 3 (BPIV3), simian virus 5 (SV5), simian virus 41 (SV41), and mumps virus. HPIV1, HPIV3, MPIV1, and BPIV3 are classified in the genus Respirovirus. HPIV2, HPIV4, SV5, SV41, and mumps virus are classified in the genus Rubulavirus. MPIV1, SV5, and BPIV3 are animal counterparts of HPIV1, HPIV2, and HPIV3, respectively (Chancock et al., Parainfluenza Viruses, Knipe et al. (Eds.), pp. 1341-1379, Lippincott Williams & Wilkins, Philadelphia, 2001). HPIV1, HPIV2, and HPIV3 represent distinct serotypes and do not elicit significant cross immunity. HPIVs are etiological agents of respiratory infections such as croup, pneumonia, or bronchitis.

The term "human parainfluenza virus type 1" or "HPIV1" refers to an isolate, clone, recombinant, or variant of human parainfluenza virus type 1 of the *Paramyxovirinae* subfamily. A "naturally occurring" isolate or "wild type" HPIV1 is a virus isolated from a natural source or has the sequence of a HPIV1 isolated from a natural source. Naturally occurring isolates may differ from one another in sequence. In some embodiments, the genome of a naturally occurring isolate of HPIV1 of the invention has at least 85% nucleic acid sequence identity to SEQ ID NO: 1 (wild-type HPIV1 genome sequence); Genbank Accession No. NC_003461). "Recombinant HPIV1" refers to virus derived from a polynucleotide that has been constructed to encode a HPIV1 genome or antigenome, and may include a sequence of a wild type or variant HPIV1. In some embodiments, the recombinant HPIV1 genome or antigenome is provided as part of an expression vector.

The HPIV1 genome encodes at least seven polypeptides. The ribonucleocapsid-associated polypeptides include the nucleocapsid protein (N) (encoded by nucleotides 120-1694 of SEQ ID NO: 1), the phosphoprotein (P) (SEQ ID NO:11 (wild-type HPIV1 P protein)), and the large polymerase (L) protein (SEQ ID NO: 13 (wild-type HPIV1 L protein)) that carry out transcription and replication. The P/C gene of HPIV1 includes an alternative open reading frame (ORF) with several alternative translational start sites encoding a set of up to four C-terminal nested proteins, the C', C, Y1, and Y2 proteins that appear to function, amongst several functions, as interferon antagonists and interferon signaling antagonists. The internal matrix protein (M) and the major protective antigens, fusion glycoprotein (F) and hemagglutinin-neuraminidase glycoprotein (HN), are envelope-associated proteins. The gene order is 3'- N-P/C- M- F- HN-L-5'.

An HPIV1 or HPIV3 encoding polynucleotide can be isolated from virus obtained from infected humans or cells or can be prepared as described herein. Viral stock of HPIV1 is available from the American Type Culture Collection under the catalog number VR-94. Viral stock of HPIV3 is available from the American Type Culture Collection under the catalog number VR-93. See also, for example, WO 03/043587 and WO 98/53078.

"Variants" of HPIV refer to a virus that has a genomic sequence that differs from the sequence of a reference virus. In some embodiments, a variant may be prepared by altering or modifying the nucleic acid sequence of the viral genome by addition, substitution, and deletion of nucleotides. As discussed previously, it is preferred that variants that have a modification due to addition or deletion of nucleotides conform to the rule of six. In some embodiments, variants may be obtained by passage of a viral particle or genome *in vitro* in a host cell or *in vivo* in a non-human host. In some embodiments, the number of nucleotides inserted or deleted is such that the total number of nucleotides in the variant viral genome is divisible by six (known as the "rule of six").

In some embodiments, the variants have a phenotype altered in at least one aspect compared to the wild-type virus. The alterations to phenotypes can include, without limitation, a change in growth characteristics *in vitro* or *in vivo,* attenuation *in vitro* or *in vivo*, temperature sensitive growth *in vitro,* cold adaptation, plaque size, host range restriction or a change in immunogenicity. In some embodiments, variant HPIV1 can be immunogenic and elicit protective antibodies in a mammal. The HPIV1 variants according to the invention are attenuated *in vivo* in some embodiments. The degree of attenuation *in vivo* that is often obtained is one that decreases rhinorrhea in an infected mammalian host when compared to infection with a wild-type HPIV1. Such degree of attenuation may be approximately 10- to 10,000-fold in the respiratory tract of the infected host. Differences in the degree of attenuation between the upper and lower respiratory tract may be observed. In some embodiments, attenuation of replication of the PIV of the invention *in vivo* is observed, without significant attenuation of replication *in vitro.*

In some embodiments, the background HPIV1 or HPIV3 genome or antigenome has at least 80% sequence identity, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or greater sequence identity to SEQ ID NO: 1 (wild-type HPIV1 sequence) or to SEQ ID NO: 21 (wild type HPIV3 sequence), respectively.

In some embodiments, the PIV genome or antigenome comprises a polynucleotide encoding a C protein having at least 80% sequence identity, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or greater sequence identity to a C gene reference sequence from HPIV1 (SEQ ID NO: 14 (wild-type HPIV1 C' nucleotide sequence)) or to a C gene reference sequence from HPIV3 (nucleotides 1794 to 2393 of SEQ ID NO:21 (wild type HPIV3 C gene sequence)).

In some embodiments, the PIV genome or antigenome comprises a polynucleotide encoding a P protein having at least 80% sequence identity, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or greater sequence identity to a P gene sequence from HPIV1 (SEQ ID NO: 10 (wild-type HPIV1 P gene sequence)) or from HPIV3 (nucleotides 1784 to 3595 of SEQ ID NO:21 (wild-type HPIV3 P gene sequence)).

In some embodiments, the PIV genome or antigenome comprises a polynucleotide encoding an L protein having at least 80% sequence identity, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or greater sequence identity to a reference polynucleotide sequence encoding an L protein from HPIV1 (SEQ ID NO: 12 (HPIV1 wild-type L gene sequence) or from HPIV3 (nucleotides 8646 to 15347 of SEQ ID NO: 21 (HPIV 3 wild-type L gene sequence)).

In some embodiments, the PIV genome or antigenome comprises a polynucleotide encoding a HN protein having at least 80% sequence identity, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or greater sequence identity to a HN gene sequence from HPIV1 (nucleotides 6903 to 8630 of SEQ ID NO: 1 (wild-type HPIV1 HN nucleotide sequence)) or to a HN gene sequence from HPIV3 (nucleotides 6806 to 8530 of SEQ ID NO: 21 (wild type HPIV3 HN gene sequence)). Some particular viruses of the present invention incorporating at least one mutation in the HN protein are rHPIV1 -C^{Δ83}-84/HN^{T553A}, rHPIV1-C^{Δ84-85}/HN^{T553A} and rHPIV1-C^{Δ169-170}/HN^{T553A}. These viruses are recombinant HPIV1 having separated C and P proteins, in which the separated C protein has the indicated deletion of two codons, the separated P protein is wild-type, and the HN protein includes the T553A point mutation.

In some embodiments, the PIV genome or antigenome comprises a polynucleotide encoding a C protein having at least 80% sequence identity, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or greater sequence identity to a C protein sequence from HPIV1 (SEQ ID NO: 17 (wild-type HPIV1 C' protein sequence) or from HPIV3 (SEQ ID NO: 23 (wild-type HPIV3 C protein sequence)).

In some embodiments, the PIV genome or antigenome comprises a polynucleotide encoding a P protein having at least 80% sequence identity, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or greater sequence identity to a P protein sequence from HPIV1 (SEQ ID NO: 11 (wild-type HPIV1 P protein sequence)) or from HPTV3 (SEQ ID NO:22 (wild-type HPIV3 P protein sequence)).

In some embodiments, the PIV genome or antigenome comprises a polynucleotide encoding an L protein having at least 80% sequence identity, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or greater sequence identity to a reference protien sequence encoding an L protein from HPIV1 (SEQ ID NO: 13 (HPIV wild-type L protein sequence)) or HPIV3 (SEQ ID NO: 25 (HPIV3 wild-type L protein sequence)).

In some embodiments, the PIV genome or antigenome comprises a polynucleotide encoding a HN protein having at least 80% sequence identity, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or greater sequence identity to a reference protien sequence encoding an HN protein from HPIV (SEQ ID NO: 18 (HPIV1 wild-type HN protein sequence)) or HPIV3 (SEQ ID NO: 24 (HPIV3 wild-type HN protein sequence)).

The term "antigenome" means a positive sense viral RNA molecule or DNA molecule complementary to the entire negative sense single stranded viral RNA genome.

A paramyxovirus that is "attenuated" or has an *"att* phenotype" refers to a paramyxovirus that has decreased reactogenicity and/or decreased replication in a mammal as compared to replication of a reference wild-type paramyxovirus under similar conditions of infection. In some embodiments, a paramyxovirus that is attenuated exhibits at least about 10-fold or greater decrease, more preferably at least about 100-fold or greater decrease, more preferably at least about 1000-fold or greater decrease in virus titer in the upper or lower respiratory tract of a mammal compared to non attenuated, wild type virus titer in the upper or lower respiratory tract, respectively, of a mammal of the same species under the same conditions of infection. Examples of mammals include, but are not limited to, humans, mice, rabbits, rats, hamsters, such as for example *Mesocricetus auratus,* and non-human primates, such as for example *Ceropithecus aethiops.* An attenuated paramyxovirus may display different phenotypes including without limitation altered growth, temperature sensitive growth, host range restricted growth or plaque size alteration.

"Carriers" as used herein include pharmaceutically acceptable carriers, excipients, or stabilizers, which are nontoxic to the cell or mammal being exposed thereto at the dosages and concentrations, employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLORONICS™.

An "infectious clone " of a paramyxovirus as used herein refers to a full-length genome or portion of a genome of a paramyxovirus isolate cloned into a replicable vector that provides for amplification of the viral genome in a cell and in some embodiments, results in viral particles. In some embodiments, a portion of the paramyxovirus genome comprises a polyhexameric nucleic acid sequence encoding at least N protein, P protein, and L protein in a single replicable vector. In other embodiments, the viral genome is a full-length genome. The replicable vector provides for introduction and amplification of the viral genome in a wide variety of prokaryotic and eukaryotic cells.

The term "immunogenic effective amount" of a paramyxovirus, component thereof, or other antigenic determinant refers to an amount of a paramyxovirus, component thereof, or other antigenic determinant that induces an immune response in an animal. The immune response may be determined by measuring a T or B cell response, or by challenging an immunized animal with a virus capable of replicating in the host species. Typically, the induction of an immune response is determined by the detection of antibodies specific for paramyxovirus, a component thereof, or other antigenic determinants. An immune response is deemed to have been "induced" if, following immunization, the replication of homotypic challenge wild type virus is reduced 10-fold or more in the upper or lower respiratory tract of a mammal.

An "isolated" nucleic acid molecule refers to a nucleic acid molecule that is identified and separated from at least one contaminant nucleic acid molecule with which it is ordinarily associated in the natural source. Preferably, the isolated nucleic acid is free of association with all components with which it is naturally associated. An isolated nucleic acid molecule is other than in the form or setting in which it is found in nature.

"Percent (%) nucleic acid sequence identity" with respect to the nucleic acid sequences identified herein is defined as the percentage of nucleotides in a candidate sequence that are identical with the nucleotides in a reference paramyxovirus nucleic acid sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared.

For purposes herein, the % nucleic acid sequence identity of a given nucleic acid sequence A to, with, or against a given nucleic acid sequence B (which can alternatively be phrased as a given nucleic acid sequence A that has or comprises a certain % nucleic acid sequence identity to, with, or against a given nucleic acid sequence B) is calculated as follows:

100 times the fraction W/Z

where W is the number of nucleotides scored as identical matches by the sequence alignment program in that program's alignment of A and B, and where Z is the total number of nucleotides in B. It will be appreciated that where the length of nucleic acid sequence A is not equal to the length of nucleic acid sequence B, the % nucleic acid sequence identity of A to B will not equal the % nucleic acid sequence identity of B to A.

"Percent (%) amino acid sequence identity" with respect to the amino acid sequences identified herein is defined as the percentage of amino acid residues in a candidate sequence that are identical with the amino acid residues polypeptide reference sequence, such as for example the amino acid sequence ofN protein, P protein, C protein, M protein, F protein, HN, or L protein, after aligning the sequences and introducing gaps, if necessary, to achieve the maximum percent sequence identity. Alignment for purposes of determining percent amino acid sequence identity can be achieved in various ways that are within the skill in the art, for instance, using publicly available computer software such as BLAST, BLAST-2, ALIGN, ALIGN-2, clustal C (DNASTAR) or Megalign (DNASTAR) software. Those skilled in the art can determine appropriate parameters for measuring alignment, including any algorithms needed to achieve maximal alignment over the full-length of the sequences being compared.

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

As used herein, "stable" paramyxovirus refers to a paramyxovirus that has a low risk of reversion to a reference virus sequence or phenotype after passaging, infection, or selective pressure. In some embodiments, the reference sequence is the sequence of a wild-type HPIV1. Non-wild type phenotypes include without limitation, a change in growth characteristics, attenuation *in vitro* or *in vivo,* temperature sensitive growth *in vitro,* cold adaptation, plaque size, host range restriction or a change in immunogenicity. A virus or sub-viral particle of the invention may exhibit more than one change in phenotype compared to a corresponding wild-type HPIV. In some embodiments, a mutation is stable if it does not revert to the reference sequence or phenotype after at least 8 *in vitro* cell culture passages. In some embodiments, the mutation is stable if it does not revert to a reference sequence or phenotype when grown at 38-40°C. In some embodiments, the mutation is stable if it does not revert to a reference sequence or phenotype at least 10 days post-infection of a mammal. Generally, genetic stability increases as the number of nucleotide substitutions increases. For example, a codon substitution that would require 3 nucleotides changes to revert to the wild type or wild type-like codon is more stable than a codon substitution that would require only 1 nucleotide change to revert to the wild type or wild type-like codon. Deletion mutations generally confer a greater level of genetic stability than codon substitutions. For example, deletion of a codon would require insertion of 3 nucleotides to revert to wild type.

"Recombinant" in reference to a polynucleotide refers to a polynucleotide that has been derived by recombinant DNA methods and includes recombinant molecules and recombinant viruses. "Recombinant" in reference to a paramyxovirus refers to a virus that is encoded or has been produced from such a polynucleotide. "Recombinant PIV genome or antigenome" or "rPIV" refers to a polynucleotide that has been constructed to encode a PIV strain or variant, and may include a sequence of a wild type or variant PIV. In some embodiments, the recombinant PIV genome or antigenome is in the form of a cDNA. Such a cDNA can include, in addition to viral genome or antigenome sequences, polynucleotides useful in rescue of virus or sub-viral particles from cultured cells, for example a promoter for transcribing a complete PIV genome or antigenome and a ribozyme sequence that processes the ends of the transcript. (See, e.g. WO 03/043587). In some embodiments, a polynucleotide sequence encoding all or a portion of a paramyxovirus viral genome or antigenome may be isolated and combined with other control sequences in a vector. The other control sequences may be those that are found in the naturally occurring gene or from other sources. The vector provides for amplification of the recombinant molecule(s) in prokaryotic or eukaryotic cells. It also can provide for introduction into host cells and expression of one or more desired proteins from the polynucleotide. The vectors described herein for recombinant paramyxovirus sequences are introduced into eukaryotic cells and propagated under suitable conditions as known to those of skill in the art, and are introduced into animal cells and expressed under suitable conditions as known to those of skill in the art. Infection of animal hosts with recombinant PIV of the invention can be used as a method for expressing a desired protein from a heterologous polynucleotide incorporated into a recombinant PIV genome or antigenome according to the invention.

The term "replicable vector," as used herein, refers to a nucleic acid molecule capable of transporting another nucleic acid to which it has been linked into a cell and providing for amplification of the nucleic acid. One type of vector is a "plasmid", which refers to a circular double stranded DNA loop into which additional DNA segments may be ligated. Another type of vector is a phage vector. Another type of vector is a viral vector, wherein additional nucleic acid segments may be ligated into the viral genome. Certain vectors are capable of autonomous replication in a host cell into which they are introduced (e.g., bacterial vectors having a bacterial origin of replication and episomal mammalian vectors). Recombinant PIV of the present invention, when used with permissive host cells or when they infect a permissive host, are of this class of vectors, provided that the recombinant PIV particle or sub-viral particle includes at least a P, N and L protein in addition to a recombinant vector genome or antigenome. Other vectors (e.g., non-episomal mammalian vectors) can be integrated into the genome of a host cell upon introduction into the host cell, and thereby are replicated along with the host genome. In some embodiments, the vector is a vector that can replicate to high copy number in a cell.

The term "shut-off temperature" refers to a temperature at which the reduction of virus titer compared to its titer at a reference temperature is 100-fold greater than the reduction of wild type virus at the same temperature. In some embodiments, the reference temperature is about 32°C, and the shutoff temperature is about 39°C, more preferably 38°C or 37°C. A determination of the shut off temperature allows a comparison of the temperature sensitivity of different virus strains or isolates and is often indicative of the level of attenuation. The lower the shutoff temperature the higher the level of attenuation of the paramyxovirus isolate or strain.

The term "transfection" as used herein refers to introducing DNA into a eukaryotic cell so that the DNA is replicable and/or expressed, either as an extrachromosomal element or by chomosomal integration. Depending on the host cell used, transfection is done using standard techniques appropriate to such cells. Methods for transfecting eukaryotic cells include polyethyleneglycol/DMSO, liposomes, electroporation, and electrical nuclear transport.

Polypeptide sequences defined herein are represented by one-letter or three letter symbols for amino acid residues as follows:

| | | | |
|---|---|---|---|
| A | ala, alanine | L | leu, leucine |
| R | arg, arginine | K | lys, lysine |
| N | asn, asparagine | M | met, methionine |
| D | asp, aspartic acid | F | phe, phenylalanine |
| C | cys, cysteine | P | pro, proline |
| Q | gln, glutamine | S | ser, serine |
| E | glu, glutamic acid | T | thr, threonine |
| G | gly, glycine | W | try, tryptophan |
| H | his, histidine | Y | tyr, tyrosine |
| I | ile, isoleucine | V | val, valine |

Strategies to generate attenuated viruses are important in the design of safe and stable viral constructs useful in an immunogenic composition. The phenotype of a viral isolate or strain may be modified to achieve a balance between attenuation of viral replication and immunogenicity of the modified variant. In some embodiments, viral replication may be decreased about 100 to 1000 fold and yet still retain immunogenicity. In some embodiments, it is desirable to generate an attenuated virus that has at least one temperature sensitive attenuating mutation and one non- ts attenuating mutation. Attenuated viruses that have more than one mutation and/or more than one phenotype can have enhanced stability.

The C proteins of paramyxoviruses are the collection of accessory proteins named as C', C, Y and Y2. The C proteins are an attractive target for introduction of one or more mutations. C proteins, amongst other functions, inhibit production of α /β interferons and decrease signaling of α /β interferons through their receptors. A paramyxovirus with one or more modified C proteins should have decreased pathogenicity since it may be defective in counteracting host cell interferon response. Replication of paramyxoviruses expressing a C protein with a carboxy terminal deletion has been found to be defective *in vivo* and *in vitro,* including in Vero cells which do not express antiviral interferons α and β (Kato et al., 1997, Embo J., 16(3):578-587; Delenda et al., 1997, Virology, 228(1):55-62; Durbin et al., 1999, Virology, 261(2):319-330; Kawano et al., 2001, Virology, 284(1):99-112; He et al., 2002, Virology, 303(1):15-32; Park et al., 2003, J. Virol., 77(17):9522-9532). The above references describe assays that are useful for assessing C protein function. The C proteins might also have other functions during viral infection, and mutations might also achieve an attenuating effect by interfering with additional functions.

In wild-type HPIV1 and HPIV3, C proteins are encoded by a polycistronic polynucleotide encoding both the P protein and the collection of C proteins. The polycistronic polynucleotide encodes a nucleocapsid-associated P phosphoprotein from an overlapping reading frame (Ohgimoto et al., 1990). P protein is a structural protein that plays a major role in transcription and replication of the viral genome. The alternative reading frames containing the C proteins (C', C, Y1 and Y2) contain several alternative translational start sites. The overlapping nature of the P and C ORFs, however, greatly restricts the number and types of mutations that can be introduced into the C proteins without also affecting the P protein, and vice versa.

One aspect of the disclosure includes polynucleotides, vectors and a viral construct comprising a polynucleotide encoding a P protein and a SSRF polynucleotide encoding one or more C proteins. Separation of the coding sequence of the C proteins and the P protein allows changes to the C proteins without affecting the function of the P protein. Preferably, the polynucleotide encoding the one or more C proteins is modified with at least one mutation that decreases the ability of at least one of the C proteins to inhibit interferon production and/or signaling. The polynucleotide encoding a P protein and the polynucleotide encoding the C protein can be on the same vector or separate vectors.

Another aspect of the disclosure includes polynucleotides, vectors and viral constructs comprising a polynucleotide encoding one or more variant C proteins and a SSRF polynucleotide encoding a P protein. Separation of the coding sequence of the C and P proteins allows changes to the P protein without affecting the function of the C protein. In an embodiment, the polynucleotide encoding the P protein is modified with at least one mutation that destroys the start site of the C and/or C' protein and/or truncates the C, C', Y1 and/or Y2 proteins. The polynucleotide encoding variant C proteins and the SSRF polynucleotide encoding the P protein can be on the same vector or separate vectors.

Another aspect of the disclosure involves a novel attenuating mutation of the L polymerase. In some embodiments, residue positions are selected for substitution based on a comparison to other related viruses and an indication that when an amino acid at the position is substituted in other related viruses an attenuating phenotype is observed. The amino acids selected for substitution at those positions are most often chosen from those amino acids that are encoded by a codon that differs in at least two nucleotide positions from the wild type amino acid found at that position and that confer an attenuation phenotype. Thus, in some embodiments, at least two nucleotide changes are made in a codon specifying the changed amino acid. In some embodiments, mutations of the L polymerase have a temperature sensitive phenotype.

The attenuating mutations and methods of the disclosure provide recombinant, infectious, self-replicating paramyxoviruses comprising a partial or complete polyhexameric genome or antigenome having a SSRF polynucleotide encoding a P protein, which can include attenuating mutations, and a polynucleotide encoding C proteins, as well as additional attenuating mutations in the L polymerase and non-coding portions of the genome. Alternatively, the attenuating mutations and methods of the disclosure provide recombinant, infectious, self-replicating paramyxoviruses comprising a partial or complete polyhexameric genome or antigenome having a polynucleotide encoding at least one variant C protein and a SSRF polynucleotide encoding a P protein, as well as additional attenuating mutations in the L polymerase and perhaps also in non-coding portions of the genome. In some embodiments, an attenuated paramyxovirus has a temperature sensitive mutation and at least one other attenuating mutation that provides a phenotype including host range restriction, reduced plaque size, or change in immunogenicity. The attenuated infectious virus can be utilized in live virus vaccines and/or in immunogenic compositions to protect against HPIV infection and/or to deliver heterologous antigens or to deliver genes for expressing a desired heterologous protein. The attenuating mutations can be utilized as part of a menu of attenuating mutations to develop paramyxovirus strains having effective degrees of attenuation *in vivo* yet retaining sufficient replication *in vitro* that may be utilized in vaccines.

One aspect of the invention provides a recombinant infectious variant of a PIV having one or more attenuating mutations in the L polymerase (L protein). The variant PIV are most often variant of HPIV1 or of HPIV3. In some embodiments, residue positions are selected for substitution based on a comparison to other related viruses and an indication that when an amino acid at a position is substituted in other related viruses an attenuating phenotype is observed. The amino acids selected for substitution at those positions are chosen from those amino acids that are encoded by a codon that differs in at least two nucleotide positions from the wild type amino acid found at that position. In some embodiments, at least two nucleotide changes are made in a codon specifying the changed amino acid.

In some embodiments, an attenuating mutation comprises a substitution at one or more amino acid residues corresponding to positions 456, 942, 992, 1710 or 1711 of SEQ ID NO:13 (wild-type HPIV1 L protein). Preferred amino acid substitutions include F456L, Y942A, Y942T, L992C, and L992N. Preferred deletions include deletion of positions 1710 and 1711.

The attenuating mutations are preferably stable. Amino acid substitutions that require two or three nucleotide substitutions are preferred. For example, Y942A requires three nucleotide substitution mutations (wild type TAT or TAC, variant GCG). The nucleotide substitutions encoding the preferred amino acid substitutions described above are shown in Table 1.

The attenuating mutation(s) can be temperature sensitive. In an embodiment, the L protein mutations are not attenuating for replication at a permissive temperature, such as for example 30-32°C, but are attenuating for replication at restrictive temperatures, such as for example 37°C - 40°C. In an embodiment, replication of the PIV variants is reduced at about 39°C. In some embodiments, the shut-off temperature of the PIV variants is preferably about 39°C, more preferably about 38°C, or about 37°C. Preferably, the paramyxovirus strains with mutations in L polymerase have a lower shutoff temperature than control paramyxovirus. In some embodiments, the control is a wild type virus. In other embodiments, the control is another attenuated paramyxovirus.

Preferably, the PIV variants are attenuated *in vivo.* In an embodiment, the HPIV1 variants exhibit reduced replication in the upper and/or lower respiratory tract of a mammal as compared to wild-type HPIV1. In an embodiment, the HPIV3 variants exhibit reduced replication in the upper and/or lower respiratory tract of a mammal as compared to wild-type HPIV3. In an embodiment, the replication is reduced at least about 10 fold, 100 fold, more preferably about 500 fold, more preferably about 1000 fold, more preferably about 1500 fold, more preferably about 2000 fold, more preferably about 3000 fold, more preferably about 4000 fold, more preferably about 5000 fold, more preferably about 6000 fold as compared to wild-type HPIV1 or HPIV3. In an embodiment, the mammal is a golden Syrian hamster (*Mesocricetus auratus*). In another embodiment, the mammal is an African green monkey (*Cercopithecus aethiops*).

The PIV variants preferably comprise a partial or complete polyhexameric genome or antigenome encoding a major nucleocapsid (N) protein, a nucleocapsid phosphoprotein (P) and a L polymerase (L) protein. PIV variants may further comprise a genome or antigenome encoding a matrix (M) protein, a fusion (F) protein and a hemagglutinin-neuraminidase (HN) protein. PIV variants, and especially HPIV1 or HPIV3 variants, that further comprise F and HN proteins are particularly useful in immunogenic compositions.

Another aspect of the disclosure includes an isolated nucleic acid or vector comprising a polynucleotide encoding a polypeptide with at least 80% sequence identity to an L protein having a sequence of SEQ ID NO: 13 (HPIV1 wt L protein) or of SEQ ID NO:25 (HPIV3 wt L protein). An isolated polypeptide comprising at least 80% sequence identity to a L polypeptide of SEQ ID NO: 13 (HPIV wt L protein) or to SEQ ID NO: 25 (HPIV3 wt L protein) and preferably comprising at least one mutation of amino acid residues corresponding to positions 456, 942, 992, 1710, or 1711 of SEQ ID NO: 13 (wt L protein) is also provided.

An attenuating mutation in the L protein can be generated by standard PCR mutagenesis and molecular cloning techniques using primers of design readily apparent to one of ordinary skill in the art.

One aspect of the invention provides recombinant, infectious, self-replicating PIV comprising a partial or complete polyhexameric genome or antigenome having a polynucleotide encoding a P protein, especially a SURF polynucleotide encoding a P protein, and a second polynucleotide encoding one or more C proteins. The SSRF polynucleotide encoding a P protein has been altered to no longer encode any C protein. Another aspect of the invention provides recombinant, infectious, self-replicating PIV comprising a partial or complete polyhexameric genome or antigenome having a SSRF polynucleotide encoding one or more C proteins and a SSRF polynucleotide encoding a P protein. In such an instance one or more point mutations or minor deletions of one, two or up to ten amino acids, may be introduced into the P and/or C proteins to provide an attenuating phenotype *in vivo* or *in vitro.* The PIV of the invention preferably includes a major nucleocapsid protein (N protein), a nucleocapsid phosphoprotein (P protein), and a large polymerase protein (L protein). PIV variants may further comprise a genome or antigenome encoding a fusion (F) protein and a hemagglutinin-neuraminidase (HN) protein. PIV variants that further comprise F and HN proteins are useful in immunogenic compositions. In some embodiments, the SSRF polynucleotide encoding a wild-type or mutant P protein and the polynucleotide encoding one or more C proteins, which may be mutated, are on separate vectors.

The paramyxovirus is a parainfluenza virus (PIV). A number of paramyxovirus have a polynucleotide encoding both a P and C protein including HPIV1, HPIV3, murine P1V1 (Sendai virus), and measles virus. In an embodiment, the PIV is human PIV (HPIV). Preferably, the HP1V is HPIV type 1 (HPIVI). In an embodiment, the genome or antigenome sequence is derived from a variant HPIV comprising at least 80% or greater nucleotide sequence identity with a HPIV1 reference sequence. In an embodiment, the reference sequence is the genomic sequence of HPIV1. In an embodiment, the genomic sequence comprises a nucleotide sequence of SEQ ID NO:1 (wt HPIV1 genome).

The polynucleotides separately encoding at least one C protein and the P protein can be inserted anywhere into the genome or antigenome of the recombinant PIV of the invention. In some embodiments, a SSRF polynucleotide encoding at least one C protein may be inserted at the 3' end, and a SSRF polynucleotide encoding the P protein may be inserted elsewhere. Thus, in some embodiments, the gene order, for example, may be 3' C- N-P-M-F-HN-L-5'. In other embodiments, the gene order, for example, may he 3' N-C-P-M-F-HN-L-5'. In some embodiments, the SSRF polynucleotide encoding the P protein and the polynucleotide encoding at least one C protein are inserted into restriction sites in the genome. Restriction sites may include *AscI, PacI NheI and NotI,* as shown in Figure 2. In an embodiment, a recombinant virus separately encoding a P and at least one C protein has an antigenomic cDNA sequence that is the complement of SEQ ID NO: 15.

The SSRF polynucleotide encoding a P protein and the polynucleotide encoding at least one C protein are optionally separated by a non-coding polynucleotide spacer sequence. In an embodiment, the spacer sequence is upstream of a C coding sequence or open reading frame (ORF) in the polynucleotide encoding at least one C protein. In another embodiment the polynucleotide encoding at least one C protein and a SSRF polynucleotide encoding a P protein are optionally separated by a non-coding polynucleotide spacer sequence. In an embodiment, the spacer sequence is upstream of a P coding sequence or open reading frame (ORF) in the polynucleotide encoding the P protein. In an embodiment, the spacer sequence comprises a gene end transcription signal, intergenic transcription signal, and/or gene start transcription signal. In an embodiment, the gene start transcription signal is cis-acting. In an embodiment, the gene start transcription signal includes a first adenosine at position 6n+1. In an embodiment, the spacer sequence comprises a nucleotide sequence of SEQ ID NO:9

When a SSRF polynucleotide encoding at least one C protein is inserted into a viral genome, the inserted polynucleotide is a heterologous sequence. Recombinant viruses comprising a SSRF polynucleotide encoding at least one C protein may have an altered phenotype. In some embodiments, the phenotype may be attenuating. In some embodiments, the phenotype is temperature sensitive. Additional nucleotide changes may be introduced into a recombinant virus encoding separate P and C genes to introduce other phenotypic changes selected from a change in growth characteristics, attenuation, temperature sensitivity, cold adaptation, plaque size, host range restriction, or a change in immunogenicity.

Preferably, a recombinant paramyxovirus having separate polynucleotides encoding P and C proteins is attenuated *in vivo.* In an embodiment, the recombinant virus having separate polynucleotides encoding P and C proteins exhibits reduced replication in the upper and/or lower respiratory tract of a mammal as compared to wild-type HPIV1 or wild-type HPIV3. In an embodiment, the replication is reduced at least about 10 fold, 100 fold, more preferably about 500 fold, more preferably about 1000 fold, more preferably about 1500 fold, more preferably about 2000 fold, more preferably about 3000 fold, more preferably about 4000 fold, more preferably about 5000 fold, more preferably about 6000 fold as compared to wild-type HPIV1 or wild-type HPIV3. In an embodiment, the mammal is a golden Syrian hamster (*Mesocricetus auratus*). In another embodiment, the mammal is an African green monkey (*Cercopithecus aethiops*). In some embodiments, the attenuated paramyxovirus is immunogenic and elicits sufficient antibodies to protect against infection.

The SSRF polynucleotide or variant polypeptide encoding at least one C protein can encode a C protein having a sequence of a naturally occurring or variant C protein. The C protein can be from a heterologous paramyxovirus, including but not limited to PIV3 (e.g., human or bovine PIV3, heterologous to HPIV1), HP1V1 (heterologous to HPIV3), Sendai virus, or measles virus. In an embodiment, the nucleotide sequence encoding the C protein has at least 80% sequence identity, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or greater sequence identity to a nucleotide sequence encoding the C protein of HPIV1 (SEQ ID NO: 17 (wild-type HPIV C protein)) or of HPIV3 (SEQ ID NO: 23 (wild-type HPIV3 C protein)). In an embodiment, the antigenomic cDNA sequence encoding the C ORF is the complement of SEQ ID NO:14. In some embodiments, an isolated variant nucleic acid comprising a polynucleotide encoding a polypeptide having at least 80 % sequence identity to a C protein having a sequence of SEQ ID NO:17 (wild type HPIV1 C protein sequence) or to SEQ ID NO: 23 (wild-type HPIV3 C protein sequence) is provided.

In an embodiment, the nucleotide sequence encoding at least one C protein comprises a mutation that inhibits the ability of C protein to interrupt production or signaling of interferon in an infected host or host cell. Preferably, the mutation does not substantially impact virus replication in cell culture. In an embodiment, the C protein has reduced activity as compared to the C protein expressed by wild-type HPIV1 or that expressed by wild-type HPIV3.

One of several approaches can be taken to identify mutations in a C protein that render rHPIV1 C+P attenuated for replication *in vivo.* The first is random mutagenesis of the C ORF to generate viruses that are restricted for replication in the respiratory tract of experimental animals. For example, alanine mutations can be introduced at each position and those positions important in the function of the protein can be identified. In other embodiments, deletions of at least 2 amino acids can be generated. Recombinant viruses bearing these mutations can be characterized *in vitro* and *in vivo.*

Alternatively, sequence alignment with heterologous paramyxovims C proteins can be used as a guide for targeted mutagenesis. For example, there are two ways to use the sequence alignment as a guide. First, conserved sequences, which are likely required for specific C protein activities, can be directly targeted with conservative amino acid substitutions or small (2 amino acid) deletions. A less conservative approach can be taken and unrelated amino acids can be used for amino acid substitutions, or large portions of the conserved regions (6 or more amino acids) can be deleted. Unrelated amino acids may be selected that require at least two nucleotide changes in the codon as compared to the codon encoding the wild type amino acid at that position. The selection of sites for mutagenesis is not limited to conserved sequences.

Substantial modifications in the biological properties of a C protein are accomplished by selecting substitutions that differ significantly in their effect on maintaining (a) the structure of the polypeptide backbone in the area of the substitution, for example, as a sheet confirmation, helical conformation, or loop structure, (b) the charge or hydrophobicity of the molecule at the target site, or (c) the bulk of the side chain. Naturally occurring residues are divided into groups based on common side-chain properties:
(1) hydrophobic: leucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions entail exchanging a member of one of these classes for another class. Such substituted residues also can be introduced into the conservative substitution sites or, more preferably, into the remaining (non-conserved) sites.

In an embodiment, the C' and C proteins comprise one or more amino acid substitutions or deletions at or between residues corresponding to positions 83-85 of the C protein. In an embodiment, all of the C proteins comprise one or more amino acid substitutions or deletions between residues corresponding to positions 169-171 of the C protein. In an embodiment, the C proteins collectively are mutated by one or more amino acid substitutions or deletions at residues corresponding to positions 83-85 and 169-171 of the C protein. Preferably, the nucleotide changes that encode a mutation comprise at least two nucleotide changes compared to the codon encoding the wild type amino acid at that position to increase the stability of the mutation. Preferably, the variant C protein has at least 80% sequence identity to that of the wild-type C protein sequence of HPIV1 or ofHPIV3.

The SSRF polynucleotide encoding a P protein includes a nucleotide sequence encoding a P protein. The SSRF polynucleotide encoding the P protein is altered so that it does not encode a C protein. The amino acid sequence of the P protein may also be a mutated sequence. In an embodiment, the nucleotide sequence encoding P protein exhibits at least 80% sequence identity, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or greater sequence identity to a nucleotide sequence encoding the P protein of HPIV1 (SEQ ID NO: 11 (wild-type P protein of HPIV1) or ofHPIV3 (SEQ ID NO: 22 (wild type P protein sequence of HPIV3). In an embodiment, an antigenomic cDNA sequence encoding a P protein has a sequence that is the complement of SEQ ID NO:10 (wild type P gene of HPIV1).

The variant polynucleotide encoding a P protein includes a P coding sequence or open reading frame (ORF). Preferably the P ORF includes one or more nucleotide substitution(s) that introduce one or more stop codons in an overlapping C ORF but does not alter the amino acid sequence ofP protein encoded by the P ORF. In an embodiment, third base codon substitutions encoding stop codons in the C ORF are introduced (see Fig. 2) into the P ORF (SEQ ID NO:15). In another embodiment, the P ORF comprises a nucleotide sequence of SEQ ID NO:10.

A PIV of the invention or polynucleotide of the disclosure including a SSRF polynucleotide encoding a P protein and a second polynucleotide encoding at least one C protein can be made using known recombinant methods such as oligonucleotide-mediated (site-directed) mutagenesis, alanine scanning, PCR mutagenesis, site-directed mutagenesis (Zoller et al., 1987, Nucl. Acids Res., 10: 6487-6500), cassette mutagenesis (Wells et al., 1985, Gene, 34:315), restriction selection mutagenesis (Wells et al., 1986, Philos. Trans. R. Soc. London SerA, 317:415), and the like. Similarly, a PIV of the invention or polynucleotide of the disclosure including a SSRF polynucleotide encoding a P protein and second polynucleotide encoding at least one C protein that is a SSRF polynucleotide can be made using these same methods.

A PIV of the invention including a partial or complete polyhexameric genome or antigenome having a SSRF polynucleotide encoding a P protein and a second polynucleotide encoding at least one C protein, which is optionally also a SSRF polynucleotide, can include any of the attenuating mutations in the L polymerase described herein or other known attenuating mutations. The polynucleotide encoding at least one C protein can also include further attenuating point mutations or deletions. Likewise, an isolated polynucleotide of the disclosure including a partial or complete polyhexameric genome or antigenome having a polynucleotide, which is optionally a SSRF polynucleotide, encoding at least one C protein and a SSRF polynucleotide encoding a P protein, can include any of the attenuating mutations in the L polymerase described herein or other known attenuating mutations. In such embodiments, the polynucleotide encoding at least one C protein can also include further attenuating point mutations or deletions.

Recombinant viral variants having more than one mutation are likely to have increased stability over those variants having a single mutation or a small number of mutations (eg., less than 3 mutations). The PIV of the invention can also include any of the attenuating L polymerase mutations described in WO 04/027037 or in WO 98/53078. The attenuating effect of the mutations in the L polymerase are additive and serve to further increase the attenuation of the PIV of the invention including a SSRF polynucleotide encoding a P protein and a second polynucleotide encoding at least one C protein. In some embodiments, it is desirable to balance the level of attenuation with the immunogenicity. In preferred embodiments, the variant paramyoviruses have about 100 fold to 5000 fold decrease in viral titer in a mammal. In some embodiments, a decrease of viral replication greater than about 100,000 fold *in vivo* may result in a loss of immunogenicity. Such a high degree of attenuation *in vitro* may result in an inability to produce the virus on a large scale for vaccine production.

In some embodiments, the variant parainfluenzaviruses of the invention have at least one temperature sensitive mutation and at least one non-temperature senstitive mutation. In an embodiment, the recombinant variant parainfluenzaviruses of the invention have at least one temperature sensitive mutation due to insertion of a SSRF polynucleotide encoding at least one C gene or due to a mutation in a polynucleotides encoding a L polymerase, wherein the change in the amino acid is due to at least two nucleotide changes to the codon of the L polymerase encoring the wild type amino acid. In another embodiment, the recombinant variant parainfluenzaviruses of the invention comprise a mutation that provides a host range restriction phenotype.

The parainfluenzaviruses of the invention are also useful as vectors for expressing heterologous antigens in an immunogenic composition. One or more supernumerary genes encoding one or more heterologous polypeptides can be cloned into and expressed by the PIV of the invention. For example, an immune response against multiple PIV serotypes or strains can be elicited by engineering protective epitopes of multiple PIV serotypes and strains into a single paramyxovirus. The supernumerary genes can be cloned and expressed in a recombinant virus encoding separate P and C proteins as described herein, which may optionally include one or more mutations in L polymerase. Insertion of additional (heterologous genes may also result in an attenuated phenotype. Preferably, the paramyxovirus comprising a polynucleotide encoding a heterologous gene is attenuated about 100 to 5000 fold or more in a cell or mammal.

In an embodiment, the genome or antigenome includes one or more heterologous genes or genome segments encoding one or more antigenic determinants of a heterologous pathogen. For example, one or more heterologous antigenic determinant(s) from PIV, measles virus, subgroup A and subgroup B respiratory syncytial viruses, mumps virus, human papilloma viruses, type 1 and type 2 human immunodeficiency viruses, herpes simplex viruses, cytomegalovirus, rabies virus, Epstein Barr virus, filoviruses, bunyaviruses, flaviviruses, alphaviruses, human metapneumoviruses, or influenza viruses can be expressed by the PIV of the invention. Examples of useful antigenic determinants include, but are not limited to, measles virus HA and F proteins, subgroup A or subgroup B respiratory syncytial virus F and G proteins, mumps virus HN and F proteins, human papilloma virus L1 protein, type 1 or type 2 human immunodeficiency virus gp160 protein, herpes simplex virus and cytomegalovirus gB, gC, gD, gE, gG, gH, gI, gJ, gK, gL, and gM proteins, rabies virus G protein, Epstein Barr Virus gp350 protein, filovirus G protein, bunyavirus G protein, flavivirus preM and E proteins, human metapneuomovirus (HMPV) G and F proteins, and alphavirus E protein, and antigenic domains, fragments and epitopes thereof.

In an embodiment, a polynucleotide encoding an open reading frame (ORF) of a measles virus HA gene is incorporated into a HPIV1 vector genome or antigenome to yield a chimeric candidate useful to immunize against measles and/or HPIV1 or another HPIV. In another embodiment, a polynucleotide comprising genes or genome segments encoding one or more heterologous PIV(s) (e.g., HPIV2, HPIV3, and/or HPIV4) N, P, V, F, HN and/or L protein(s) or fragment(s) thereof is incorporated into a HPIV1 vector genome or antigenome. In another embodiment, one or more supernumerary heterologous gene(s) or genome segment(s) selected from HPIV1 HN, HPIV1 F, HPIV2 HN, HPIV2 F, HPIV3 HN, HPIV3 F, measles HA and F, HMPV G and F proteins, and/or RSV subgroup A or B G and F proteins are cloned into a PIV of the invention.

Some methods of inserting one or more supernumerary genes or transcriptional units into a paramyxovirus viral genome or antigenome are described in WO04/027037. Supernumerary heterologous gene(s) or genome segment(s) can be inserted at various sites within the recombinant genome or antigenome, for example at a position 3' to N, between the N/P, P/M, and/or HN/L genes, or at another intergenic junction or non-coding region of a HPIV1 vector genome or antigenome. Preferably, the heterologous or supernumery gene or transcriptional unit is inserted at a restriction site, for example, *AscI, PacI, NheI and NotI.* Any insertions or deletions of the viral genome, preferably, conform to the rule of six.

The infectious PIVs of the invention and polynucleotides of the disclosure are produced by synthetic and recombinant methods. Accordingly, the disclosure relates to polynucleotides encoding infectious PIV clones of the invention and host cells including the infectious clone, as well as methods of making such vectors and host cells by recombinant methods.

The PIV of the invention or polynucleotides of the disclosure may be synthesized or prepared by techniques well known in the art. See, for example, WO 94/027037. Nucleotide sequences for wild type paramyxovirus genomes are known and readily available, for example, on the Internet at GenBank (accessible via the internet). The nucleotide sequences encoding the PIV of the invention may be synthesized or amplified using methods known to those of ordinary skill in the art including utilizing DNA polymerases in a cell free environment.

Amino acid substitutions, insertions, and deletions can be made using known recombinant methods such as oligonucleotide-mediated (site-directed) mutagenesis, alanine scanning, PCR mutagenesis, site-directed mutagenesis (Zoller et al., 1987, Nucl. Acids Res., 10: 6487-6500), cassette mutagenesis (Wells et al., 1985, Gene, 34:315), restriction selection mutagenesis (Wells et al., 1986, Philos. Trans. R. Soc. London SerA, 317:415), and the like.

The PIV of the invention can be produced from virus isolated from biological samples. The polynucleotides and vectors may be produced by standard recombinant methods known in the art, such as polymerase chain reaction (Sambrook, et al., 1989, Molecular Cloning, A Laboratory Manual, Vols. 1-3, Cold Spring Harbor Press, Cold Spring Harbor, NY). Methods of altering or modifying nucleic acid sequences are also known to those of skill in the art.

The paramyxovirus genome may be assembled from polymerase chain reaction cassettes sequentially cloned into a vector including a selectable marker for propagation in a host. Such markers include dihydrofolate reductase or neomycin resistance for eukaryotic cell culture and tetracycline or ampicillin resistance genes for culturing in *E. coli* and other bacteria.

The polynucleotide may be inserted into a replicable vector for cloning using standard recombinant methods. Various vectors are publicly available. The vector may, for example, be in the form of a plasmid, cosmid, viral particle, or phage. The appropriate nucleic acid sequence may be inserted into the vector by a variety of procedures. In general, a nucleic acid is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors including one or more of these components employs standard ligation techniques that are known to the skilled artisan.

Examples of suitable replicable vectors include, without limitation, pUC 19 or pTM1. The polynucleotide can be operably linked to an appropriate promoter such as, for example, T7 polymerase promoter, cytomegalovirus promoter, cellular polymerase II promoter, or SP1 promoter. The replicable vectors may further include sites for transcription initiation, transcription termination, and a ribosome binding site for translation.

In an embodiment, an isolated polynucleotide of the disclosure, comprising a polynucleotide of a partial or complele PIV genome or antigenome and including a SSRF polynucleotide encoding a P protein and a second polynucleotide, optionally also a SSRF polynucleotide, encoding at least one C protein are cloned by introducing unique restriction enzyme recognition sequences into paramyxovirus cDNA such that the recognition sequences flank the bicistronic polynucleotide encoding the P/C proteins, digesting the genome with one or more restriction enzymes that cut the genome at the restriction sites flanking the bicistronic polynucleotide encoding P/C proteins, inserting the SSRF polynucleotide encoding the P protein and /or a second polynucleotide, optionally also a SSRF polynucleotide, encoding at least one C protein at the cleaved restriction sites, and religating the genome. Examples of suitable restriction enzyme recognition sequences include but are not limited to, *AscI, PacI, NheI and NotI.* In some embodiments, the restrictions sites are introduced into the non-coding regions upstream or downstream of the bicistronic P/C ORFs. In an embodiment, an *AscI* site is upstream of the bicistronic P/C ORFs and the *NotI* site is downstream of the bicistronic P/C ORFs (Figure 2). In an embodiment, the variant polynucleotide encoding a P protein is introduced into the genome using the *PacI, and NheI* restriction sites. In an embodiment, the monocistronic polynucleotide encoding a C protein is introduced into the genome using the *AscI and NotI* restrictions sites.

In an embodiment, a PIV of the invention including a variant polynucleotide, which is optionally a SSRF polynucleotide, encoding at least one C protein and a SSRF polynucleotide encoding a P protein are cloned by introducing unique restriction enzyme recognition sequences into PIV cDNA such that the recognition sequences flank the bicistronic polynucleotide encoding the P/C proteins, digesting the genome with one or more restriction enzymes that cut the genome at the restriction sites flanking the bicistronic polynucleotide encoding P/C proteins, inserting the polynucleotide encoding at least one C protein and/or a SSRF polynucleotide encoding a P protein at the cleaved restriction sites, and religating the genome. Examples of suitable restriction enzyme recognition sequences, include but are not limited to, *AscI, PacI, NheI and NotI*. In some embodiments, the restrictions sites are introduced into the non-coding regions upstream or downstream of the bicistronic P/C ORFs. In an embodiment, an *AscI* site is upstream of the bicistronic P/C ORFs and the *NotI* site is downstream of the bicistronic P/C ORFs (Figures 2). In an embodiment, the polynucleotide encoding at least one C protein is introduced into the genome using the *AscI* and *NotI* restriction sites. In an embodiment, the SSRF polynucleotide encoding a P protein is introduced into the genome using the *Pac*I *and Nhe*I restrictions sites.

Introduction of a recombinant vector composed of a paramyxovirus genome or polynucleotide encoding a paramyxovirus protein into a host cell, such as for example a bacterial cell or eukaryotic cell, can be affected by calcium phosphate transfection, DEAE-dextran mediated transfection, cationic lipid-mediated transfection, electroporation, electrical nuclear transport, chemical transduction, electrotransduction, infection, or other methods. Such methods are described in standard laboratory manuals such as Sambrook, et al., 1989, Molecular Cloning, A Laboratory Manual, Vols. 1-3, Cold Spring Harbor Press, Cold Spring Harbor, NY or Davis et al., 1986, Basis Methods in Molecular Biology*.* Commercial transfection reagents, such as Lipofectamine (Invitrogen, Carlsbad, CA) and FuGENE 6™ (Roche Diagnostics, Indianapolis, IN), are also available. In some embodiments, transfection efficiency of the host cells is about 15% or greater, about 20% or greater, about 30% or greater, about 40% or greater, or about 50% or greater. Suitable host cells include, but are not limited to BHK-T7 cells, HEp-2 cells, FRhL-DBS2 cells, LLC-MK2 cells, MRC-5 cells, and Vero cells.

The invention provides isolated, infectious, recombinant PIV including one or more attenuating mutations for use in immunogenic compositions, including live attenuated virus vaccines. The PIVS of the invention are useful in immunogenic compositions for eliciting an immune response in a mammal. The attenuated PIV includes a SSRF polynucleotide encoding a P protein and a second polynucleotide, which is optionally also a SSRF polynucleotide, encoding at least one C protein. In an embodiment, the C gene comprises a nucleotide sequence encoding at least one C protein having a mutation that inhibits the ability of the C protein to interrupt production and /or signaling of interferon in an infected host or alter another function of C that results in attenuation. In an embodiment, the attenuated virus is HPIV1.

Recombinant HPIV1 of the invention can be combined with viruses of other PIV serotypes or strains and paramyxoviruses from multiple genera in a composition to elicit an immune response against multiple genera, serotypes, and strains. The immunogenic composition can comprise paramyxoviruses from two or more serotypes. In an embodiment, at least one of the serotypes is HPIV1, HPIV2, HPIV3, or HPIV4. The immunogenic composition can comprise paramyxovirus from two or more strains. In an embodiment, at least one of the strains is an HPIV1. The immunogenic composition can comprise paramyxovirus from two of more genera. In an embodiment, one genus is Respirovirus genus.

The PIVs of the invention are also useful as vectors for expressing heterologous antigens in an immunogenic composition. One or more supernumerary genes encoding one or more heterologous polypeptides can be cloned into and expressed by the PIV of the invention. For example, an immune response against multiple PIV serotypes or strains can be elicited by engineering protective epitopes of multiple PIV serotypes and strains into a single paramyxovirus. In an embodiment, the genome or antigenome includes one or more heterologous genes or genome segments encoding one or more antigenic determinants of a heterologous pathogen.

For example, one or more heterologous antigenic determinant(s) from PIN1, PIV2, PIV3, PIV4, measles virus, subgroup A and subgroup B respiratory syncytial viruses, mumps virus, human papilloma viruses, type 1 and type 2 human immunodeficiency viruses, herpes simplex viruses, cytomegalovirus, rabies virus, Epstein Barr virus, filoviruses, bunyaviruses, flaviviruses, alphaviruses, human metapneumovituses, or influenza viruses can be expressed by the PIV of the invention. Examples of useful antigenic determinants include, but are not limited to, PIV2, 3, or 4 HN and F proteins, measles virus HA and F proteins, subgroup A or subgroup B respiratory syncytial virus F and G proteins, mumps virus HN and F proteins, human papilloma virus L1 protein, type 1 or type 2 human immunodeficiency virus gp160 protein, herpes simplex virus and cytomegalovirus gB, gC, gD, gE, gG, gH, gI, gJ, gK, gL, and gM proteins, rabies virus G protein, Epstein Barr Virus gp350 protein, filovirus G protein, bunyavirus G protein, flavivirus preM and E proteins, human metapneuomovirus (HMPV) G and F proteins, and alphavirus E protein, and antigenic domains, fragments and epitopes thereof.

In an embodiment, a polynucleotide encoding an open reading frame (ORF) of a measles virus HA gene is incorporated into a HPIV1 vector genome or antigenome to yield a chimeric candidate useful to immunize against measles and/or HPIV1 or another HPIV. In another embodiment, a polynucleotide comprising genes or genome segments encoding one or more heterologous PIV(s) (*e.g*., HPIV2, HPIV3, and/or HPIV4) N, P, C, V, F, HN and/or L protein(s) or fragment(s) thereof is incorporated into a HPIV1 vector genome or antigenome. In another embodiment, one or more supernumerary heterologous gene(s) or genome segment(s) selected from HPIV2 HN, HPIV2 F, HP1V3 HN, HPIV3 F, measles HA and F, HMPV G and F proteins, and/or RSV subgroup A or B G and F proteins are cloned into a PIV of the invention.

The recombinant PIV of the invention can also incorporate one or more antigenic determinants of a non-viral pathogen such as a bacterium or a parasite. Also, the host immune response can be affected by including in the recombinant PIV of the invention a gene encoding an immunomodulatory molecule, such as an interleukin or colony stimulating factor, or any other chemokine or cytokine.

The isolated nucleic acids of the disclosure and recombinant PIV of the invention can also serve as vectors for introducing heterologous genes (to PIV) into a mammalian host cell or animal for expression of a desired gene.

Some methods of inserting one or more supernumerary genes or transcriptional units into a paramyxovirus viral genome or antigenome are described in WO04/027037. Supernumerary heterologous gene(s) or genome segment(s) can be inserted at various sites within the recombinant genome or antigenome, for example at a position 3' to N, between the N/P, P/M, and/or HN/L genes, or at another intergenic junction or non-coding region of a HPIV1 vector genome or antigenome.

Candidate viruses for use in an immunogenic composition, such as for example a vaccine, are selected based on their attenuation and immunogenicity. These vaccine selection criteria are determined according to well-known methods. Preferably, candidate viruses have a stable attenuation phenotype, exhibit replication in an immunized host, and effectively elicit production of an immune response in a recipient, preferably a protective immune response. Preferably, the candidate viruses stimulate and expand the immune response, e.g., induce an immune response against different viral strains or subgroups and/or stimulate an immune response mediated by a different immunologic basis (e.g. secretory versus serum immunoglobulins, cellular immunity, and the like).

Recombinant PIVs of the invention can be tested in well-known and *in vitro* and *in vivo* models to confirm adequate attenuation, resistance to phenotypic reversion, and immunogenicity. In *in vitro* assays, the modified virus PIV of the invention is tested for one or more desired phenotypes, such as, for example, temperature sensitive replication. PIV of the invention can also be tested in animal models of PIV infection. A variety of animal models are known. For example, PIV model systems, including rodents and non-human primates, for evaluating attenuation and immunogenic activity of PIV vaccine candidates, are known, and the data obtained therefrom are known to correlate with PIV infection, attenuation, and immunogenicity in humans.

In some embodiments, recombinant variant paramyxoviruses have at least one attenuating mutation with a is phenotype and at least one mutation with a non-ts phenotype. The recombinant attenuated paramyxoviruses are preferably attenuated about 100 to 5000 fold in a cell or mammal compared to wild type paramyxovirus. In some embodiments, attenuation of greater than 100,000 fold may result in reduced immunogenicity. In some embodiments, it is preferred that the level of viral replication in vitro is sufficient to provide for production of viral vaccine for use on a wide spread scale. In some embodiments, it is preferred that the level of viral replication of attenuated paramyxovirus in vitro is at least 10⁶, more preferably at least 10⁷, and most preferably at least 10⁸ per ml. The attenuating mutation is preferably one that is stable. For example, for mutations in L polymerase, it is preferable that a change in amino acid at a position requires at least two nucleotide changes in the codon as compared to the codon encoding the wild type amino acid at that position. A recombinant paramyxovirus with at least two, three, four or even more attenuating mutations is likely to be more stable. Insertion of a supernumerary gene whose total length conforms to the rule of six, such as a polynucleotide encoding a separate C gene, can also provide a stable phenotype.

Immunogenicity of a recombinant attenuated paramyxovirus can be assessed in an animal model by determining the number of animals that form antibodies to the paramyxovirus after one immunization or after a second immunization. In some embodiments, a recombinant paramyxovirus has sufficient immunogenicity of about 60 to 80 % of the animals develop antibodies after the first immunization and about 80 to 100% of the animals develop antibodies after the second immunization. The preferred animal for a determination of immunogenicity is African green monkey. Preferably, the immune response protects against infection with a paramyxovirus of the same strain or multiple strains.

The disclosure also provides for immunogenic compositions comprising isolated polynucleotides or polypeptides of the disclosure. For example, an immunogenic composition can include a polynucleotide encoding a polypeptide that has at least 80% sequence identity to a C polypeptide having a sequence of SEQ ID NO:17 (wild-type HPIV1 C protein) or a polypeptide having a sequence at least 80% sequence identity to a C polypeptide having an sequence of SEQ ID NO:23 (wild-type HPIV3 C protein). In other embodiments, an immunogenic composition can include a polynucleotide encoding a polypeptide that has at least 80% sequence identity to a L polypeptide having a sequence of SEQ ID NO:13 (wild type HPIV1 L protein) or a polypeptide having a sequence at least 80% sequence identity to a L polypeptide having an sequence of SEQ ID NO:25 (wild type HPIV3 L protein). In other embodiments, an immunogenic composition can include a nucleic acid comprising a polynucleotide of SEQ ID NO:13 or SEQ ID NO:25.

Recombinant PIVs of the invention are preferably present in the immunogenic composition in an immunogenic effective amount. An immunogenic effective amount is an amount of recombinant paramyxovirus that induces an immune response in an animal. The actual amount of the recombinant paramyxovirus may vary depending on the animal to be immunized, the route of administration and adjuvants. The actual amount of recombinant paramyxovirus necessary to elicit an immune response, and the timing and repetition of administration, can be determined using conventional methods based on the state of health and weight of the host, mode of administration, nature of formulation, etc. Immunogenic dosages can be determined by those of skill in the art. Dosages will generally range from about 10³ to about 10⁷ infectious units (e.g., plaque forming units or 50% tissue culture infectious dose) or more of virus per host, more commonly from about 10⁴ to 10⁶ infectious doses of virus per host. In any event, the formulations should provide a quantity of attenuated recombinant PIV the invention sufficient to effectively stimulate or induce an anti-PIV or other anti-pathogen immune response.

The immune response may be indicated by T and/or B cell responses. Typically, the immune response is detected by the presence of antibodies that specifically bind to a particular antigen. Methods of detecting antibodies to a particular antigen are known to those of skill in the art and include such assays as ELISA assays, western blot assays, hemagglutination-inhibition assays, and infectivity neutralization assays. Host receiving immunogenic compositions of the disclosure are preferably monitored for signs and symptoms of upper and lower respiratory tract illness. Preferably, attenuated virus administered intranasally grows in the nasopharynx of recipients at levels about 10-fold or more lower than wild-type virus, or about 10-fold or more lower when compared to levels of incompletely attenuated virus.

In neonates and infants, multiple administrations may be required to elicit sufficient levels of immunity. Administration could begin within the first month of life, and at intervals throughout the first several years of childhood, such as at two months, six months, one year and two years, as necessary to maintain an immune response against native (wild-type) PIV infection. Similarly, adults who are particularly susceptible to repeated or serious PIV infection, such as, for example, health care workers, day care workers, family members of young children, the elderly, individuals with compromised cardiopulmonary function, may require multiple immunizations to establish and/or maintain immune responses. Levels of induced immunity can be monitored by measuring amounts of neutralizing secretory and serum antibodies, and dosages adjusted or immunizations repeated as necessary to maintain desired levels of immune response.

Recombinant PIVs of the invention and polynucleotides, and polypeptides of the disclosure can be used directly in formulations, or lyophilized, as desired, using well known methods. Lyophilized virus is typically maintained at about 4°C. When ready for use, the lyophilized virus is reconstituted in an appropriate stabilizing solution. Many stabilizing solutions are known. Immunogenic compositions including PIV of the invention can include a physiologically acceptable carrier and/or adjuvant. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™ polyethylene glycol (PEG), and PLURONICS™. Lyophilized preparations are generally combined with a sterile solution prior to administration.

The compositions may include pharmaceutically acceptable auxiliary substances as required to approximate physiological conditions, such as pH adjusting and buffering agents, tonicity adjusting agents, wetting agents and the like, such as for example, sodium acetate, sodium lactate, sodium chloride, potassium chloride, calcium chloride, sorbitan monolaurate, triethanolamine oleate, and the like. Acceptable adjuvants include, but are not limited to, Freund's adjuvant (incomplete or complete), MPLTM (3-0-deacylated monophosphoryl lipid A; Corixa, Hamilton IN) and IL-12 (Genetics Institute, Cambridge MA), CpG oligonucleotides, immunostimulating compositions and alum salts.

The immunogenic compositions of the disclosure can be administered nasally in droplet, aerosol, or nebulizer form, orally, or parentally, including subcutaneous injection, intravenous, intramuscular, intrasternal or infusion techniques, in dosage unit formulations including conventional non-toxic pharmaceutically acceptable carriers, adjuvants or vehicles. Compositions of the disclosure can be in the form of suspensions or tablets suitable for oral administration or sterile injectable preparations, such as sterile injectable aqueous or oleagenous suspensions.

For administration as injectable solutions or suspensions, the immunogenic compositions of the disclosure can be formulated according to techniques well-known in the art, using suitable dispersing or wetting and suspending agents, such as sterile oils, including synthetic mono- or diglycerides, and fatty acids, including oleic acid.

Immunization by the nasal route may be more effective compared with intramuscular or subcutaneous injection because the production of local secretory IgA in the upper respiratory tract can protect against PIV infection. For example, the increased immunity provided by PIV specific secretory IgA can result in a broader cross-reactivity for variant strains of PIV and thus may offer a greater degree of protection against mutant PIV. In contrast, injectable vaccines are inefficient at inducing mucosal IgA. In particular, nasal administration of the immunogenic compositions of the disclosure may be more effective in the elderly since, unlike the systemic immune system, mucosal immune responses do not deteriorate with age. Immunogenic compositions of the disclosure that also stimulate systemic immune responses may protect the lower respiratory tract (lungs) due to transudation of antibodies from the serum. In addition, PIV-specific cytotoxic T cells (CTL) in nasal associated lymphoid tissue can contribute to recovery from infection.

Immunogenic compositions for nasal administration are preferably formulated so that they are similar to nasal secretions in regard to toxicity, pH, and viscosity so that normal ciliary action is maintained. In an embodiment, the immunogenic compositions of the disclosure are formulated in an aqueous solution that is isotonic and slightly buffered to maintain a pH of about seven. Antimicrobial preservatives and appropriate stabilizers, if required, are included in the formulation.

The disclosure also provides methods of making and using the recombinant PIV of the invention. In one embodiment, the disclosure relates to methods of generating an infectious, self-replicating paramyxovirus as have been described herein. The methods generally include the steps of removing or altering a polycistronic polynucleotide encoding a P and C protein from the viral genome or antigenome of a paramyxovirus and inserting a SSRF polynucleotide encoding a P protein and/or a second polynucleotide, which optionally is also a SSRF polynucleotide, encoding at least one C protein. In an alternative embodiment, the variant polynucleotide encoding the P gene in the paramyxovirus can be altered *in situ* to no longer encode any C protein using the standard methods and the second polynucleotide encoding at least one C protein can be inserted into the paramyxovirus genome or antigenome.

In another embodiment, the methods generally include the steps of removing or altering a polycistronic polynucleotide encoding a P and C protein from the viral genome or antigenome of a paramyxovirus and inserting a SSRF polynucleotide encoding a C protein and/or a SSRF polynucleotide encoding a P protein. Tn an alternative embodiment, the polynucleotide encoding the C gene in the paramyxovirus can he altered *in situ* to no longer encode the P protein using the standard methods and the SSRF polynucleotide encoding the P protein can be inserted into the paramyxovirus genome or antigenome.

One or more of the C proteins optionally has at least one mutation that reduces the ability of the C protein to inhibit production of interferon, interferon signaling, or other C functions.

In an embodiment, the removing step includes introducing unique restriction enzyme recognition sequences into the genome or antigenome such that the recognition sequences flank the polycistronic polynucleotide encoding P and C proteins and digesting the genome or antigenome with one or more restriction enzymes that cut the genome or antigenome at the restriction sites flanking the polycistronic polynucleotide. In an embodiment, the inserting step includes inserting the SSRF polynucleotide encoding a P protein and a second polynucleotide encoding at least one C protein at the cleaved restriction sites and religating the genome or antigenome. In another embodiment, the inserting step includes inserting a SSRF polynucleotide encoding a C protein and a SSRF polynucleotide encoding a P protein at the cleaved restriction sites and religating the genome or antigenome.

In another embodiment, the disclosure relates to methods of producing infectious, self-replicating, recombinant paramyxovirus. The methods of the disclosure include transfecting a population of cells with an expression vector comprising a partial or complete polyhexameric genome or antigenome sequence and one or more supporting vectors including one or more polynucleotides encoding N protein, P protein, and L polymerase, and incubating the transfected cells under conditions to allow for viral replication. The paramyxoviruses have a SSRF polynucleotide encoding a P protein and/or a second polynucleotide encoding at least one C protein.

In another embodiment, the disclosure relates to methods of eliciting an immune response in a mammal. The methods of the disclosure include administering an immunogenic composition of the disclosure Preferably, the immune response produces antibodies that are protective (e.g. inhibit infection or reduce the severity of infection). In an embodiment, the antibodies are anti-PIV antibodies. In an embodiment, the anti-PIV antibodies are IgA. In some embodiments, the immune response produces antibodies that bind one or more antigenic determinants of a heterologous pathogen encoded by supernumerary genes or genome segments. Examples of heterologous pathogens include but are not limited to, HPIV1 (heterologous to HPIV3), HPIV2, HPIV3 (heterologous to HPIV1), measles virus, subgroup A or subgroup B respiratory syncytial virus, mumps virus, human papilloma virus, type 1 or type 2 human immunodeficiency virus, herpes simplex virus, cytomegalovirus, rabies virus, Epstein Barr virus, filovirus, bunyavirus, flavivirus, alphavirus, human metapneurnovirus, or influenza virus. In an embodiment, the antigenic determinants include measles virus HA, HPIV1 HN or F, HPIV2 HN or F, and/or HPIV3 HN or F.

In another embodiment, the disclosure relates to methods of inhibiting a paramyxovirus infection including, but not limited to, PIV infection, including HPIV1 or HPIV3 infection. The methods of the disclosure include administering an immunogenic composition of the disclosure comprising an attenuated PIV of the invention. The paramyxovirus used in this aspect of the disclosure includes a SSRF polynucleotide encoding a P protein and a second polynucleotide, which is optionally also a SSRF polynucleotide, encoding at least one C protein. In an embodiment, the paramyxovirus is PIV. In an embodiment, the PIV is HPIV1 or HPIV3. Preferably the immunogenic composition elicits antibodies that are protective (e.g. inhibit infection or reduce the severity of infection). In an embodiment, the antibodies are anti-PIV antibodies. In an embodiment, the anti-PIV antibodies are IgA.

Different embodiments of the invention may display different aspects of the invention. Not every embodiment will exhibit every advantage of the invention.

The following examples are provided for illustrative purposes only, and are in no way intended to limit the scope of the present invention.

### Example 1: Cloning Strategy for the Production of the pFLC-rHPIV1-C+P cDNA plasmid:

### Separating the overlapping open reading frames (ORFs) of the PlC gene of HPIV1 into two separately transcribed gene units.

The starting point in constructing pFLC-rHPIV1-C+P was the consensus sequence of the wild type (wt) HPIV1 strain Washington/20993/1964 (Genbank accession # AF457102). A recombinant full-length DNA clone (FLC) of this HPIV1 was made and, using site-directed mutagenesis, 3 additional unique restriction enzyme sites were introduced: *MluI* (ACGCGT, pre-N ORF position, nucleotide numbers 113-118), *AscI* (GGCGCGCC, pre-P/C ORF position, nucleotide numbers 1801-1808) and *Notl* (GCGGCCGC, post-P/C ORF position, nucleotide numbers 3634-3641). In addition this clone contained a single point mutation in the HN gene, HN^{T553A}, which has been previously described [Bartlett, E.J. 2006. Vaccine. 24:2674-2684]. This cDNA clone was termed pFLC-rHPIV1-*MluI*/*AscIlNotI,* and was used to prepare pFLC-rHPIV1-C+P. Whereas HPIV1 expresses the P and C proteins from overlapping ORFs in a single mRNA (Figure 1), in pFLC-rHPIV1-C+P these two overlapping ORFs have been separated into two gene units that express P and C proteins separately from two individual mRNAs (Figure 2). Standard recombinant DNA methods were used to construct the two new gene units, one designed to express the C accessory proteins (C', C, Y1 and Y2) but not the P protein, and the other to express the P protein but not the C proteins. The full-length viral cDNA was designed to conform to the rule of six. In the description below, note that all nucleotide (nt) sequence positions are based on the sequence of the fully assembled pFLC-rHPIV1-C+P HN^{T553A} sequence provided in the accompanying sequence listing (SEQ ID NO:2).

### Assembly of the C(P-) Gene Unit

The C gene unit was produced first (Gene Unit 1 of Figure 2) and was designed to incorporate changes which would enable the C proteins but not the P protein to be expressed from this gene unit. The incorporated changes would not affect the amino acid sequence of the C proteins, i.e., these changes would be silent in the C ORF. In addition, this clone contained a single point mutation in the HN gene, HN T553A, which has been previously described and does not on its own confer a significant *att* or *ts* phenotype [Bartlett, E.J. et al., Vaccine 24:2674-2684 (2006)]. The cDNA was PCR amplified using primers containing the *AscI* (3' end) and *NotI* (5' end) restriction enzyme sites on each end to enable cloning into the pFLC-rHPIV1-*MluIlAscIlNotI* vector*.* Features incorporated into the C gene unit cDNA included:
1. The PCR was designed to amplify the C ORF and to truncate the P/C gene 25 nt after the C stop codon. It also placed *PacI, NheI,* and *NotI* sites in tandem succession on the downstream end. The *PacI* and *NheI* sites would later be used to accept gene junction sequence and P gene unit sequence (see **Assembly of the P(C-) Gene Unit**, below) and in the final pFLC-rHPIV1-C+P HN^{T553A} construct would have the following sequence positions: *PacI (nt #2492-2499), Nhel (nt #4245-4250).*
2. Interruptions to the P open reading frame (ORF) were introduced such that the P protein would not be expressed from the C gene unit. These changes would be silent in the C ORF. This included:
   - Conversion of the P Start codon (ATG → GTG)
      *Nt #1844-1846, pFLC-rHPIV1-C+P HNT553A cDNA* (Sequence file #:4).
   - Stop codons inserted 2 and 5 codons after the P start codon.
      *Nt* #*1850-1852, pFLC-rHPIV1-C+P HNT553A cDNA* (Sequence file #:4).
      *Nt* #*1859-1861, pFLC-rHPIV1-C+P HNT553A cDNA* (Sequence file #:4).
   - The above-mentioned truncation of the P/C gene 25 nt after the C stop codon, which removed much of the P ORF.

This C(P-) gene unit was cloned into the pFLC-rHPIV1-HN^{T553A} cDNA at the unique *AscI* and *NotI* restriction enzyme sites using standard molecular cloning techniques. The resulting cDNA clone was termed pFLC-rHPIV1-C(P-) HN^{T553A}.

### Assembly of the P(C-) Gene Unit

The P gene unit was produced next (Gene Unit 2 of Figure 2) and was designed to incorporate changes which would enable the P protein, but not the C proteins, to be expressed from this gene unit, i.e., these changes would be silent in the P ORF. The cDNA was PCR amplified using primers containing the *PacI* and *NheI* restriction enzyme sites at each end (3' and 5' ends, respectively) to enable cloning into the new pFLC-rHPIV1-C(P-) HN^{T553A}, Features incorporated into the P gene unit cDNA included:
1. The addition at the upstream end of a new gene-end sequence to act as the "C gene end" (sequence same as P gene end sequence); an intergenic region; and a new gene-start sequence to act as the "P gene start" (sequence same as N gene start sequence); these were added directly after the *PacI* restriction enzyme site.
   *C Gene End, nt #2494-2505, pFLC-rHPIV1-C+P HNT553A cDNA* (See, SEQ ID NO: 2). *(note that the PacI site and C Gene End share sequence identity for 6 nt and overlap at nt #2494-2499)*
   *Intergenic sequence, nt #2506-2508, pFLC-rHPIV1-C+P HNT553A cDNA* (See, SEQ ID NO:2). *P Gene Start, nt #2509-2518, pFLC-rHPIV1-C+P HNT553A cDNA* (SEQ ID NO: 2).
2. Truncation of the start of the P/C gene such that only 6 nucleotides lie between the P gene start sequence and the P start codon. These nts are nonviral in origin and have the sequence *CCCAAC (nt #2519-2524, pFLC-rHPIV1-C+P HNT553A cDNA* (SEQ ID NO:2).
3. Interruptions to the C open reading frame (ORF) such that the C proteins would not be expressed from the P gene unit and that these changes would be silent in the P ORF. This included:
   - Conversion of the C start (ATG → ACG)
      *Nt #2535-2537, pFLC-rHPIV1-C+P HNT553A cDNA* (SEQ ID NO:2).
   - Stop codons inserted 1 codon after Y1 start codon and 1 and 10 codons after Y2 start codon. (The Y1 and Y2 start codons were not modified because they could not be converted without affecting the P ORF).
      *Nt* #*2607-2609, pFLC-rHPIV1-C*+*P HNT553A cDNA* (SEQ ID NO:2).
      *Nt* #*2625-2627, pFLC-rHPIV1-C+P HNT553A cDNA* (SEQ ID NO:2).
      *Nt* #*2652-2654, pFLC-rHPTV1-C+P HNT553A cDNA* (SEQ ID NO:2).
   - The end of the P/C gene was truncated, leaving 13 nt sequence immediately after the P stop codon, followed by the *NheI* and *NotI* sites.

The P(C-) gene unit was cloned into the pFLC-rHPIV1-C(P-) HN^{T553A} cDNA at the *PacI* and *NheI* restriction enzyme sites. This generated the pFLC-rHPIV1-C+P HN^{T553A} cDNA.

The entire full-length HPIV1 antigenomic cDNA clone (FLC) was sequenced to confirm that it contained the correct introduced sequences and that no adventitious mutation(s) had been introduced during PCR amplification. (See Figure 2).

### Example 2: Production of variants of rHPIV1-C+P containing mutations in the C gene unit:

### A) Introducing mutations into the pFLC-rHPIV1-C+P cDNA plasmid:

Modified versions of the pFLC-rHPIV1 -C+P HN^{T553A} cDNA clone were prepared by site-directed mutagenesis and standard molecular cloning techniques to introduce mutations of interest into the C proteins of the virus, see Tables 1 and 3. Mutations were inserted into Gene Unit 1 of the pFLC-rHPIV1-C+P HN^{T553A} cDNA (Figure 2) such that the virus would express variant C proteins and a wild type P protein. When introducing defined mutations into the C gene unit, it is desirable not to have unintended mutations introduced into the P protein since such mutations could affect a function of P that could adversely alter the properties of the recovered virus. Such properties could include an alteration in the growth of such a vaccine candidate, i.e., one bearing an attenuating mutation in C and an unintended mutation in P, in cells used for vaccine manufacture.

The mutations introduced in C included point and deletion mutations affecting codons 83, 84, 85, 169, 170 or 171 of the C protein (Table 3). Note in Figure 3, that each of these mutations affects the C', C. Y1, and Y2 proteins. In each case, the region containing the introduced mutation in each FLC was sequenced to ensure that the FLC contained the introduced mutation. It can be seen by examining Table 3 and Figure 3, that viable recombinant HPIV1 mutants bearing genetically stable deletion mutations in C, but expressing a wild type P protein, are made that are capable of replication in cells in tissue culture (see below).

### B) Deleting codon 84 in the rHPIV1 wild type P/C backbone has a greater negative effect on replication in vitro than does such a deletion in the rHPIV1-C+P backbone.

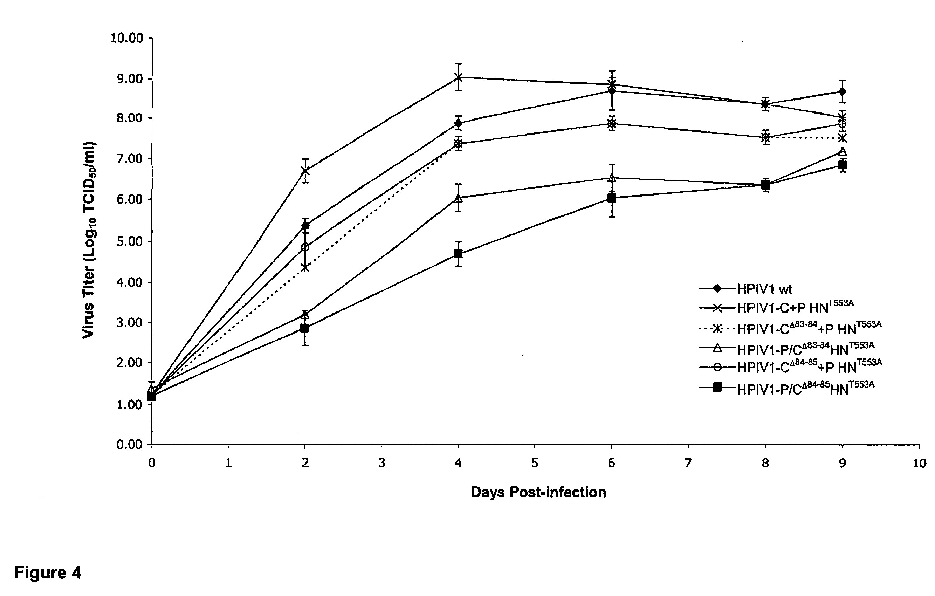

Viruses containing deletions at codon 84 in the C protein (C^{Δ83-84} and C^{Δ84-85}) in both the rHPIV1 wt and rHPIV1-C+P backbones were recovered and designated rHPIV1-P/C^{Δ83-84}HN^{T553A}, rHPIV1-P/C^{Δ84-85}HN^{T553A}, rHPIV1-C^{Δ83-84}+P HN^{T553A}, or rHPIV1-C^{Δ84-85}+P HN^{T553A} (Groups 2 and 3, Table 3). The kinetics of replication for these viruses was examined in a multi-cycle growth curve in LLC-MK2 cells (multiplicity of infection = 0.01) compared to the corresponding rHPIV1 wt and rHPIV1-C+P viruses (Figure 4). The viruses included in this growth curve were from uncloned virus suspensions, and this data has been confirmed with biologically cloned virus for select clones.

The rHPIV1-C+P grew to similar titers as rHPIV1 wt with peak titers reaching 9.0 and 8.7 log₁₀TCID₅₀/ml, respectively. This indicates that the separation of the two genes in rHPIV1-C+P results in a virus that can replicate as well as wild type rHPIV1. The rHPIV1-P/C^{Δ83-84}HN^{T553A} and rHPIV1-P/C^{Δ84-85}HN^{T553A} viruses in which both the C and P ORFs are affected by the deletions were moderately attenuated for replication in LLC-MK2 cells *in vitro* with peak titers reaching 7.2 and 6.9 log₁₀TCID₅₀/ml, respectively. The rHPIV1-C^{Δ83-84}+P HN^{T553^} and rHPIV1-C^{Δ84-85}+P HN^{T553A} viruses in which the deletion affects only the C proteins, but not P, both grew to 7.9 log₁₀TCID₅₀/ml, i.e., about 8-10-fold higher. Deletion of codons in both the C and P proteins specifies a stronger attenuating effect on replication *in vitro* than deleting codons in C only. This is probably due to a deleterious effect of the mutation in the P protein on virus replication for both the rHPIV1-P/C^{Δ83-84}HN^{T553A} and rHPIV1-P/C^{Δ84-85}HN^{T553A} viruses, i.e., viruses that contained desired mutations in C but unwanted mutations in P. The 10-fold increase of replication of this virus rHPIV1-C^{Δ83-84}+P HN^{T553A} and rHPIV1-C^{Δ84-85}+P HN^{T553A} versus the rHPIV1-P/C^{Δ83-84}HN^{T553A} and rHPIV1-P/C^{Δ84-85}HN ^{T553A} viruses would be advantageous during manufacture of the former viruses.

### Example 3. Cloning strategy for the production of HPIV3-P+C.

The starting point for constructing HPIV3-P+C was pUC(GE/GS)_{P-M} (Skiadopoulos et al Virology 297:136-152, 2002). This was a *Pme*I-*BamH*I fragment spanning nt 1215-3903 of the HPIV3 wt genome that had been subcloned and modified as follows: (i) individual nucleotides within positions 3693-3698 (of the complete antigenome sequence) were changed to introduce an *Afl*I site, and (ii) this *Afl*I site was used to accept a short DNA insert that contained an HPIV3 gene junction followed by several convenient cloning sites (Skiadopoulos et al Virology 297:136-152,2002). The resulting plasmid pUC(GE/GS)_{P-M} was thus designed so that an insert cloned into a *Mlu*I site would be situated downstream of the P gene and under the control of an independent set of gene-start and gene-end signals.

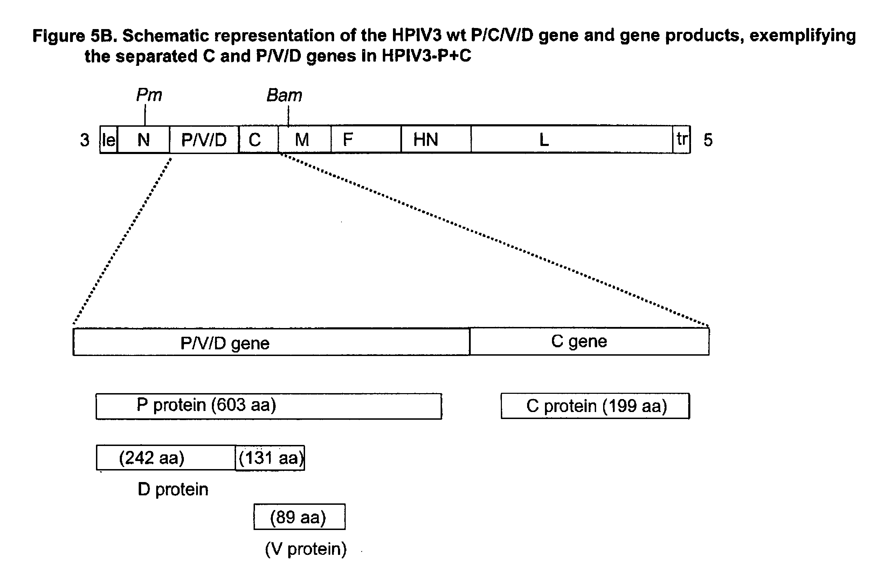

As shown in Fig. 5A, the P/C/V/D gene of HPIV3 has the potential to encode a complex array of proteins. P and C are expressed separately from overlapping ORFs. In addition, there are downstream D and V ORFs. D is accessed by RNA editing, and its synthesis is directly linked to that of the P gene (for general reference, use the Collins and Karron 2006 Fields Chapter). The mechanism of expression of V is unknown, although there is genetic evidence that it is indeed expressed. In most other paramyxoviruses, V is accessed by RNA editing, and its synthesis is directly linked to that of P. In any event, both D and V are located downstream of C and their expression is thought to be independent of C. Thus, in HPIV3-P+C, the P and C ORFs are segregated into separate polynucleotides, and D and V co-segregate with P (Fig. 5B).

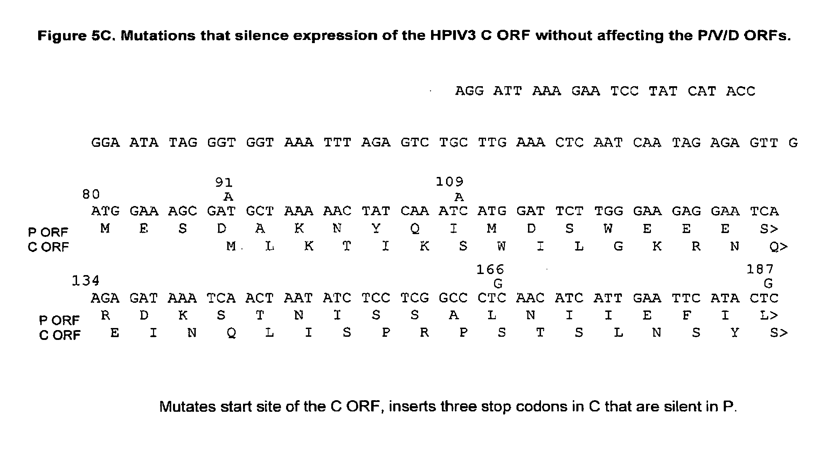

The first step is to engineer a copy of the P/C/V/D gene to make a polynucleotide in which expression of C was silenced (Fig. 5C). This involved using standard methods of site-directed mutagenesis to introduce point mutations into pUC(GE/GS)_{P-M} that (i) ablate the start codon of the C ORF, and (iii) introduce three translational start codons into the C ORF (Fig 5C). Each of these changes would be silent with regard to the overlapping P ORF (as well as the V and D ORFs, of course, since these are downstream and do not overlap C).

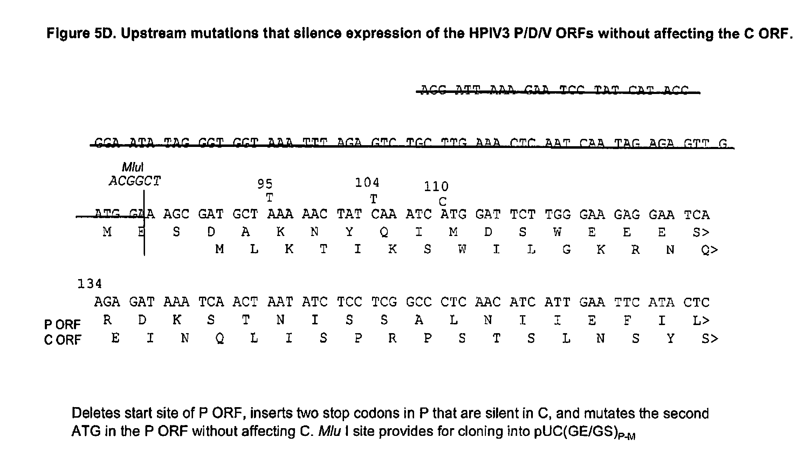

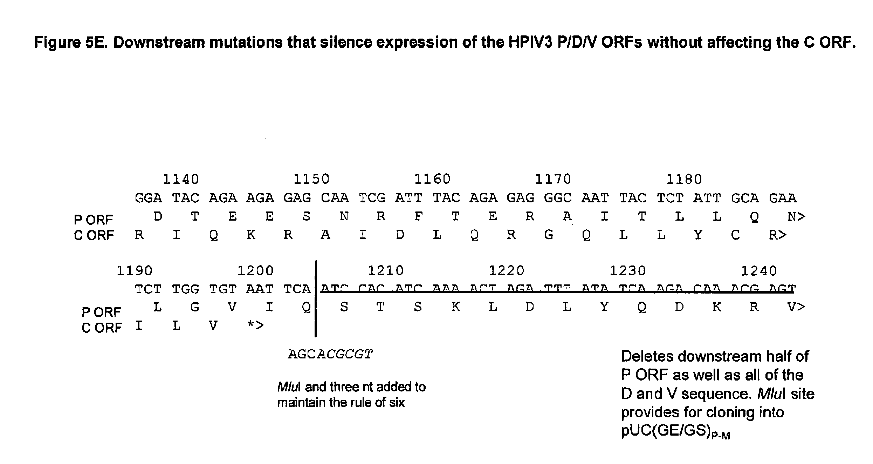

The second step is to engineer a second copy of the P/C/V/D gene to make a second polynucleotide in which expression of P is silenced and in which V and D are deleted. This involves PCR using primers that introduce a *Mlu*I site on either side of the ORF and, in addition, delete the start site of the P ORF as well as the downstream half of the P/C/V/D gene (Figures 5D and 5E). The resulting *Mlu*I fragment is cloned into the *Mlu*I site of pUC(GE/GS)_{P-M}. The resulting plasmid would then be subjected to site-directed mutagenesis using conventional methods to introduce two point mutations that create two translational stop codons in the P ORF without affecting the C ORF, and which change the second ATG in the P ORF to CTG without affecting the C ORF (Figure 5D).

The resulting modified version of pUC(GE/GS)_{P-M}, designated pUC(GE/GS)_{P-M}(P+C). would be subjected to restriction digestion with *Pml*I and *BamH*I, and the resulting fragment cloned into the corresponding window of the complete antigenomic cDNA.

**Table 2. Summary of the preferred L gene mutations for introduction into rHPIV1-C+P.**

| | **Mutation^{a}** | **ORF** | **nt changes wt→mutant^{b}** | **Type of mutation** | **Codon position in L** | **Amino acid change** | **# nt changes for reversion to wt amino acid** |
|---|---|---|---|---|---|---|---|
| 1 | Δ1710-11^{c} | L | **GCT GAG** --> →deletion | Deletion | 1710-11 | AE deletion | 6 (insertions) |
| 2 | Y942A^{d} | L | **TAT** → **GCG** | Point | 942 | Y → A | 3^{e} |
| 3 | F456L | L | **TTT** → **CTG** | Point | 456 | F → L | 2 |
| 4 | Y942T^{d} | L | **TAT** → **ACA** | Point | 942 | Y → T | 3 |
| 5 | L992C^{d} | L | **TTA** → **TGC** | Point | 992 | L → C | 2 |
| 6 | L992N^{d} | L | **CTC** → **AAC** | Point | 992 | L → N | 2 |

| | | | | | | | |
|---|---|---|---|---|---|---|---|
| ^{a} The nomenclature used to describe each mutation indicates the wt amino acid, the codon position and the new amino acid, or the position of the deletion (Δ), with respect to the C, HN or L protein. ^{b}The nucleotides (nt) affected by substitution or deletion are shown underlined and in bold type. ^{c}Designed for increased genetic stability by use of a deletion. Deletions involved six nt to conform to the rule of six. ^{d} Designed for increased genetic stability by the use of a codon that differs by three nucleotides from codons yielding a wild type assignment. | | | | | | | |

**Table 3. Summary of the C and P mutations introduced into the rHPIV1 genome,**

| **Mutations of interest^{a}** | | **Backbone** | **nt changes wt→mutant ^{b}** | **Type of mutation** | **nt # (viral genome) ^{c}** | **Affected Gene** | **Affected ORFs** | **Recovered as Infectious Virus?** | **Attenuated for replication?** | |
|---|---|---|---|---|---|---|---|---|---|---|
| | | | | | | | | | ***In Vitro*** | ***In Vivo*** |
| 1 | R84G | pFLC-rHPIV1 wt | **A**→**G** | point | 2103 | P/C | C'/C/Y1/Y2 and P | yes | no | yes (Hamsters only) [also yes as a set with HN^{T553A} only in African Green Monkeys] |
| 2 | Δ83-84 | pFLC-rHPIV1 wt | **AGG AGA** | deletion | 2100-2105 | P/C | C'/C/Y1/Y2 and P | yes | yes (to be confirmed) | yes (Hamsters only) |
| | | pFLC-rHPIV1-C+P ^{d} | | | 2100-2105 | C | C'/C/Y1/Y2 | yes | no (to be confirmed) | yes (Hamsters only) |
| 3 | Δ84-85 | pFLGrHPIV1 wt | **AGA GTG** | deletion | 2103-2108 | P/C | C'/C/Y1/Y2 and P | yes | yes | Not done |
| | | pFLC-rHPIV1-C+P ^{d} | | | 2103-2108 | C | C'/C/Y1/Y2 | yes | no | yes (Hamsters and African Green Monkeys) |
| 4 | F170S | pFLC-rHPIV1 wt | **T**→**C** | point | 2362 | P/C | C'/C/Y1/Y2 (Hamsters | yes | no | yes and African Green Monkeys) |
| 5 | Δ170 | pFLC-rHPIV1 wt | **GGA TTT** | deletion | 2357-2362 | P/C | C'/C/Y1/Y2 and P | yes | no | yes (Hamsters and African Green Monkeys) |
| 6 | Δ169-170 | pFLC-rHPIV1-C+P ^{d} | **GAT TTC** | deletion | 2358-2363 | C | C'/C/Y1/Y2 | yes | Not done | yes (Moderate in African Green Monkeys) |
| 7 | Δ170-171 | pFLC-rHPIV1-C+P ^{d} | **TTC CAG** | deletion | 2361-2366 | C | C'/C/Y1/Y2 | yes | Not done | Not done |

| | | | | | | | | | | |
|---|---|---|---|---|---|---|---|---|---|---|
| ^{a}The nomenclature used to describe each mutation within the genome contains the wt amino acid, the codon position and the new amino acid or deletion (Δ) with respect ^{b}The altered nucleotides (nt) are shown underlined and In bold type. ^{c}The nucleotide numbers refer to the nucleotide position in the viral genome of the indicated rHPIV1 backbone. ^{d}This backbone contains the HN^{T553A} mutation. | | | | | | | | | | |

### Example 4: Kinetics of replication of rHPTV-P(C-) in culture

The rHPN1 wt and mutant viruses were compared in multicycle growth curves. Confluent monolayer cultures of LLC-MK2 and Vero cells in 6-well plates were infected in triplicate at a MOI of 0.01 TCID₅₀/cell. Virus adsorption was performed for 1 h in media containing trypsin. The inoculum was then removed and cells were washed three times, after which fresh medium containing trypsin was added and then harvested as the day 0 sample and replaced with fresh media containing trypsin. On days 1-7 p.i., the entire supernatant was removed for virus quantitation and was replaced with fresh medium containing trypsin. Supernatants containing virus were frozen at -80°C, and virus titers (log₁₀ TCID₅₀/ml) were determined with endpoints identified by hemadsorption. Cytopathic effect (cpe) was visually monitored. The amount of cpe observed under the microscope was given a score ranging from 1-5 based on the percentage of cells in the monolayer showing cpe. Cpe of less than 20% of cells was scored as 1; 21-40% as 2; 41%-60% as 3; 61-80% as 4; 81-100% as 5. Results are shown in Table 4.

**Table 4. Cytopathicity of HPIV mutants**

| | | CPE Scores | | | | | | | | | | | | | |
|---|---|---|---|---|---|---|---|---|---|---|---|---|---|---|---|
| | | MK2 Cells (Days post-infection) | | | | | | | Vero Cells (Days post-infection) | | | | | | |
| Virus | | 1 | 2 | 3 | 4 | 5 | 6 | 7 | 1 | 2 | 3 | 4 | 5 | 6 | 7 |
| 1 | rHPIV1-HN^{T553A} | 1 | 1 | 1 | 1 | 1 | 1 | 1 | 1 | 1 | 1 | 1 | 1 | 1 | 1.7 |
| 2 | rHPIV1-C+P HN^{T553A} | 1 | 1 | 1 | 2.5 | 2.7 | 3.5 | 4 | 1 | 1 | 1 | 1 | 1 | 1.3 | 2 |
| 3 | rHPIV1-P/C^{Δ84-85} HN^{T553A} | 1 | 1 | 1 | 1.8 | 2.7 | 3.3 | 4.7 | 1 | 1 | 1 | 1 | 1 | 1.2 | 2 |
| 4 | rHPIV1-C^{Δ84-85}+P HN^{T553A} | 1 | 1 | 2.2 | 4 | 4.5 | 5 | 5 | 1 | 1 | 1 | 1.5 | 2 | 3.7 | 4.7 |

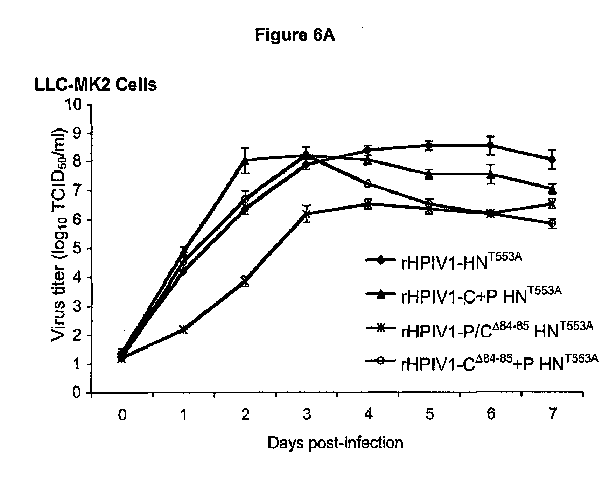

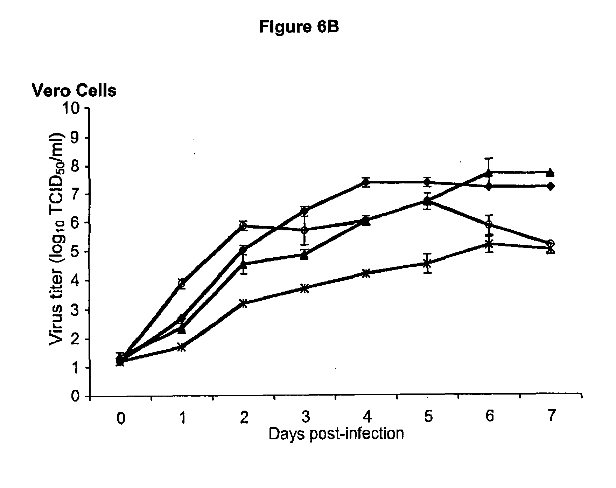

Separating the P and C open reading frames into two separate gene units offers a distinct advantage for *in vitro* replication of RHPIV1s with mutations in the C proteins. This is shown in multicycle growth curves comparing the replication of mutants containing the Δ84-85 mutation on the wt backbone vs the C+P backbone, i.e. rHPIV1-P/C^{Δ84-85} and rHPIV1-C^{Δ84-85}+P, respectively (Figure 6). These data clearly demonstrate a replication advantage for mutants containing the Δ84-85 deletion mutation on the C+P backbone. rHPIV1-C^{Δ84-85}+P reached peaks of 8.2 and 6.7 log₁₀ TCID₅₀/ml in LLC-MK2 and Vero cells respectively, compared to rHPIV1-P/CΔ⁸⁴⁻⁸⁵ at 6.5 and 5.2 log₁₀ TCID₅₀/ml. Clinical lots of the viruses will be prepared in Vero cells and a titer of approximately 7 is required for efficient manufacturing purposes. Therefore, it is evident that separating the P and C genes in viruses to be used for HPIV vaccines is advantageous in respect to providing high titer for manufacturing. The the ability to introduce mutations into the C gene without affecting P can greatly improve the in vitro replication competence of viruses to be used for vaccine purposes.

### Example 5: The effect of infection with rHPIV1 wt or mutants on type I IFN signaling.

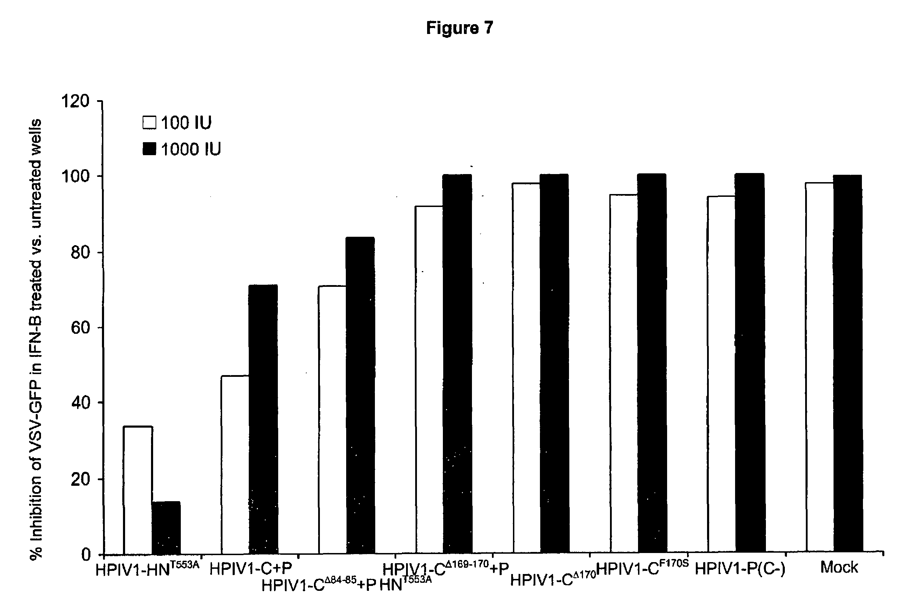

The ability of the rHPIV1s to inhibit type I IFN signaling was determined by their ability to block the establishment of an antiviral state by IFN, as indicated by the level of VSV replication following IFN treatment. Vero cells in 6-well plates were infected with the indicated rHPIV1s at a MOI of 5 TCID₅₀/cell and incubated for 24 h. Cells were then left untreated or were treated with 100 or 1000 IU/ml IFN-β (AVONEX; Biogen, Inc., Cambridge, MA) (1 well per treatment per virus) for 24 h. The cells were then infected with VSV-GFP, under a 0.8% methyl cellulose overlay, and incubated for 48 h. The VSV-GFP foci were visualized using a typhoon 8600 phosphorimager (Molecular Dynamics, Sunnyvale, CA) and counted. Figure 7 presents the percent inhibition of VSV-GFP replication in IFN-β treated versus untreated cells based on two independent experiments.

### Example 6: Efficacy of rHPIV1 wt and mutants against challenge with wild-type HPIV1.

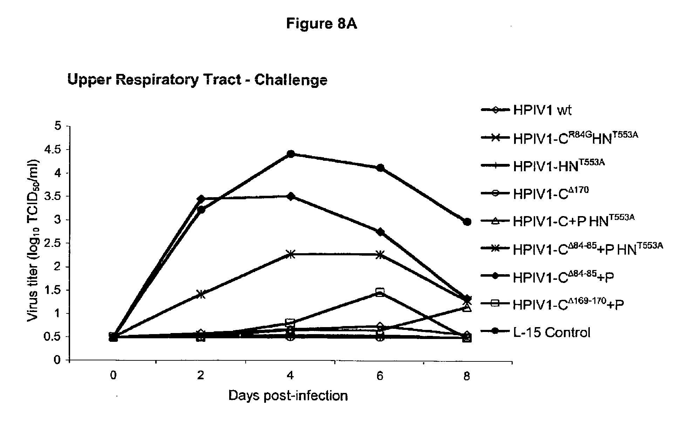

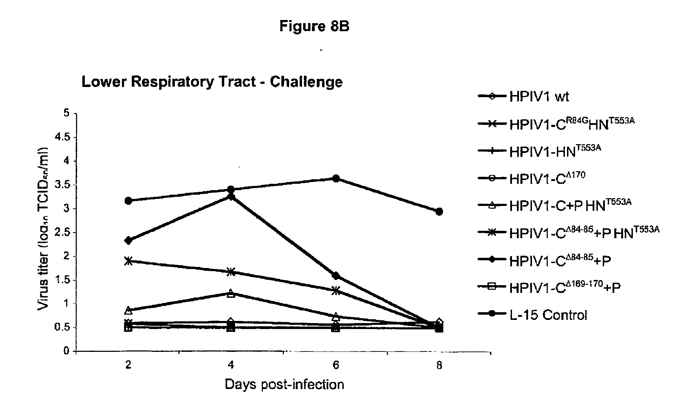

Replication and induction of a protective immune response after immunization with wild-type and various mutant HPIV1 were assessed in Syrian Golden hamsters and in African Green Monkeys (Tables 5-7, Figure 8).

Inoculation of hamsters with rHPIV1 mutants containing deletions at codon 84 either on the wild type or the C+P backbone, demonstrated attenuation of replication in both the upper respiratory tract (URT) and lower respiratory tract (LRT), regardless of the backbone the deletion was in. In contrast, inoculation of hamsters with a rHPIV1 containing a single point mutation at codon 84 demonstrated replication at wt level in the URT and a moderate reduction of replication in the LRT. Therefore, the use of a deletion mutation at codon 84 offers advantages over the use of a point mutation at this codon since it is not only more stable but also more attenuating in hamsters. Protective efficacy against HPIV1 wt challenge was determined next in hamsters that had been inoculated with these viruses. Although rHPIV1-C^{Δ83-84}+P HN^{T553A}, rHPIV1-C^{Δ84-85}+P HN^{T553A} and rHPIV1-C/P^{Δ83-84} HN^{T553A} all grew to similar levels following initial inoculation, only rHPIV1-C^{Δ84-85}+P HN^{T553A} protected against challenge with HPIV1 wt. Since rHPIV1-C^{Δ84-85}+P HN^{T553A} replicated efficiently *in vitro* and was attenuated and efficacious in hamsters, we proceeded to studies in African green monkeys with this virus.

AGMs (n≥4) were inoculated i.n. and intratracheally (i.t.) with 10⁶ TCID₅₀ of either HPIV1 wt or mutant rHPIV1 in a 1ml inoculum at each site. Nasopharyngeal (NP) swab samples were collected on days 0 to 10 post-infection and tracheal lavage (TL) fluid samples were collected on days 2, 4, 6, 8, and 10 post-infection. On day 28 p.i., the AGMs were challenged i.n. and i.t. with 10⁶ TCID₅₀ of HPIV1 wt in 1 ml L-15 per site. NP swab samples and TL fluid samples were collected for virus quantitation on days 0, 2, 4, 6 and 8 and days 2, 4, 6 and 8 post-challenge, respectively. The specimens were flash frozen and stored at -80°C until they were assayed in parallel. Virus present in the samples was titered in serial dilutions on LLC-MK2 cell monolayers in 96-well plates, and an undiluted 100µl aliquot also was tested in 24-well plates. Following incubation for 7 days, virus was detected by hemadsorption, and the mean log₁₀ TCID₅₀/ml titer was calculated for each sample day. The limit of detection was 0.5 log₁₀ TCID₅₀/ml. The mean daily titer for each group is represented on the graph. All animal studies were performed under protocols approved by the National Institute of Allergy and Infectious Disease (NIAID) Animal Care and Use Committee (ACUC).

**Table 5. Replication of rHPIV1 mutants in the upper and lower respiratory tract of hamsters.**

| Virus ^{a} | | Number of animals | Nasal turbinates ^{b} | | Lungs ^{b} | |
|---|---|---|---|---|---|---|
| | | | Day 4 ^{c} | Day 5 ^{c} | Day 4 ^{c} | Day 5 ^{c} |
| 1 | rHPNI-HN^{T553A} | 11 | 5.2 ± 0.2 | 5.3 ± 0.4 | 4.6 ± 0.5 | 4.6 ± 0.4 |
| 2 | rHPIV1-C+P-HN^{T553A} | 10 | 4.1 ± 0.3 | 4.8 ± 0.3 | 2.6 ± 0.3 | 3.1 ± 0.2 |
| 3 | rHPIV1-C^{R84G}HN^{T553A} | 6 | 5.2 ± 0.2 | 4.6 ± 0.2 | 2.8 ± 0.3 | 3.5 ± 0.4 |
| 4 | rHPIV1-C^{Δ83-84}+P-HN^{T553A} | 10 | 2.0 ± 0.2 | 1.9 ± 0.1 | 2.2 ± 0.1 | 2.3 ± 0.1 |
| 5 | rHPNI-C^{Δ84-85}+P-HN^{T553A} | 10 | 2.4 ± 0.1 | 2.2 ± 0.2 | 2.4 ± 0.2 | 2.3 ± 0.2 |
| 6 | rHPIV1-C/P^{Δ83-84}-HN^{T553A} | 5 | 1.6 ± 0.1 | 1.7 ± 0.2 | 1.7 ± 0.2 | 2.0 ± 0.2 |

| | | | | | | |
|---|---|---|---|---|---|---|
| ^{a}Hamsters in groups of 5 or 6 were inoculated i.n. with 10^{5.5} TCID₅₀ of the indicated virus. The data is representative of two independent experiments. rHPIV1-HN^{T553A} has been shown to replicate to wt-like effect *in vivo* [Bartlett, E.J. et al., Vaccine 24:2674-2684 (2006)]. ^{b}Virus present in the tissues was quantified by serial dilution on LLC-MK2 monolayer cultures at 32°C and is expressed as the mean virus titer for each group, log₁₀ TCID₅₀/g ± SE. The limit of detection was 1.5 log₁₀ TCID₅₀/g. ^{c}Nasal turbinates and lungs from each group were harvested on day 4 and 5 post-infection. ^{d}Underlined values indicate a 100-fold reduction in virus replication compared to rHPIV1-HN^{T553A}. | | | | | | |

**Table 6. Protection against HPIV wt challenge in hamsters following immunization with rHPIVI mutants.**

| Virus^{a} | | No. animals | Mean HPIV1 wt titer (log₁₀ TCID₅₀/g)^{b} | |
|---|---|---|---|---|
| | | | Nasal turbinates ^{c} | Lungs^{c} |
| 1 | rHPIV1-HN^{T553A} | 5 | 1.5 ± 0.0 | 1.7 ± 0.2 |
| 2 | rHPIV1-C^{R84G}HN^{T553A} | 5 | 1,7 ± 0.2 | 1.7 ± 0.1 |
| 3 | rHPIV1-C+P HN^{T553A} | 5 | 1.5 ± 0.0 | 1.6 ± 0.1 |
| 4 | rHPIV1-C^{Δ83-84}+P HN^{T553A} | 5 | 2.8 ± 0.5 | 3.0 ± 0.4 |
| 5 | rHPIV1-C^{Δ84-85}+P HN^{T553A} | 5 | 1.9 ± 0.2 | 2.1 ± 0.2 |
| 6 | rHPIV1-C/P^{Δ83-84} HN^{T553A} | 5 | 4.4 ± 0.4 | 2.7 ± 0.2 |
| 7 | Non-immunized | 5 | 4.5 ± 0.2 | 3.9 ± 0.4 |

| | | | | |
|---|---|---|---|---|
| ^{a}Hamsters in groups of 5 or 6 were inoculated i.n. with 10⁶ TCID₅₀ of the indicated virus. rHPIV1-HN^{T553A} has been shown to replicate to wt-like effect *in vivo* [Bartlett, E.J. et al., Vaccine 24:2674-2684 (2006)]. ^{b}Virus present in the tissues was quantified by serial dilution on LLC-MK2 monolayer cultures at 32°C and is expressed as the mean of virus titer, log₁₀ TCID₅₀/g ± SE. The limit of detection was 1.5 log₁₀ TCID₅₀/g. ^{c}Nasal turbinates and lungs from each group were harvested on day 4 post-infection. ^{d}Underlined values underlined indicate a 100-fold reduction in virus replication compared to the non-immunized control group. | | | | |

**Table 7. Replication of rHPIV1 mutants in AGMs.**

| Virus^{a} | | No. animals | Mean peak virus titer (log₁₀ TCID₅₀/ml)^{c} | | Mean sum of the daily virus titers (log₁₀ TCID₅₀/ml)^{d} | |
|---|---|---|---|---|---|---|
| | | | NP swab | TL | NP swab | TL |
| 1 | HPIV1 wt | 18 | 4.3 ± 0.1 | 4.1 ± 0.3 | 27.4 ± 1.4 | 13.6 ± 1.4 |
| 2 | rHPIV1-HN^{T553A} | 4 | 4.3 ± 0.2 | 4.6 ± 0.2 | 24.9 ± 0.7 | 13.7 ± 1.8 |
| 3 | rHPIV1-C+P HN^{T553A} | 6 | 3.4 ± 0.4 | 4.7 ± 0.4 | 22.3 ± 2.3 | 15.3 ± 1.5 |
| 4 | rHPIV1 -C^{R84G} HN^{T553A} | 12 | 2.1 ± 0.2 | 4.8 ± 0.3 | 10.5 ± 0.9 | 14.3 ± 1.1 |
| 5 | rHPIV1-C^{Δ84-85}+P | 4 | 1.6 ± 0.3 | 1.7 ± 0.1 | 7.9 ± 1.3 | 7.8 ± 0.2 |
| 6 | rHPIV1-C^{Δ84-85}+P HN^{T553A} | 4 | 1.7 ± 0.2 | 1.8 ± 0.3 | 9.4 ± 0.9 | 7.1 ± 0.2 |
| 7 | rHPIV1-C^{F170S} HN^{T553A} | 4 | 3.2 ± 0.9 | 2.7 ± 0.9 | 15.4 ± 3.6 | 6.2 ± 1.3 |
| 8 | rHPIV1-C^{Δ170} | 6 | 3.4 ± 0.5 | 2.3 ± 0.5 | 14.8 ± 1.9 | 5.1 ± 0.8 |
| 9 | rHPIV1 -C^{Δ169-170}+P HN^{T553A} | 4 | 3.4 ± 0.4 | 3.7 ± 0.3 | 19.4 ± 1.2 | 10.2 ± 1.4 |

| | | | | | | |
|---|---|---|---|---|---|---|
| ^{a}Monkeys were inoculated i.n. and i.t. with 10⁶ TCID₅₀ of the indicated virus in a 1ml inoculum at each site. Monkeys lacked HAI antibody to HPIV wt at the start of the study. ^{b}NP samples were collected on days 0 to 10 post-infection. The titers on day 0 were ≤0.5 log₁₀ TCID₅₀/ml. ^{c}TL samples were collected on days 2, 4, 6, 8, and 10 post-infection. The limit of detection was 0.5 log₁₀ TCID₅₀/ml. ^{d}Mean of the peak virus titers ± SE, irrespective of day, for the animals in each group. Virus titrations were performed on LLC-MK2 cells at 32°C. The limit of detection was 0.5 log₁₀ TCID₅₀/ml. ^{e}Reduction in mean peak virus titer versus HPIV1 wt is expressed as log₁₀ TCID₅₀/g. ^{f}The daily titers for all sampling days were summed for each animal and the mean was determined, shown ± SE. This indicates the total load of shed virus. The limit of detection for NP samples was 5.5 log₁₀ TCID₅₀/ml and for TL samples was 2.5 log₁₀ TCID₅₀/ml. | | | | | | |

Examples 5 and 6 show that C+P mutants containing the Δ84-85 and Δ169-170 demonstrated no inhibition of type I IFN signaling (Figure 5), a characteristic that has previously been associated with attenuation *in vivo* (Van Cleve, W., 2006). Indeed, C+P mutants containing these deletions were attenuated in animal models including Syrian Golden hamsters and African Green monkeys (AGMs) (Tables 5 and 7). rHPIVI-C^{Δ84-85}+P was highly attenuated in both AGMs and hamsters and rHPIV1-C^{Δ169-170}+P, demonstrated a moderate level of attenuation in AGMs (Table 7). These mutants provided varying levels of protection in Syrian Golden hamsters against challenge with HPIV1 wt (Table 6 and Figure 8).

## Claims

1. An infectious self-replicating parainfluenza virus (PIV) comprising a partial or complete PIV1 or PIV3 genome or antigenome comprising
a) a first open reading frame polynucleotide encoding a P protein; and
b) a second open reading frame polynucleotide encoding a C protein wherein the first and second open reading frame polynucleotides each consist of a single open reading frame and do not overlap.

2. An infectious self-replicating parainfluenza virus (PIV) comprising a partial or complete PIV1 or PIV3 genome or antigenome comprising
a) a first open reading frame polynucleotide encoding a P protein from which expression of C, C', Y1 and Y2 proteins has been ablated or greatly reduced; and
b) a second open reading frame polynucleotide encoding a C protein having at least one nucleotide change that ablates or reduces expression of said C protein or that expresses a truncated C protein or that expresses a C protein with reduced activity compared to a native C protein, and from which expression of a P protein has been ablated or greatly reduced
wherein the first and second open reading frame polynucleotides each consist of a single open reading frame and do not overlap.

3. The PIV according to claim 2, wherein said second polynucleotide encodes C proteins having a C-terminal deletion of 14 amino acids.

4. The PIV according to claim 1 or 2, further comprising a polynucleotide sequence encoding an HN or an L protein.

5. An isolated polynucleotide comprising:
a) a first open reading frame polynucleotide encoding a parainfluenza virus (PIV) P protein; and
c) a second open reading frame polynucleotide encoding a parainfluenza virus (PIV) C protein
wherein the first and second open reading frame polynucleotides each consist of a single open reading frame and do not overlap.

6. An isolated polynucleotide comprising a polynucleotide encoding a partial or complete PIV genome or antigenome comprising the isolated polynucleotide of claim 5.

## Patentansprüche

1. Infektiöse selbstreplizierendes Parainfluenzavirus (PIV), umfassend ein partielles oder vollständiges PIV1- oder PIV3-Genom oder -Antigenom, umfassend
a) ein in einem offenen Leseraster gelegenes erstes Polynukleotid, das ein P-Protein codiert; und
b) ein in einem offenen Leseraster gelegenes zweites Polynukleotid, das ein C-Protein codiert,
wobei die in offenen Leserastern gelegenen ersten und zweiten Polynukleotide jeweils aus einem einzelnen offenen Leseraster bestehen und nicht überlappen.

2. Infektiöses selbstreplizierendes Parainfluenzavirus (PIV), umfassend ein partielles oder vollständiges PIV1- oder PIV3-Genom oder -Antigenom, umfassend
a) ein in einem offenen Leseraster gelegenes erstes Polynukleotid, das ein P-Protein codiert, bei dem die Expression der C-, C'-, Y1- und Y2-Proteine unterbunden oder stark reduziert ist; und
b) ein in einem offenen Leseraster gelegenes zweites Polynukleotid, das ein C-Protein mit mindestens einer Nukleotidänderung codiert, die die Expression des C-Proteins unterbindet oder reduziert oder die ein verkürztes C-Protein exprimiert oder die ein C-Protein mit reduzierter Aktivität im Vergleich zu einem nativen C-Protein exprimiert, und bei dem die Expression eines P-Proteins unterbunden ist oder stark reduziert ist,
wobei die in offenen Leserastern gelegenen ersten und zweiten Polynukleotide jeweils aus einem einzelnen offenen Leseraster bestehen und nicht überlappen.

3. PIV nach Anspruch 2, wobei das zweite Polynukleotid C-Proteine mit einer C-terminalen Deletion von 14 Aminosauren codiert.

4. PIV nach Anspruch 1 oder 2, zudem umfassend eine Polynukleotidsequenz, die ein HN- oder L-Protein codiert.

5. Isoliertes Polynukleotid, umfassend:
a) ein in einem offenen Leseraster gelegenes erstes Polynukleotid, das ein Parainfluenzavirus(PIV)-P-Protein codiert; und
b) ein in einem offenen Leseraster gelegenes zweites Polynukleotid, das ein Parainfluenzavizus(PIV)-C-Protein codiert,
wobei die in offenen Leserastern gelegenen ersten und zweiten Polynukleotide jeweils aus einem einzelnen offenen Leseraster bestehen und nicht überlappen.

6. Isoliertes Polynukleotid, umfassend ein Polynukleotid, das ein partielles oder vollständiges PIV-Genom oder -Antigenom codiert, umfassend das isolierte Polynukleotid nach Anspruch 5.

## Revendications

1. Virus para-influenza (PIV) auto-réplicant infectieux, comprenant un génome ou un antigénome partiel ou complet du PIV1 ou du PIV3, comprenant
a) un premier nucléotide cadre ouvert de lecture codant pour une protéine P ; et
b) un deuxième polynucléotide cadre ouvert de lecture codant pour une protéine C,
le premier et le deuxième polynucléotides cadres ouverts de lecture consistant chacun en un cadre ouvert de lecture unique et ne se chevauchant pas.

2. Virus para-influenza (PIV) auto-réplicant infectieux comprenant un génome ou un antigénome partiel ou complet du PIV1 ou du PIV3, comprenant :
a) un premier nucléotide cadre ouvert de lecture codant pour une protéine P, dont l'expression des protéines C, C', Y1 et Y2 a été ablatée ou fortement réduite ; et
b) un deuxième polynucléotide cadre ouvert de lecture codant pour une protéine C ayant au moins un changement de nucléotide qui provoque une ablation ou une réduction de l'expression de ladite protéine C ou qui exprime une protéine C tronquée, ou qui exprime une protéine C présentant une activité réduite par comparaison avec celle d'une protéine C native, et dont l'expression d'une protéine P a été ablatée ou fortement réduite,
le premier et le deuxième polynucléotides cadres ouverts de lecture consistant chacun en un cadre ouvert de lecture unique, et ne se chevauchant pas.

3. PIV selon la revendication 2, dans lequel ledit deuxième polynucléotide code pour des protéines C ayant une délétion C-terminale de 14 acides aminés.

4. PIV selon la revendication 1 ou 2, comprenant en outre une séquence polynucléotidique codant pour une protéine HN ou une protéine L.

5. Polynucléotide isolé, comprenant :
a) un premier polynucléotide cadre ouvert de lecture pour une protéine P du virus para-influenza (PIV) ; et
c) un deuxième polynucléotide cadre ouvert de lecture codant pour une protéine C du virus para-influenza (PIV),
le premier et le deuxième polynucléotides cadres ouverts de lecture consistant chacun en un cadre de lecture ouvert unique, et ne se chevauchant pas.

6. Polynucléotide isolé comprenant un polynucléotide codant pour un génome ou un antigénome partiel ou complet du PIV, comprenant le polynucléotide isolé de la revendication 5.


### SEQUENCE LISTING

<110> Murphy, Brian Collins, Peter Schmidt, Alexander Bartlett, Emmalene
<120> HUMAN PARAINFLUENZA VIRUSES HAVING SEPARATED P AND C GENES
<130> 1173-1071PUS1
<160> 58
<170> PatentIn version 3.4
<210> 1
   <211> 15600
   <212> DNA
   <213> Human parainfluenza virus
<400> 1
   accaaacaag aggaaaaact tgtttggaat atataataat attaaatagt attttagggt      60
   taaagtaata ctttaaaggg acaagtcaca gacatttgat cttagtataa atttttataa     120
   tggccgggct actaagtact tttgacacat ttagttccag gagaagtgag agcatcaata     180
   agtctggcgg aggagcaatt atacctggtc aaagaagtac cgtttctgtc ttcacattag     240
   gcccgagtgt gacagatgat gcagataaat tattaatagc aaccactttc ttagcccact     300
   cattggacac agataaacaa cactctcaaa gaggaggatt tttagtatca ctccttgcaa     360
   tggcctacag tagtccggag ttatatctca ctacaaacgg tgtcaatgct gatgtcaagt     420
   atgtgatata caatatagag agagatccta aaagaacaaa aacagatggg ttcattgtca     480
   aaacaagaga catggagtat gaaagaacaa cagagtggtt gtttggacct atgatcaaca     540
   agaacccatt gttccaaggg caaagagaga atgcggatct agaggcattg cttcagacat     600
   atggatatcc tgcatgtctc ggagctataa tagttcaagt ttggatagtg ttggttaaag     660
   ccataacaag tagtgctggt ctaagaaaag gattcttcaa tagattagaa gcattcagac     720
   aggatggaac cgttaaaagt gccctggtct tcacaggaga cacagttgaa ggtattggtg     780
   cagtgatgag gtcacaacaa agcttagtat ctcttatggt agaaactctg gtgactatga     840
   acacatccag gtcagattta actacattag agaagaacat tcagattgta ggaaattaca     900
   taagagatgc aggattagca tctttcatga acaccatcaa gtatggtgta gaaacgaaga     960
   tggccgccct gacactatca aatctgagac cagatataaa caaattgaga agccttgttg    1020
   atatctatct atcaaaggga gcccgagccc cttttatatg tatactcaga gacccagttc    1080
   atggagactt tgcccctgga aactatccag cactgtggag ctacgcaatg ggcgttgctg    1140
   tggtacaaaa caaagctatg caacagtatg taactggaag aacatatttg gacatggaaa    1200
   tgttcctact tggacaagct gtagctaaag atgctgattc caaaatcagc agtgctctgg 1260
   aggaagaact aggtgtgaca gatacagcaa aagagagact aagacaccat ctgacaaacc 1320
   tttcaggagg ggatggtgcg taccacaagc ctacaggtgg tggagctata gaagtggcaa 1380
   ttgatcatac agacataaca tttggagtcg aggacactgc tgatcgggac aacaagaact 1440
   ggacaaatga cagcaatgaa agatggatga atcactcgat cagcaaccac acaatcacga 1500
   ttcgtggtgc agaagaactt gaagaagaga caaatgatga agacatcact gatatagaaa 1560
   acaagattgc acgaaggctg gccgacagaa aacagagact aagccaggca aacaataaac 1620
   gagacaccag cagtgatgct gactatgaga atgatgatga tgctacagcg gctgcaggga 1680
   taggaggaat ttaacaggat acttggacaa tagaagccag atcaaaagta agaaaaactt 1740
   agggtgaatg acaattcaca gatcagctca accagacacc accagcatac acgaaaccaa 1800
   ccttcacagt ggatacctca gcatccaaaa ctctccttcc cgaatggatc aggatgcctt 1860
   cttttttgag agggatcctg aagccgaagg agaggcacca cgaaaacaag aatcactctc 1920
   agatgtcatc ggactccttg acgtcgtctt atcctacaag cccacagaaa ttggagaaga 1980
   cagaagctgg ctccatggta tcatcgacaa cccaaaagaa aacaagccat catgcaaagc 2040
   cgacgataac aacaaagaca gagcaatctc aacgtcgacc caagatcata gatcaagtga 2100
   ggggagtgga atctctagga gaacaagtga gtcaaaaaca gagacacatg ctagaatcct 2160
   tgatcaacaa ggtatacaca gggcctctag gcgaggaact agtccaaacc ctctacctga 2220
   gaatatgggc aatgaaagaa acaccagaat cgacgaagat tctccaaatg agagaagaca 2280
   tcagagatca gtacttacgg atgaagacag aaagatggct gagaactcta ataagaggga 2340
   agaagaccaa gttgagggat ttccagaaga ggtacgaaga agtacaccct tatctgatga 2400
   tggagagggt agaacaaata ataatggaag aagcatggaa actagcagca cacatagtac 2460
   aagaataact gatgtcatta ccaacccaag tccagagctt gaagatgccg ttctacaaag 2520
   gaacaaaaga cggccgacga ccatcaagcg taaccaaaca agatcagaga gaacacagag 2580
   ttcagaactc cacaaatcaa caagtgaaaa tagctccaac ctcgaagacc acaacaccaa 2640
   aaccagccca aaagttccac cgtcaaagaa cgaagagtca gcagccactc caaagaacaa 2700
   ccacaaccac agaaaaacaa gatacacaac aaacaatgca aacaacaaca caaaaagtcc 2760
   accaactccc gaacacgacg caaccgcaaa tgaagaggaa accagcaaca catcggtcga 2820
   tgagatggcc aagttattag taagtcttgg tgtaatgaaa tcacaacatg aatttgaatt 2880
   atctaggagt gcaagtcatg tatttgctaa gcgcatgtta aaatctgcaa attacaaaga 2940
   aatgacattt aatctctgtg gtatgcttat atcagttgaa aaatcacttg agaataaagt 3000
   agaagaaaat agaacattac ttaaacaaat tcaagaggaa ataaattcat ccagggatct 3060
   tcacaaacgg ttctcggaat accaaaaaga acagaactca ctcatgatgg ccaatctatc 3120
   cacactccat ataattacag atagaggcgg gaaaacggga aatcccagtg atactacaag 3180
   gtcaccatca gtcttcacaa aagggaaaga caataaggtc aaaaagacaa ggtttgaccc 3240
   ctctatggaa gctctaggag gtcaagagtt caagcctgac ttgataagag aggatgaact 3300
   gagagatgac atcaaaaatc cggtactaga agaaaacaac aatgagcctc aagcatccaa 3360
   tgcatcacgc ctgattccgt ccactgaaaa acacactctg cactcactca aactagttat 3420
   cgaaaacagt cctctaagca gagtagagaa gaaggcttac atcaaatccc tttataagtg 3480
   tcggacaaac caagaggtta aaaatgtaat ggagctattc gaggaagaca tagattcact 3540
   aactaactaa acatgaatct acaatttcaa ccagcaatca aaatcaatat ccagagccaa 3600
   ctcaaaaagc tccctcaaaa caattaagaa aaacttaggg tcaaagaaat tttgcccgga 3660
   gaaaggaaat ggctgaaaca tacaggttcc ccagattctc acacgaagaa aatgggacag 3720
   tagaacctct ccctctcaaa acaggtcctg acaaaaaagc aatccctcac atcagaatag 3780
   tcaaggtagg agatcctcca aaacatggag tcaggtatct tgatgtgcta ctattgggat 3840
   tctttgaaac acctaagcaa ggacctctat ctggcagcat atctgatctc acagaatcaa 3900
   ccagttattc aatctgtgga tccggatcct taccaattgg catagccaag tattacggca 3960
   cagatcaaga attattaaaa gcctgcattg acctcaaaat aactgtacga agaacagtta 4020
   gatctggaga aatgatagta tacatggtag attcgatcca tgctcctcta ctaccatggt 4080
   ccagccgact gagacaaggg atgatatata atgccaataa agtagctcta gcacctcaat 4140
   gtctcccagt cgacaaagac atcagattca gggttgtatt tgtcaatgga acatcactag 4200
   gtacaattac aattgctaag gtcccaaaaa ctcttgcaga tcttgcatta ccgaactcaa 4260
   tatcagtgaa tctgctggtt acacttaggg caggagtatc aacggaacaa aaaggaatcc 4320
   tccccgttct agacgatgat ggagaaaaga agctcaactt catggtacac ctaggaatca 4380
   taagaagaaa agttgggaag atatattcag ttgaatactg caaaaataaa attgagaaga 4440
   tgaagctaat attctctctc gggcttgtag gtggaataag tttccatgta catgcaacag 4500
   gcacattatc caaaactcta atgagccaac ttgcatggaa aaaagcagtt tgctatcctt 4560
   taatggatgt aaatccacat atgaatctag tcatctgggc agcttcagta gaaatcacaa 4620
   gtgtcgatgc tgtgttccaa cctgcaattc cgaaagaatt tcgctattac ccaaatgttg 4680
   ttgcaaaaag catcgggaaa atcaggagga tataagtcta cactcctcaa taatgacacc 4740
   cattagctct aaatcgtacc attaatcaaa tacagatcaa ttcgatacaa tcagttcaaa 4800
   taagaaaaac gtagggacaa agtcctctac caacatcaag gaagacaaga gtctcaaaaa 4860
   gctcagccta agcagagaga aaaacaacaa cacaaagaaa gaaaaggaca agatcacaaa 4920
   caagaacaaa agcaaaaaca aaaacaagaa caaaaaaggg aagaaaaaca aaagtataca 4980
   caaaaaccaa aaaagaaaaa aggccagaga caaaaacgga ggcaagaaca aaaatttaaa 5040
   caaaaacaga atttaaattc ataataaaca ccaagataga gacaaaaatg caaaaatcag 5100
   agatcctctt cttagtatac tcaagcttgc tattatcttc atcattatgt caaattccgg 5160
   tagaaaaact ttcaaatgta ggggttataa tcaatgaggg caaattactt aaaatagcag 5220
   gatcttatga atctagatac atagtgttaa gcttggtacc ttcaattgac ctacaagatg 5280
   gatgtggaac aactcaaatt attcaataca agaatttatt aaatagactt ctaattcctc 5340
   tgaaggatgc cttggatctt caggaatccc tgataacaat aactaatgac accactgtga 5400
   caaatgataa tccacaaact agattctttg gtgctgtcat tggtaccata gcactaggag 5460
   tagccacagc tgctcaaata actgcgggca ttgcattagc tgaagcacga gaagccagga 5520
   aggacatagc actaataaaa gattccatag tcaagacaca caattctgta gaactcattc 5580
   aaagaggtat aggagaacag ataattgcat taaagacatt acaagatttt gtaaatgacg 5640
   agataagacc tgcaatagga gaactaaggt gtgagactac ggcattgaaa ctagggatca 5700
   agctcaccca acactactct gaattagcaa cagcattcag ctccaatctt gggactatag 5760
   gagaaaaaag tcttaccttg caggcattat catctctcta ctctgctaat ataacagaaa 5820
   ttctaagtac aactaaaaag gataaatcag atatatatga catcatttac actgaacagg 5880
   ttaagggaac tgtgatagat gttgatttgg aaaaatacat ggttaccctc ttagttaaaa 5940
   taccaatttt atcagaaata ccaggcgtgt tgatatacag agcttcatct atatcttata 6000
   atattgaagg agaagaatgg catgtcgcaa tcccaaatta cataatcaat aaggcatcat 6060
   ccttaggagg tgcagatgtc acaaactgta tagaatcaaa attggcatat atatgtccta 6120
   gagatcctac acaattaata cctgataacc aacagaagtg tatactcggg gatgtatcaa 6180
   agtgccctgt gactaaagta ataaacaatc tagtaccaaa gttcgcattc atcaatggtg 6240
   gtgtagtggc taattgcatt gcatccacat gtacatgcgg gacaaacaga ataccagtga 6300
   atcaagatcg ctcaagagga gttacattct tgacctatac caattgcggt ttaataggta 6360
   taaatggaat agaactatat gccaataaaa ggggacgaga cactacttgg gggaatcaaa 6420
   tcatcaaagt gggtccagca gtctccatta gacctgtaga catttcttta aatcttgcat 6480
   ctgccacaaa tttcctagag gaatccaaga cagagctcat gaaggcaagg gcaatcatat 6540
   cagcagttgg aggatggcac aacacagaga gtactcagat aatcatgata ataattgtgt 6600
   gcatacttat aataatcata tgtggtatat tatactatct atacagggtt agaagactat 6660
   tagtaatgat taattcaact cataattcac ctgttaatgc ttatactctg gagtcaagaa 6720
   tgagaaatcc ctacatgggt aacaactcca attaaaaaat cagatcaagt acattgtagc 6780
   atacatacaa caatcaaatc tatccacaac ttcaccaatc aggtgtacaa caagtaagaa 6840
   aaacttaggg ttaaagacaa tccagtcaac ctataaggca acagcatccg attatacaaa 6900
   cgatggctga aaaagggaaa acaaatagtt catattggtc tacaacccga aatgacaatt 6960
   ccacggtaaa cacacacatt aatacaccag caggaaggac acacatctgg ctactgattg 7020
   caacaacaat gcatacagta ttgtccttca ttatcatgat cctatgcatt gacctaatta 7080
   taaaacaaga cacttgtatg aagacaaaca tcatgacagt atcctccatg aacgaaagtg 7140
   ccaaaataat caaagagaca atcacagaat taatcagaca agaagtaata tcaaggacca 7200
   taaacataca aagttcagta caaagcggga tcccaatatt gttaaacaag caaagcagag 7260
   atctcacaca attaatagag aagtcatgca acagacagga attggctcag atatgcgaaa 7320
   acaccattgc tattcaccat gcagacggca tatctcctct ggacccacac gatttctgga 7380
   gatgtcccgt aggggaaccc ctactgagca acaaccccaa tatctcatta ttacctggac 7440
   caagtctact ttctggatcc accacaattt caggatgtgt tagactacct tcattatcaa 7500
   ttggtgatgc aatatatgcg tattcatcaa acttaatcac tcaaggatgt gcagatatag 7560
   ggaagtcata tcaggtttta caattaggtt acatatcctt aaattcagat atgtatcctg 7620
   atttaaaccc ggtaatttct catacctatg acatcaacga caacaggaaa tcatgttctg 7680
   taatagctgc aggaacaagg ggttatcagt tatgctcctt gcccactgtg aatgagacta 7740
   cagactactc gagtgaaggt atagaagatt tagtatttga catattagat ctcaagggaa 7800
   agaccaaatc tcatcgatac aaaaatgaag atataacttt tgaccatcct ttttctgcaa 7860
   tgtatccgag tgtaggaagt gggataaaaa ttgaaaatac actcattttc ctagggtacg 7920
   gtggcttaac aactccgctc caaggcgaca ctaagtgtgt gataaacaga tgtaccaatg 7980
   ttaatcagag tgtttgcaat gatgctctta agataacttg gctaaagaaa agacaagttg 8040
   tcaatgtctt aattcgtatc aataattatt tatctgatag gccaaagatt gttgtcgaga 8100
   caattccaat aactcaaaat tacttaggtg ccgaaggtag gctacttaaa ctaggtaaaa 8160
   agatctacat atatactaga tcttcaggtt ggcactccaa cctgcaaata ggatcattag 8220
   atatcaacaa ccccatgacc attaaatggg cgcctcatga agtcctgtct cgaccaggaa 8280
   accaagactg caactggtac aacagatgtc cgagagaatg catatcaggt gtatatactg 8340
   atgcatatcc actatctcct gatgcagtca atgttgctac aaccacactg tacgcaaaca 8400
   catcacgtgt taatcccacc ataatgtact caaatacctc agaaattatc aacatgctaa 8460
   gactcaagaa tgtacaacta gaggcagcat acactactac atcatgtatc actcatttcg 8520
   ggaagggcta ctgcttccac attgttgaaa tcaaccaagc cagccttaat accttacaac 8580
   ctatgttgtt caagacaagt atccctaaaa tatgtaaaat cacatcttga gcagatcaag 8640
   acccaacact atatcaatta tgtgaaaacc agatatgatg tataaaaatt taaaaacaaa 8700
   gcatgaatag acatttatat gacaaataga ataagaaaaa cttagggtta atgcctgcct 8760
   atttgtcaaa tatggataaa caggagtcaa ctcagaattc ctcagacatc ttatatccag 8820
   aatgtcactt gaactctccg attgtaaaaa gcaagattgc tcaacttcac gttttgctag 8880
   atatcaatca accctatgat ttaaaagata acagtataat aaatatcacc aaatacaaaa 8940
   tcagaaatgg aggtttatcg ccccggcaga tcaaaatcag atcgctaggc aaaatcctta 9000
   aacaagaaat taaggatatt gatcgttaca cttttgaacc ttatccgatt ttctcattag 9060
   agttactcag actggatatc ccagaaatat gtgacaaaat aagatccatt ttttcagtct 9120
   ctgatagatt aataagagaa ctatcatctg gatttcaaga attgtggtta aatattctta 9180
   gacaattagg ctgtgttgaa gggaaagagg gatttgactc attaaaggat gtagatatca 9240
   tcccagatat aactgataaa tataataaaa acacatggta tcgcccattc ttaacatggt 9300
   ttagcatcaa atatgatatg agatggatgc aaaagaataa gtcggggaac catttagatg 9360
   tctcaaattc tcacaatttt cttgactgta aatcatatat tttgattata tatagagatt 9420
   tagtgataat aataaataaa ttaaaattaa ccggttatgt ccttacacct gaattagtat 9480
   taatgtattg tgatgttgtc gaaggaagat ggaatatgtc ttcagctgga cgactcgata 9540
   aaaggtcatc aaaaataaca tgtaaggggg aagaattatg ggagcttatc gactctttat 9600
   ttcccaatct tggtgaggat gtatataata ttatatcact actagaacct ttatcacttg 9660
   ctttaataca gttggatgac cctgtaacta atttaaaagg agctttcatg agacatgttt 9720
   tgactgagct acatacaatt ttaataaaag ataatatata cacagattca gaagcagaca 9780
   gcataatgga atcattgata aagattttca gagagacatc aattgatgaa aaagcagaaa 9840
   ttttctcctt ttttagaacg tttggacatc ctagcttaga agcaataact gctgccgata 9900
   aagtaaggac acatatgtat tcctccaaaa aaatcatact aaagacacta tatgagtgtc 9960
   atgcaatctt ctgtgcaatt ataataaacg gatatagaga aagacacggt ggtcaatggc 10020
   cgccatgcga attccccaat catgtatgtc ttgaactcaa gaatgcacaa ggatccaact 10080
   ctgcaatttc gtatgaatgt gccgtagaca attatagtag ttttatagga tttaaatttt 10140
   taaaatttat tgagcctcaa ttagatgaag atttgacaat ttatatgaag gataaggctc 10200
   tatcacctag gaaagcagca tgggattcag tatatcccga cagtaattta tattacaaag 10260
   tccctgaatc agaagagact cgtaggttaa tcgaggtttt tataaatgat aataatttta 10320
   accctgcgga tattattaat tatgtagagt caggagaatg gttaaatgac gatagcttca 10380
   acatatctta cagtctcaaa gaaaaagaaa ttaaacaaga gggtcgactc tttgccaaga 10440
   tgacatataa gatgagagca gtccaggtat tagcagaaac actactagca aaaggagtag 10500
   gtgagttatt cagtgaaaat gggatggtaa agggagaaat tgacctacta aagagactga 10560
   ctacattatc tgtctcaggt gttccaagat ccaactcagt ttacaataat cccatattac 10620
   atgagaaatt gatcaaaaat atgaataagt gcaattcaaa tgggtattgg gatgaaagaa 10680
   agaaatctaa aaatgaattc aaagctgcag actcatcaac cgaggggtat gagactctga 10740
   gctgtttttt aaccaccgat ttgaaaaaat actgtctcaa ctggagattt gaaagtacag 10800
   cgttgttcgg tcaaagatgt aatgagatat tcgggtttaa aactttcttt aactggatgc 10860
   accctattct agaaaaaagt acaatttatg taggagatcc ttactgtcca gtacctgata 10920
   gaatgcacaa agaactccaa gatcatgatg ataccggaat ctttatccat aatccaagag 10980
   ggggaataga gggttattgc cagaaattat ggacactaat ctctattagt gcaatccatc 11040
   ttgcagctgt taaagttggt gtcagagtgt cagcaatggt acaaggagac aatcaagcta 11100
   tagcagtgac atccagagtt cctgtcacac aaacctataa gcaaaaaaag actcacgtct 11160
   atgaagaaat cacaagatat ttcggtgcct tgagagaagt tatgtttgat attggacatg 11220
   aattaaaatt aaatgagacc attataagta gcaaaatgtt tgtatacagc aaacggatat 11280
   attatgatgg gaaaatcctc ccacagtgcc tcaaagcttt aacaagatgt gtattttggt 11340
   cagagactct tgtagatgaa aacaggtcag catgctcaaa cattgcaaca tctatagcca 11400
   aagctattga gaatggatat tcacctatct taggctattg tattgctctt tttaaaactt 11460
   gccaacaggt atgtatatca ttaggaatga ccattaatcc tactattacg tcaactatca 11520
   aagatcaata ttttaaaggg aaaaattggt taagatgtgc aatattgatc ccagctaaca 11580
   taggagggtt caactatatg tctacagcta gatgttttgt cagaaatata ggtgatccag 11640
   cagttgcagc tctagcagac ttaaagagat tcatcaaagc aggtctgtta gataaacagg 11700
   tattatatcg tgtgatgaat caagaaccag gagactcaag cttcttagat tgggcatcag 11760
   acccttattc atgcaatctc ccacactcac aaagtataac aactataatc aaaaatgtaa 11820
   cagctagatc agtattgcag gaatcaccta atcctctcct atcaggtctc ttttcagaat 11880
   caagtagtga agaagatctc aacttagcat catttttgat ggataggaaa gccatattgc 11940
   ccagagtagc tcacgagatc ttagataact cacttacagg tgtaagagaa gctatagccg 12000
   ggatgcttga tacaacgaaa tctctagtaa gagctagtgt caggagagga ggattatcat 12060
   atagtatctt aagaagactt ataaattatg atctattaca atatgagacc ttaacaagga 12120
   cactcagaaa accggttaag gataatatag aatatgagta tatgtgttca gtagaattgg 12180
   caataggatt gaggcaaaaa atgtggtttc atctaactta tggaagacca atccacggtt 12240
   tagaaactcc agacccgtta gaattattaa gaggatcatt tattgaaggc tcagaaatat 12300
   gtaaattttg tagatcagaa gggaataacc ctatgtatac ttggttctat cttcctgaca 12360
   acatcgactt agatacactt agcaatggaa gtcctgccat acgtatccct tattttggtt 12420
   ctgctactga tgaaagatca gaggctcaac taggttatgt taagaactta agcaagccgg 12480
   caaaagcagc aataagaatc gcaatggttt acacttgggc ttatggaact gatgaaatat 12540
   catggatgga agcagcactt atagctcaaa ccagggctaa cttaagttta gagaatttga 12600
   agttactcac ccctgtatcg acttctacaa atttgtccca cagattgaga gatactgcta 12660
   cacagatgaa attttcaagt gctactttag ttcgagcgag tcgatttatt accatatcta 12720
   atgataatat ggcattaaaa gaggcaggag agtctaaaga tactaattta gtttatcaac 12780
   aaattatgtt aaccggattg agcttatttg aattcaatat gaggtataaa caaggatcat 12840
   tatctaaacc tatgatatta cacttacatt tgaataataa atgctgtatc atagaatctc 12900
   ctcaagaatt gaatattcct cctagatcta cattggactt agagatcact caggaaaata 12960
   acaagttaat ctatgatcct gatcctctca aggacataga tctagagtta tttagtaagg 13020
   ttagggatgt agtacacaca attgatatga attattggtc tgatgatgaa ataattagag 13080
   caactagtat atgtacagct atgactattg cagacacaat gtctcaatta gatagagaca 13140
   atcttaaaga aatgatagca ctgataaatg atgatgatat aaatagttta atcaccgaat 13200
   ttatggttat tgatataccc ttattttgtt ccactttcgg gggtattcta atcaatcaat 13260
   ttgcatattc actttacggg ttaaacgtca gagggaggga tgaaatatgg ggatatgtga 13320
   tacgcataat taaagacaca tcacatgcag tcctaaaagt actgtccaat gcattatcac 13380
   atcctaaaat attcaaacga ttctgggatg caggagttgt agagcctgtt tatggaccta 13440
   acttgtccaa tcaagacaag atactgttag ccatttcagt atgtgaatac tctgttgacc 13500
   tcttcatgcg tgattggcaa gagggcatac cgcttgaaat atttatttgt gataacgacc 13560
   caaatatagc agaaatgaga aaactttcat ttttagctag acatctagca tacttgtgta 13620
   gtttggcaga gatagctaaa gagggaccaa aattggaatc tatgacatct ctcgaacgac 13680
   tcgaatcatt gaaagagtat ctagaactta cttttttaga cgatcctata ttaagatata 13740
   gtcaattgac aggcttagtt attaagatat tcccttcaac gttaacttac atcaggaaat 13800
   cttcaattaa ggtgttgaga gtaagaggta tagggatacc agaagtctta gaggactggg 13860
   atcctgatgc cgatagtatg ctactagata atataactgc tgaggttcaa cacaatatac 13920
   ctttaaagaa gaacgaaaga actcccttct gggggttaag ggtatcaaaa tcacaagttc 13980
   tgcgacttag aggttatgaa gagataaaaa gggaagaaag aggaagatca ggtgtaggat 14040
   taactctacc ttttgatggg cgatatttat cacaccaatt gagacttttc gggattaata 14100
   gcaccagttg tttgaaagca ttggaactta cctatttact gaatcctcta gtcaataagg 14160
   ataaagatag attatatctc ggagaaggtg caggtgcaat gctgtcttgt tatgatgcta 14220
   cattaggacc ctgcatgaac tattataatt caggtgttaa ttcttgtgat ctcaacggac 14280
   aaagagaatt aaatatttat ccttcagaag tggcactggt agggaagaaa ttgaataatg 14340
   tcacgagttt atgtcaaaga gttaaggttt tattcaatgg gaatcctgga tcaacttgga 14400
   tagggaatga tgaatgtgaa acactaatct ggaatgaatt acagaataat tcaatagggt 14460
   ttattcattg tgacatggaa ggtggagaac acaaatgtga tcaggtggtc ttacatgaac 14520
   attatagtgt gatcaggatt gcataccttg ttggggataa ggacgttatc ttagtaagca 14580
   aaattgcacc aagattaggt acagactgga caaaacaatt aagtttgtat ttaagatact 14640
   ggagagatgt cagcttaata gtgttgaaaa catctaaccc agcctctaca gaaatgtatc 14700
   tgatatcaaa agatcctaaa tctgatatta tagaggatag taatacagta ttggcaaacc 14760
   ttcttccatt atctaaagag gatagtatta agatagaaaa atggattcta gttgagaaag 14820
   ccaaagttca tgattggata gttagagaat taaaggaagg gagtgcatcg tcaggtatgc 14880
   taagacctta ccatcaagca ttacaaatct tcggatttga gcctaattta aacaaattat 14940
   gtagagattt cttatctaca ctaaatatag tagacacaaa aaattgtatt atcacatttg 15000
   atagagtatt aagagataca atctttgagt ggactcggat aaaagacgca gataagaagc 15060
   taagacttac aggtaaatat gatctatatc ctcttagaga ttcaggtaag ttaaaagtta 15120
   tttctagaag gcttgtaata tcttggatag cattgtctat gtctacaaga ctagtaacag 15180
   ggtcatttcc agacattaaa tttgaatcaa gactccaatt aggtatagta tcaatatcct 15240
   ctcgtgaaat caaaaatctt agggttatat caaagattgt cattgacaaa tttgaagata 15300
   ttatacatag tgtgacctat aggttcttga ctaaagaaat aaaaatattg atgaaaattt 15360
   tgggagcagt caaattattt ggggcaagac agagcacatc tgctgatatc actaatatcg 15420
   atacatcgga ctccatacaa tgatcttata tcttctcatc tttattatct aatttgttta 15480
   aagagatgag ttaacaagat aagaaatccc tttaactgac tcataaaaac atagtaagaa 15540
   aaacttacaa cagacaagag tattaataat atatcgatat ttcttaaact cttgtctggt 15600
<210> 2
   <211> 16242
   <212> DNA
   <213> Human parainfluenza virus
<400> 2
<210> 3
   <211> 16236
   <212> DNA
   <213> Human parainfluenza virus
<400> 3
   accaaacaag aggaaaaact tgtttggaat atataataat attaaatagt attttagggt 60
   taaagtaata ctttaaaggg acaagtcaca gacatttgat cttagtataa atacgcgtaa 120
   tggccgggct actaagtact tttgacacat ttagttccag gagaagtgag agcatcaata 180
   agtctggcgg aggagcaatt atacctggtc aaagaagtac cgtttctgtc ttcacattag 240
   gcccgagtgt gacagatgat gcagataaat tattaatagc aaccactttc ttagcccact 300
   cattggacac agataaacaa cactctcaaa gaggaggatt tttagtatca ctccttgcaa 360
   tggcctacag tagtccggag ttatatctca ctacaaacgg tgtcaatgct gatgtcaagt 420
   atgtgatata caatatagag agagatccta aaagaacaaa aacagatggg ttcattgtca 480
   aaacaagaga catggagtat gaaagaacaa cagagtggtt gtttggacct atgatcaaca 540
   agaacccatt gttccaaggg caaagagaga atgcggatct agaggcattg cttcagacat 600
   atggatatcc tgcatgtctc ggagctataa tagttcaagt ttggatagtg ttggttaaag 660
   ccataacaag tagtgctggt ctaagaaaag gattcttcaa tagattagaa gcattcagac 720
   aggatggaac cgttaaaagt gccctggtct tcacaggaga cacagttgaa ggtattggtg 780
   cagtgatgag gtcacaacaa agcttagtat ctcttatggt agaaactctg gtgactatga 840
   acacatccag gtcagattta actacattag agaagaacat tcagattgta ggaaattaca 900
   taagagatgc aggattagca tctttcatga acaccatcaa gtatggtgta gaaacgaaga 960
   tggccgccct gacactatca aatctgagac cagatataaa caaattgaga agccttgttg 1020
   atatctatct atcaaaggga gcccgagccc cttttatatg tatactcaga gacccagttc 1080
   atggagactt tgcccctgga aactatccag cactgtggag ctacgcaatg ggcgttgctg 1140
   tggtacaaaa caaagctatg caacagtatg taactggaag aacatatttg gacatggaaa 1200
   tgttcctact tggacaagct gtagctaaag atgctgattc caaaatcagc agtgctctgg 1260
   aggaagaact aggtgtgaca gatacagcaa aagagagact aagacaccat ctgacaaacc 1320
   tttcaggagg ggatggtgcg taccacaagc ctacaggtgg tggagctata gaagtggcaa 1380
   ttgatcatac agacataaca tttggagtcg aggacactgc tgatcgggac aacaagaact 1440
   ggacaaatga cagcaatgaa agatggatga atcactcgat cagcaaccac acaatcacga 1500
   ttcgtggtgc agaagaactt gaagaagaga caaatgatga agacatcact gatatagaaa 1560
   acaagattgc acgaaggctg gccgacagaa aacagagact aagccaggca aacaataaac 1620
   gagacaccag cagtgatgct gactatgaga atgatgatga tgctacagcg gctgcaggga 1680
   taggaggaat ttaacaggat acttggacaa tagaagccag atcaaaagta agaaaaactt 1740
   agggtgaatg acaattcaca gatcagctca accagggcgc gccagcatac acgaaaccaa 1800
   ccttcacagt ggatacctca gcatccaaaa ctctccttcc cgagtggatt aggatgccta 1860
   gttttttgag agggatcctg aagccgaagg agaggcacca cgaaaacaag aatcactctc 1920
   agatgtcatc ggactccttg acgtcgtctt atcctacaag cccacagaaa ttggagaaga 1980
   cagaagctgg ctccatggta tcatcgacaa cccaaaagaa aacaagccat catgcaaagc 2040
   cgacgataac aacaaagaca gagcaatctc aacgtcgacc caagatcata gatcaagtgg 2100
   tggaatctct aggagaacaa gtgagtcaaa aacagagaca catgctagaa tccttgatca 2160
   acaaggtata cacagggcct ctaggcgagg aactagtcca aaccctctac ctgagaatat 2220
   gggcaatgaa agaaacacca gaatcgacga agattctcca aatgagagaa gacatcagag 2280
   atcagtactt acggatgaag acagaaagat ggctgagaac tctaataaga gggaagaaga 2340
   ccaagttgag ggatttccag aagaggtacg aagaagtaca cccttatctg atgatggaga 2400
   gggtagaaca aataataatg gaagaagcat ggaaactagc agcacacata gtacaagaat 2460
   aactgatgtc attaccaacc caagtttaat taagaaaaac ttagggttaa agcccaacat 2520
   ggatcaggac gccttctttt ttgagaggga tcctgaagcc gaaggagagg caccacgaaa 2580
   acaagaatca ctctcagatg tgatcggact ccttgacgta gtcttatcct acaagcccac 2640
   agaaatagga gaagacagaa gctggctcca tggtatcatc gacaacccaa aagaaaacaa 2700
   gccatcatgc aaagccgacg ataacaacaa agacagagca atctcaacgt cgacccaaga 2760
   tcatagatca agtgaggaga gtggaatctc taggagaaca agtgagtcaa aaacagagac 2820
   acatgctaga atccttgatc aacaaggtat acacagggcc tctaggcgag gaactagtcc 2880
   aaaccctcta cctgagaata tgggcaatga aagaaacacc agaatcgacg aagattctcc 2940
   aaatgagaga agacatcaga gatcagtact tacggatgaa gacagaaaga tggctgagaa 3000
   ctctaataag agggaagaag accaagttga gggatttcca gaagaggtac gaagaagtac 3060
   acccttatct gatgatggag agggtagaac aaataataat ggaagaagca tggaaactag 3120
   cagcacacat agtacaagaa taactgatgt cattaccaac ccaagtccag agcttgaaga 3180
   tgccgttcta caaaggaaca aaagacggcc gacgaccatc aagcgtaacc aaacaagatc 3240
   agagagaaca cagagttcag aactccacaa atcaacaagt gaaaatagct ccaacctcga 3300
   agaccacaac accaaaacca gcccaaaagt tccaccgtca aagaacgaag agtcagcagc 3360
   cactccaaag aacaaccaca accacagaaa aacaagatac acaacaaaca atgcaaacaa 3420
   caacacaaaa agtccaccaa ctcccgaaca cgacgcaacc gcaaatgaag aggaaaccag 3480
   caacacatcg gtcgatgaga tggccaagtt attagtaagt cttggtgtaa tgaaatcaca 3540
   acatgaattt gaattatcta ggagtgcaag tcatgtattt gctaagcgca tgttaaaatc 3600
   tgcaaattac aaagaaatga catttaatct ctgtggtatg cttatatcag ttgaaaaatc 3660
   acttgagaat aaagtagaag aaaatagaac attacttaaa caaattcaag aggaaataaa 3720
   ttcatccagg gatcttcaca aacggttctc ggaataccaa aaagaacaga actcactcat 3780
   gatggccaat ctatccacac tccatataat tacagataga ggcgggaaaa cgggaaatcc 3840
   cagtgatact acaaggtcac catcagtctt cacaaaaggg aaagacaata aggtcaaaaa 3900
   gacaaggttt gacccctcta tggaagctct aggaggtcaa gagttcaagc ctgacttgat 3960
   aagagaggat gaactgagag atgacatcaa aaatccggta ctagaagaaa acaacaatga 4020
   gcctcaagca tccaatgcat cacgcctgat tccgtccact gaaaaacaca ctctgcactc 4080
   actcaaacta gttatcgaaa acagtcctct aagcagagta gagaagaagg cttacatcaa 4140
   atccctttat aagtgtcgga caaaccaaga ggttaaaaat gtaatggagc tattcgagga 4200
   agacatagat tcactaacta actaaacatg aatctacagc tagcgcggcc gcaaaacaat 4260
   taagaaaaac ttagggtcaa agaaattttg cccggagaaa ggaaatggct gaaacataca 4320
   ggttccccag attctcacac gaagaaaatg ggacagtaga acctctccct ctcaaaacag 4380
   gtcctgacaa aaaagcaatc cctcacatca gaatagtcaa ggtaggagat cctccaaaac 4440
   atggagtcag gtatcttgat gtgctactat tgggattctt tgaaacacct aagcaaggac 4500
   ctctatctgg cagcatatct gatctcacag aatcaaccag ttattcaatc tgtggatccg 4560
   gatccttacc aattggcata gccaagtatt acggcacaga tcaagaatta ttaaaagcct 4620
   gcattgacct caaaataact gtacgaagaa cagttagatc tggagaaatg atagtataca 4680
   tggtagattc gatccatgct cctctactac catggtccag ccgactgaga caagggatga 4740
   tatataatgc caataaagta gctctagcac ctcaatgtct cccagtcgac aaagacatca 4800
   gattcagggt tgtatttgtc aatggaacat cactaggtac aattacaatt gctaaggtcc 4860
   caaaaactct tgcagatctt gcattaccga actcaatatc agtgaatctg ctggttacac 4920
   ttagggcagg agtatcaacg gaacaaaaag gaatcctccc cgttctagac gatgatggag 4980
   aaaagaagct caacttcatg gtacacctag gaatcataag aagaaaagtt gggaagatat 5040
   attcagttga atactgcaaa aataaaattg agaagatgaa gctaatattc tctctcgggc 5100
   ttgtaggtgg aataagtttc catgtacatg caacaggcac attatccaaa actctaatga 5160
   gccaacttgc atggaaaaaa gcagtttgct atcctttaat ggatgtaaat ccacatatga 5220
   atctagtcat ctgggcagct tcagtagaaa tcacaagtgt cgatgctgtg ttccaacctg 5280
   caattccgaa agaatttcgc tattacccaa atgttgttgc aaaaagcatc gggaaaatca 5340
   ggaggatata agtctacact cctcaataat gacacccatt agctctaaat cgtaccatta 5400
   atcaaataca gatcaattcg atacaatcag ttcaaataag aaaaacgtag ggacaaagtc 5460
   ctctaccaac atcaaggaag acaagagtct caaaaagctc agcctaagca gagagaaaaa 5520
   caacaacaca aagaaagaaa aggacaagat cacaaacaag aacaaaagca aaaacaaaaa 5580
   caagaacaaa aaagggaaga aaaacaaaag tatacacaaa aaccaaaaaa gaaaaaaggc 5640
   cagagacaaa aacggaggca agaacaaaaa tttaaacaaa aacagaattt aaattcataa 5700
   taaacaccaa gatagagaca aaaatgcaaa aatcagagat cctcttctta gtatactcaa 5760
   gcttgctatt atcttcatca ttatgtcaaa ttccggtaga aaaactttca aatgtagggg 5820
   ttataatcaa tgagggcaaa ttacttaaaa tagcaggatc ttatgaatct agatacatag 5880
   tgttaagctt ggtaccttca attgacctac aagatggatg tggaacaact caaattattc 5940
   aatacaagaa tttattaaat agacttctaa ttcctctgaa ggatgccttg gatcttcagg 6000
   aatccctgat aacaataact aatgacacca ctgtgacaaa tgataatcca caaactagat 6060
   tctttggtgc tgtcattggt accatagcac taggagtagc cacagctgct caaataactg 6120
   cgggcattgc attagctgaa gcacgagaag ccaggaagga catagcacta ataaaagatt 6180
   ccatagtcaa gacacacaat tctgtagaac tcattcaaag aggtatagga gaacagataa 6240
   ttgcattaaa gacattacaa gattttgtaa atgacgagat aagacctgca ataggagaac 6300
   taaggtgtga gactacggca ttgaaactag ggatcaagct cacccaacac tactctgaat 6360
   tagcaacagc attcagctcc aatcttggga ctataggaga aaaaagtctt accttgcagg 6420
   cattatcatc tctctactct gctaatataa cagaaattct aagtacaact aaaaaggata 6480
   aatcagatat atatgacatc atttacactg aacaggttaa gggaactgtg atagatgttg 6540
   atttggaaaa atacatggtt accctcttag ttaaaatacc aattttatca gaaataccag 6600
   gcgtgttgat atacagagct tcatctatat cttataatat tgaaggagaa gaatggcatg 6660
   tcgcaatccc aaattacata atcaataagg catcatcctt aggaggtgca gatgtcacaa 6720
   actgtataga atcaaaattg gcatatatat gtcctagaga tcctacacaa ttaatacctg 6780
   ataaccaaca gaagtgtata ctcggggatg tatcaaagtg ccctgtgact aaagtaataa 6840
   acaatctagt accaaagttc gcattcatca atggtggtgt agtggctaat tgcattgcat 6900
   ccacatgtac atgcgggaca aacagaatac cagtgaatca agatcgctca agaggagtta 6960
   cattcttgac ctataccaat tgcggtttaa taggtataaa tggaatagaa ctatatgcca 7020
   ataaaagggg acgagacact acttggggga atcaaatcat caaagtgggt ccagcagtct 7080
   ccattagacc tgtagacatt tctttaaatc ttgcatctgc cacaaatttc ctagaggaat 7140
   ccaagacaga gctcatgaag gcaagggcaa tcatatcagc agttggagga tggcacaaca 7200
   cagagagtac tcagataatc atgataataa ttgtgtgcat acttataata atcatatgtg 7260
   gtatattata ctatctatac agggttagaa gactattagt aatgattaat tcaactcata 7320
   attcacctgt taatgcttat actctggagt caagaatgag aaatccctac atgggtaaca 7380
   actccaatta aaaaatcaga tcaagtacat tgtagcatac atacaacaat caaatctatc 7440
   cacaacttca ccaatcaggt gtacaacaag taagaaaaac ttagggttaa agacaatcca 7500
   gtcaacctat aaggcaacag catccgatta tacaaacgat ggctgaaaaa gggaaaacaa 7560
   atagttcata ttggtctaca acccgaaatg acaattccac ggtaaacaca cacattaata 7620
   caccagcagg aaggacacac atctggctac tgattgcaac aacaatgcat acagtattgt 7680
   ccttcattat catgatccta tgcattgacc taattataaa acaagacact tgtatgaaga 7740
   caaacatcat gacagtatcc tccatgaacg aaagtgccaa aataatcaaa gagacaatca 7800
   cagaattaat cagacaagaa gtaatatcaa ggaccataaa catacaaagt tcagtacaaa 7860
   gcgggatccc aatattgtta aacaagcaaa gcagagatct cacacaatta atagagaagt 7920
   catgcaacag acaggaattg gctcagatat gcgaaaacac cattgctatt caccatgcag 7980
   acggcatatc tcctctggac ccacacgatt tctggagatg tcccgtaggg gaacccctac 8040
   tgagcaacaa ccccaatatc tcattattac ctggaccaag tctactttct ggatccacca 8100
   caatttcagg atgtgttaga ctaccttcat tatcaattgg tgatgcaata tatgcgtatt 8160
   catcaaactt aatcactcaa ggatgtgcag atatagggaa gtcatatcag gttttacaat 8220
   taggttacat atccttaaat tcagatatgt atcctgattt aaacccggta atttctcata 8280
   cctatgacat caacgacaac aggaaatcat gttctgtaat agctgcagga acaaggggtt 8340
   atcagttatg ctccttgccc actgtgaatg agactacaga ctactcgagt gaaggtatag 8400
   aagatttagt atttgacata ttagatctca agggaaagac caaatctcat cgatacaaaa 8460
   atgaagatat aacttttgac catccttttt ctgcaatgta tccgagtgta ggaagtggga 8520
   taaaaattga aaatacactc attttcctag ggtacggtgg cttaacaact ccgctccaag 8580
   gcgacactaa gtgtgtgata aacagatgta ccaatgttaa tcagagtgtt tgcaatgatg 8640
   ctcttaagat aacttggcta aagaaaagac aagttgtcaa tgtcttaatt cgtatcaata 8700
   attatttatc tgataggcca aagattgttg tcgagacaat tccaataact caaaattact 8760
   taggtgccga aggtaggcta cttaaactag gtaaaaagat ctacatatat actagatctt 8820
   caggttggca ctccaacctg caaataggat cattagatat caacaacccc atgaccatta 8880
   aatgggcgcc tcatgaagtc ctgtctcgac caggaaacca agactgcaac tggtacaaca 8940
   gatgtccgag agaatgcata tcaggtgtat atactgatgc atatccacta tctcctgatg 9000
   cagtcaatgt tgctacaacc acactgtacg caaacacatc acgtgttaat cccaccataa 9060
   tgtactcaaa tacctcagaa attatcaaca tgctaagact caagaatgta caactagagg 9120
   cagcatacac tactacatca tgtatcactc atttcgggaa gggctactgc ttccacattg 9180
   ttgaaatcaa ccaagccagc cttaatacct tacaacctat gttgttcaag acaagtatcc    9240
   ctaaaatatg taaaatcaca tcttgagcag atcaagaccc aacactatat caattatgtg    9300
   aaaaccagat atgatgtata aaaatttaaa aacaaagcat gaatagacat ttatatgaca    9360
   aatagaataa gaaaaactta gggttaatgc ctgcctattt gtcaaatatg gataaacagg    9420
   agtcaactca gaattcctca gacatcttat atccagaatg tcacttgaac tctccgattg    9480
   taaaaagcaa gattgctcaa cttcacgttt tgctagatat caatcaaccc tatgatttaa    9540
   aagataacag tataataaat atcaccaaat acaaaatcag aaatggaggt ttatcgcccc    9600
   ggcagatcaa aatcagatcg ctaggcaaaa tccttaaaca agaaattaag gatattgatc    9660
   gttacacttt tgaaccttat ccgattttct cattagagtt actcagactg gatatcccag    9720
   aaatatgtga caaaataaga tccatttttt cagtctctga tagattaata agagaactat    9780
   catctggatt tcaagaattg tggttaaata ttcttagaca attaggctgt gttgaaggga    9840
   aagagggatt tgactcatta aaggatgtag atatcatccc agatataact gataaatata    9900
   ataaaaacac atggtatcgc ccattcttaa catggtttag catcaaatat gatatgagat    9960
   ggatgcaaaa gaataagtcg gggaaccatt tagatgtctc aaattctcac aattttcttg    10020
   actgtaaatc atatattttg attatatata gagatttagt gataataata aataaattaa    10080
   aattaaccgg ttatgtcctt acacctgaat tagtattaat gtattgtgat gttgtcgaag    10140
   gaagatggaa tatgtcttca gctggacgac tcgataaaag gtcatcaaaa ataacatgta    10200
   agggggaaga attatgggag cttatcgact ctttatttcc caatcttggt gaggatgtat    10260
   ataatattat atcactacta gaacctttat cacttgcttt aatacagttg gatgaccctg    10320
   taactaattt aaaaggagct ttcatgagac atgttttgac tgagctacat acaattttaa    10380
   taaaagataa tatatacaca gattcagaag cagacagcat aatggaatca ttgataaaga    10440
   ttttcagaga gacatcaatt gatgaaaaag cagaaatttt ctcctttttt agaacgtttg    10500
   gacatcctag cttagaagca ataactgctg ccgataaagt aaggacacat atgtattcct    10560
   ccaaaaaaat catactaaag acactatatg agtgtcatgc aatcttctgt gcaattataa    10620
   taaacggata tagagaaaga cacggtggtc aatggccgcc atgcgaattc cccaatcatg    10680
   tatgtcttga actcaagaat gcacaaggat ccaactctgc aatttcgtat gaatgtgccg    10740
   tagacaatta tagtagtttt ataggattta aatttttaaa atttattgag cctcaattag    10800
   atgaagattt gacaatttat atgaaggata aggctctatc acctaggaaa gcagcatggg    10860
   attcagtata tcccgacagt aatttatatt acaaagtccc tgaatcagaa gagactcgta    10920
   ggttaatcga ggtttttata aatgataata attttaaccc tgcggatatt attaattatg    10980
   tagagtcagg agaatggtta aatgacgata gcttcaacat atcttacagt ctcaaagaaa    11040
   aagaaattaa acaagagggt cgactctttg ccaagatgac atataagatg agagcagtcc 11100
   aggtattagc agaaacacta ctagcaaaag gagtaggtga gttattcagt gaaaatggga 11160
   tggtaaaggg agaaattgac ctactaaaga gactgactac attatctgtc tcaggtgttc 11220
   caagatccaa ctcagtttac aataatccca tattacatga gaaattgatc aaaaatatga 11280
   ataagtgcaa ttcaaatggg tattgggatg aaagaaagaa atctaaaaat gaattcaaag 11340
   ccgcagactc atcaaccgag gggtatgaga ctctgagctg ttttttaacc accgatttga 11400
   aaaaatactg tctcaactgg agatttgaaa gtacagcgtt gttcggtcaa agatgtaatg 11460
   agatattcgg gtttaaaact ttctttaact ggatgcaccc tattctagaa aaaagtacaa 11520
   tttatgtagg agatccttac tgtccagtac ctgatagaat gcacaaagaa ctccaagatc 11580
   atgatgatac cggaatcttt atccataatc caagaggggg aatagagggt tattgccaga 11640
   aattatggac actaatctct attagtgcaa tccatcttgc agctgttaaa gttggtgtca 11700
   gagtgtcagc aatggtacaa ggagacaatc aagctatagc agtgacatcc agagttcctg 11760
   tcacacaaac ctataagcaa aaaaagactc acgtctatga agaaatcaca agatatttcg 11820
   gtgccttgag agaagttatg tttgatattg gacatgaatt aaaattaaat gagaccatta 11880
   taagtagcaa aatgtttgta tacagcaaac ggatatatta tgatgggaaa atcctcccac 11940
   agtgcctcaa agctttaaca agatgtgtat tttggtcaga gactcttgta gatgaaaaca 12000
   ggtcagcatg ctcaaacatt gcaacatcta tagccaaagc tattgagaat ggatattcac 12060
   ctatcttagg ctattgtatt gctcttttta aaacttgcca acaggtatgt atatcattag 12120
   gaatgaccat taatcctact attacgtcaa ctatcaaaga tcaatatttt aaagggaaaa 12180
   attggttaag atgtgcaata ttgatcccag ctaacatagg agggttcaac tatatgtcta 12240
   cagctagatg ttttgtcaga aatataggtg atccagcagt tgcagctcta gcagacttaa 12300
   agagattcat caaagcaggt ctgttagata aacaggtatt atatcgtgtg atgaatcaag 12360
   aaccaggaga ctcaagcttc ttagattggg catcagaccc ttattcatgc aatctcccac 12420
   actcacaaag tataacaact ataatcaaaa atgtaacagc tagatcagta ttgcaggaat 12480
   cacctaatcc tctcctatca ggtctctttt cagaatcaag tagtgaagaa gatctcaact 12540
   tagcatcatt tttgatggat aggaaagcca tattgcccag agtagctcac gagatcttag 12600
   ataactcact tacaggtgta agagaagcta tagccgggat gcttgataca acgaaatctc 12660
   tagtaagagc tagtgtcagg agaggaggat tatcatatag tatcttaaga agacttataa 12720
   attatgatct attacaatat gagaccttaa caaggacact cagaaaaccg gttaaggata 12780
   atatagaata tgagtatatg tgttcagtag aattggcaat aggattgagg caaaaaatgt 12840
   ggtttcatct aacttatgga agaccaatcc acggtttaga aactccagac ccgttagaat 12900
   tattaagagg atcatttatt gaaggctcag aaatatgtaa attttgtaga tcagaaggga 12960
   ataaccctat gtatacttgg ttctatcttc ctgacaacat cgacttagat acacttagca 13020
   atggaagtcc tgccatacgt atcccttatt ttggttctgc tactgatgaa agatcagagg 13080
   ctcaactagg ttatgttaag aacttaagca agccggcaaa agcagcaata agaatcgcaa 13140
   tggtttacac ttgggcttat ggaactgatg aaatatcatg gatggaagca gcacttatag 13200
   ctcaaaccag ggctaactta agtttagaga atttgaagtt actcacccct gtatcgactt 13260
   ctacaaattt gtcccacaga ttgagagata ctgctacaca gatgaaattt tcaagtgcta 13320
   ctttagttcg agcgagtcga tttattacca tatctaatga taatatggca ttaaaagagg 13380
   caggagagtc taaagatact aatttagttt atcaacaaat tatgttaacc ggattgagct 13440
   tatttgaatt caatatgagg tataaacaag gatcattatc taaacctatg atattacact 13500
   tacatttgaa taataaatgc tgtatcatag aatctcctca agaattgaat attcctccta 13560
   gatctacatt ggacttagag atcactcagg aaaataacaa gttaatctat gatcctgatc 13620
   ctctcaagga catagatcta gagttattta gtaaggttag ggatgtagta cacacaattg 13680
   atatgaatta ttggtctgat gatgaaataa ttagagcaac tagtatatgt acagctatga 13740
   ctattgcaga cacaatgtct caattagata gagacaatct taaagaaatg atagcactga 13800
   taaatgatga tgatataaat agtttaatca ccgaatttat ggttattgat atacccttat 13860
   tttgttccac tttcgggggt attctaatca atcaatttgc atattcactt tacgggttaa 13920
   acgtcagagg gagggatgaa atatggggat atgtgatacg cataattaaa gacacatcac 13980
   atgcagtcct aaaagtactg tccaatgcat tatcacatcc taaaatattc aaacgattct 14040
   gggatgcagg agttgtagag cctgtttatg gacctaactt gtccaatcaa gacaagatac 14100
   tgttagccat ttcagtatgt gaatactctg ttgacctctt catgcgtgat tggcaagagg 14160
   gcataccgct tgaaatattt atttgtgata acgacccaaa tatagcagaa atgagaaaac 14220
   tttcattttt agctagacat ctagcatact tgtgtagttt ggcagagata gctaaagagg 14280
   gaccaaaatt ggaatctatg acatctctcg aacgactcga atcattgaaa gagtatctag 14340
   aacttacttt tttagacgat cctatattaa gatatagtca attgacaggc ttagttatta 14400
   agatattccc ttcaacgtta acttacatca ggaaatcttc aattaaggtg ttgagagtaa 14460
   gaggtatagg gataccagaa gtcttagagg actgggatcc tgatgccgat agtatgctac 14520
   tagataatat aactgctgag gttcaacaca atataccttt aaagaagaac gaaagaactc 14580
   ccttctgggg gttaagggta tcaaaatcac aagttctgcg acttagaggt tatgaagaga 14640
   taaaaaggga agaaagagga agatcaggtg taggattaac tctacctttt gatgggcgat 14700
   atttatcaca ccaattgaga cttttcggga ttaatagcac cagttgtttg aaagcattgg 14760
   aacttaccta tttactgaat cctctagtca ataaggataa agatagatta tatctcggag 14820
   aaggtgcagg tgcaatgctg tcttgttatg atgctacatt aggaccctgc atgaactatt 14880
   ataattcagg tgttaattct tgcgatctca acggacaaag agaattaaat atttatcctt 14940
   cagaagtggc actggtaggg aagaaattga ataatgtcac gagtttatgt caaagagtta 15000
   aggttttatt caatgggaat cctggatcaa cttggatagg gaatgatgaa tgtgaaacac 15060
   taatctggaa tgaattacag aataattcaa tagggtttat tcattgtgac atggaaggtg 15120
   gagaacacaa atgtgatcag gtggtcttac atgaacatta tagtgtgatc aggattgcat 15180
   accttgttgg ggataaggac gttatcttag taagcaaaat tgcaccaaga ttaggtacag 15240
   actggacaaa acaattaagt ttgtatttaa gatactggag agatgtcagc ttaatagtgt 15300
   tgaaaacatc taacccagcc tctacagaaa tgtatctgat atcaaaagat cctaaatctg 15360
   atattataga ggatagtaat acagtattgg caaaccttct tccattatct aaagaggata 15420
   gtattaagat agaaaaatgg attctagttg agaaagccaa agttcatgat tggatagtta 15480
   gagaattaaa ggaagggagt gcatcgtcag gtatgctaag accttaccat caagcattac 15540
   aaatcttcgg atttgagcct aatttaaaca aattatgtag agatttctta tctacactaa 15600
   atatagtaga cacaaaaaat tgtattatca catttgatag agtattaaga gatacaatct 15660
   ttgagtggac tcggataaaa gacgcagata agaagctaag acttacaggt aaatatgatc 15720
   tatatcctct tagagattca ggtaagttaa aagttatttc tagaaggctt gtaatatctt 15780
   ggatagcatt gtctatgtct acaagactag taacagggtc atttccagac attaaatttg 15840
   aatcaagact ccaattaggt atagtatcaa tatcctctcg tgaaatcaaa aatcttaggg 15900
   ttatatcaaa gattgtcatt gacaaatttg aagatattat acatagtgtg acctataggt 15960
   tcttgactaa agaaataaaa atattgatga aaattttggg agcagtcaaa ttatttgggg 16020
   caagacagag cacatctgct gatatcacta atatcgatac atcggactcc atacaatgat 16080
   cttatatctt ctcatcttta ttatctaatt tgtttaaaga gatgagttaa caagataaga 16140
   aatcccttta actgactcat aaaaacatag taagaaaaac ttacaacaga caagagtatt 16200
   aataatatat cgatatttct taaactcttg tctggt 16236
<210> 4
   <211> 16236
   <212> DNA
   <213> Human parainfluenza virus
<400> 4
   accaaacaag aggaaaaact tgtttggaat atataataat attaaatagt attttagggt 60
   taaagtaata ctttaaaggg acaagtcaca gacatttgat cttagtataa atacgcgtaa     120
   tggccgggct actaagtact tttgacacat ttagttccag gagaagtgag agcatcaata     180
   agtctggcgg aggagcaatt atacctggtc aaagaagtac cgtttctgtc ttcacattag     240
   gcccgagtgt gacagatgat gcagataaat tattaatagc aaccactttc ttagcccact     300
   cattggacac agataaacaa cactctcaaa gaggaggatt tttagtatca ctccttgcaa     360
   tggcctacag tagtccggag ttatatctca ctacaaacgg tgtcaatgct gatgtcaagt     420
   atgtgatata caatatagag agagatccta aaagaacaaa aacagatggg ttcattgtca     480
   aaacaagaga catggagtat gaaagaacaa cagagtggtt gtttggacct atgatcaaca     540
   agaacccatt gttccaaggg caaagagaga atgcggatct agaggcattg cttcagacat     600
   atggatatcc tgcatgtctc ggagctataa tagttcaagt ttggatagtg ttggttaaag     660
   ccataacaag tagtgctggt ctaagaaaag gattcttcaa tagattagaa gcattcagac     720
   aggatggaac cgttaaaagt gccctggtct tcacaggaga cacagttgaa ggtattggtg     780
   cagtgatgag gtcacaacaa agcttagtat ctcttatggt agaaactctg gtgactatga     840
   acacatccag gtcagattta actacattag agaagaacat tcagattgta ggaaattaca     900
   taagagatgc aggattagca tctttcatga acaccatcaa gtatggtgta gaaacgaaga     960
   tggccgccct gacactatca aatctgagac cagatataaa caaattgaga agccttgttg    1020
   atatctatct atcaaaggga gcccgagccc cttttatatg tatactcaga gacccagttc    1080
   atggagactt tgcccctgga aactatccag cactgtggag ctacgcaatg ggcgttgctg    1140
   tggtacaaaa caaagctatg caacagtatg taactggaag aacatatttg gacatggaaa    1200
   tgttcctact tggacaagct gtagctaaag atgctgattc caaaatcagc agtgctctgg    1260
   aggaagaact aggtgtgaca gatacagcaa aagagagact aagacaccat ctgacaaacc    1320
   tttcaggagg ggatggtgcg taccacaagc ctacaggtgg tggagctata gaagtggcaa    1380
   ttgatcatac agacataaca tttggagtcg aggacactgc tgatcgggac aacaagaact    1440
   ggacaaatga cagcaatgaa agatggatga atcactcgat cagcaaccac acaatcacga    1500
   ttcgtggtgc agaagaactt gaagaagaga caaatgatga agacatcact gatatagaaa    1560
   acaagattgc acgaaggctg gccgacagaa aacagagact aagccaggca aacaataaac    1620
   gagacaccag cagtgatgct gactatgaga atgatgatga tgctacagcg gctgcaggga    1680
   taggaggaat ttaacaggat acttggacaa tagaagccag atcaaaagta agaaaaactt    1740
   agggtgaatg acaattcaca gatcagctca accagggcgc gccagcatac acgaaaccaa    1800
   ccttcacagt ggatacctca gcatccaaaa ctctccttcc cgagtggatt aggatgccta    1860
   gttttttgag agggatcctg aagccgaagg agaggcacca cgaaaacaag aatcactctc 1920
   agatgtcatc ggactccttg acgtcgtctt atcctacaag cccacagaaa ttggagaaga 1980
   cagaagctgg ctccatggta tcatcgacaa cccaaaagaa aacaagccat catgcaaagc 2040
   cgacgataac aacaaagaca gagcaatctc aacgtcgacc caagatcata gatcaagtga 2100
   gggaatctct aggagaacaa gtgagtcaaa aacagagaca catgctagaa tccttgatca 2160
   acaaggtata cacagggcct ctaggcgagg aactagtcca aaccctctac ctgagaatat 2220
   gggcaatgaa agaaacacca gaatcgacga agattctcca aatgagagaa gacatcagag 2280
   atcagtactt acggatgaag acagaaagat ggctgagaac tctaataaga gggaagaaga 2340
   ccaagttgag ggatttccag aagaggtacg aagaagtaca cccttatctg atgatggaga 2400
   gggtagaaca aataataatg gaagaagcat ggaaactagc agcacacata gtacaagaat 2460
   aactgatgtc attaccaacc caagtttaat taagaaaaac ttagggttaa agcccaacat 2520
   ggatcaggac gccttctttt ttgagaggga tcctgaagcc gaaggagagg caccacgaaa 2580
   acaagaatca ctctcagatg tgatcggact ccttgacgta gtcttatcct acaagcccac 2640
   agaaatagga gaagacagaa gctggctcca tggtatcatc gacaacccaa aagaaaacaa 2700
   gccatcatgc aaagccgacg ataacaacaa agacagagca atctcaacgt cgacccaaga 2760
   tcatagatca agtgaggaga gtggaatctc taggagaaca agtgagtcaa aaacagagac 2820
   acatgctaga atccttgatc aacaaggtat acacagggcc tctaggcgag gaactagtcc 2880
   aaaccctcta cctgagaata tgggcaatga aagaaacacc agaatcgacg aagattctcc 2940
   aaatgagaga agacatcaga gatcagtact tacggatgaa gacagaaaga tggctgagaa 3000
   ctctaataag agggaagaag accaagttga gggatttcca gaagaggtac gaagaagtac 3060
   acccttatct gatgatggag agggtagaac aaataataat ggaagaagca tggaaactag 3120
   cagcacacat agtacaagaa taactgatgt cattaccaac ccaagtccag agcttgaaga 3180
   tgccgttcta caaaggaaca aaagacggcc gacgaccatc aagcgtaacc aaacaagatc 3240
   agagagaaca cagagttcag aactccacaa atcaacaagt gaaaatagct ccaacctcga 3300
   agaccacaac accaaaacca gcccaaaagt tccaccgtca aagaacgaag agtcagcagc 3360
   cactccaaag aacaaccaca accacagaaa aacaagatac acaacaaaca atgcaaacaa 3420
   caacacaaaa agtccaccaa ctcccgaaca cgacgcaacc gcaaatgaag aggaaaccag 3480
   caacacatcg gtcgatgaga tggccaagtt attagtaagt cttggtgtaa tgaaatcaca 3540
   acatgaattt gaattatcta ggagtgcaag tcatgtattt gctaagcgca tgttaaaatc 3600
   tgcaaattac aaagaaatga catttaatct ctgtggtatg cttatatcag ttgaaaaatc 3660
   acttgagaat aaagtagaag aaaatagaac attacttaaa caaattcaag aggaaataaa 3720
   ttcatccagg gatcttcaca aacggttctc ggaataccaa aaagaacaga actcactcat 3780
   gatggccaat ctatccacac tccatataat tacagataga ggcgggaaaa cgggaaatcc 3840
   cagtgatact acaaggtcac catcagtctt cacaaaaggg aaagacaata aggtcaaaaa 3900
   gacaaggttt gacccctcta tggaagctct aggaggtcaa gagttcaagc ctgacttgat 3960
   aagagaggat gaactgagag atgacatcaa aaatccggta ctagaagaaa acaacaatga 4020
   gcctcaagca tccaatgcat cacgcctgat tccgtccact gaaaaacaca ctctgcactc 4080
   actcaaacta gttatcgaaa acagtcctct aagcagagta gagaagaagg cttacatcaa 4140
   atccctttat aagtgtcgga caaaccaaga ggttaaaaat gtaatggagc tattcgagga 4200
   agacatagat tcactaacta actaaacatg aatctacagc tagcgcggcc gcaaaacaat 4260
   taagaaaaac ttagggtcaa agaaattttg cccggagaaa ggaaatggct gaaacataca 4320
   ggttccccag attctcacac gaagaaaatg ggacagtaga acctctccct ctcaaaacag 4380
   gtcctgacaa aaaagcaatc cctcacatca gaatagtcaa ggtaggagat cctccaaaac 4440
   atggagtcag gtatcttgat gtgctactat tgggattctt tgaaacacct aagcaaggac 4500
   ctctatctgg cagcatatct gatctcacag aatcaaccag ttattcaatc tgtggatccg 4560
   gatccttacc aattggcata gccaagtatt acggcacaga tcaagaatta ttaaaagcct 4620
   gcattgacct caaaataact gtacgaagaa cagttagatc tggagaaatg atagtataca 4680
   tggtagattc gatccatgct cctctactac catggtccag ccgactgaga caagggatga 4740
   tatataatgc caataaagta gctctagcac ctcaatgtct cccagtcgac aaagacatca 4800
   gattcagggt tgtatttgtc aatggaacat cactaggtac aattacaatt gctaaggtcc 4860
   caaaaactct tgcagatctt gcattaccga actcaatatc agtgaatctg ctggttacac 4920
   ttagggcagg agtatcaacg gaacaaaaag gaatcctccc cgttctagac gatgatggag 4980
   aaaagaagct caacttcatg gtacacctag gaatcataag aagaaaagtt gggaagatat 5040
   attcagttga atactgcaaa aataaaattg agaagatgaa gctaatattc tctctcgggc 5100
   ttgtaggtgg aataagtttc catgtacatg caacaggcac attatccaaa actctaatga 5160
   gccaacttgc atggaaaaaa gcagtttgct atcctttaat ggatgtaaat ccacatatga 5220
   atctagtcat ctgggcagct tcagtagaaa tcacaagtgt cgatgctgtg ttccaacctg 5280
   caattccgaa agaatttcgc tattacccaa atgttgttgc aaaaagcatc gggaaaatca 5340
   ggaggatata agtctacact cctcaataat gacacccatt agctctaaat cgtaccatta 5400
   atcaaataca gatcaattcg atacaatcag ttcaaataag aaaaacgtag ggacaaagtc 5460
   ctctaccaac atcaaggaag acaagagtct caaaaagctc agcctaagca gagagaaaaa 5520
   caacaacaca aagaaagaaa aggacaagat cacaaacaag aacaaaagca aaaacaaaaa 5580
   caagaacaaa aaagggaaga aaaacaaaag tatacacaaa aaccaaaaaa gaaaaaaggc 5640
   cagagacaaa aacggaggca agaacaaaaa tttaaacaaa aacagaattt aaattcataa 5700
   taaacaccaa gatagagaca aaaatgcaaa aatcagagat cctcttctta gtatactcaa 5760
   gcttgctatt atcttcatca ttatgtcaaa ttccggtaga aaaactttca aatgtagggg 5820
   ttataatcaa tgagggcaaa ttacttaaaa tagcaggatc ttatgaatct agatacatag 5880
   tgttaagctt ggtaccttca attgacctac aagatggatg tggaacaact caaattattc 5940
   aatacaagaa tttattaaat agacttctaa ttcctctgaa ggatgccttg gatcttcagg 6000
   aatccctgat aacaataact aatgacacca ctgtgacaaa tgataatcca caaactagat 6060
   tctttggtgc tgtcattggt accatagcac taggagtagc cacagctgct caaataactg 6120
   cgggcattgc attagctgaa gcacgagaag ccaggaagga catagcacta ataaaagatt 6180
   ccatagtcaa gacacacaat tctgtagaac tcattcaaag aggtatagga gaacagataa 6240
   ttgcattaaa gacattacaa gattttgtaa atgacgagat aagacctgca ataggagaac 6300
   taaggtgtga gactacggca ttgaaactag ggatcaagct cacccaacac tactctgaat 6360
   tagcaacagc attcagctcc aatcttggga ctataggaga aaaaagtctt accttgcagg 6420
   cattatcatc tctctactct gctaatataa cagaaattct aagtacaact aaaaaggata 6480
   aatcagatat atatgacatc atttacactg aacaggttaa gggaactgtg atagatgttg 6540
   atttggaaaa atacatggtt accctcttag ttaaaatacc aattttatca gaaataccag 6600
   gcgtgttgat atacagagct tcatctatat cttataatat tgaaggagaa gaatggcatg 6660
   tcgcaatccc aaattacata atcaataagg catcatcctt aggaggtgca gatgtcacaa 6720
   actgtataga atcaaaattg gcatatatat gtcctagaga tcctacacaa ttaatacctg 6780
   ataaccaaca gaagtgtata ctcggggatg tatcaaagtg ccctgtgact aaagtaataa 6840
   acaatctagt accaaagttc gcattcatca atggtggtgt agtggctaat tgcattgcat 6900
   ccacatgtac atgcgggaca aacagaatac cagtgaatca agatcgctca agaggagtta 6960
   cattcttgac ctataccaat tgcggtttaa taggtataaa tggaatagaa ctatatgcca 7020
   ataaaagggg acgagacact acttggggga atcaaatcat caaagtgggt ccagcagtct 7080
   ccattagacc tgtagacatt tctttaaatc ttgcatctgc cacaaatttc ctagaggaat 7140
   ccaagacaga gctcatgaag gcaagggcaa tcatatcagc agttggagga tggcacaaca 7200
   cagagagtac tcagataatc atgataataa ttgtgtgcat acttataata atcatatgtg 7260
   gtatattata ctatctatac agggttagaa gactattagt aatgattaat tcaactcata 7320
   attcacctgt taatgcttat actctggagt caagaatgag aaatccctac atgggtaaca 7380
   actccaatta aaaaatcaga tcaagtacat tgtagcatac atacaacaat caaatctatc 7440
   cacaacttca ccaatcaggt gtacaacaag taagaaaaac ttagggttaa agacaatcca 7500
   gtcaacctat aaggcaacag catccgatta tacaaacgat ggctgaaaaa gggaaaacaa 7560
   atagttcata ttggtctaca acccgaaatg acaattccac ggtaaacaca cacattaata 7620
   caccagcagg aaggacacac atctggctac tgattgcaac aacaatgcat acagtattgt 7680
   ccttcattat catgatccta tgcattgacc taattataaa acaagacact tgtatgaaga 7740
   caaacatcat gacagtatcc tccatgaacg aaagtgccaa aataatcaaa gagacaatca 7800
   cagaattaat cagacaagaa gtaatatcaa ggaccataaa catacaaagt tcagtacaaa 7860
   gcgggatccc aatattgtta aacaagcaaa gcagagatct cacacaatta atagagaagt 7920
   catgcaacag acaggaattg gctcagatat gcgaaaacac cattgctatt caccatgcag 7980
   acggcatatc tcctctggac ccacacgatt tctggagatg tcccgtaggg gaacccctac 8040
   tgagcaacaa ccccaatatc tcattattac ctggaccaag tctactttct ggatccacca 8100
   caatttcagg atgtgttaga ctaccttcat tatcaattgg tgatgcaata tatgcgtatt 8160
   catcaaactt aatcactcaa ggatgtgcag atatagggaa gtcatatcag gttttacaat 8220
   taggttacat atccttaaat tcagatatgt atcctgattt aaacccggta atttctcata 8280
   cctatgacat caacgacaac aggaaatcat gttctgtaat agctgcagga acaaggggtt 8340
   atcagttatg ctccttgccc actgtgaatg agactacaga ctactcgagt gaaggtatag 8400
   aagatttagt atttgacata ttagatctca agggaaagac caaatctcat cgatacaaaa 8460
   atgaagatat aacttttgac catccttttt ctgcaatgta tccgagtgta ggaagtggga 8520
   taaaaattga aaatacactc attttcctag ggtacggtgg cttaacaact ccgctccaag 8580
   gcgacactaa gtgtgtgata aacagatgta ccaatgttaa tcagagtgtt tgcaatgatg 8640
   ctcttaagat aacttggcta aagaaaagac aagttgtcaa tgtcttaatt cgtatcaata 8700
   attatttatc tgataggcca aagattgttg tcgagacaat tccaataact caaaattact 8760
   taggtgccga aggtaggcta cttaaactag gtaaaaagat ctacatatat actagatctt 8820
   caggttggca ctccaacctg caaataggat cattagatat caacaacccc atgaccatta 8880
   aatgggcgcc tcatgaagtc ctgtctcgac caggaaacca agactgcaac tggtacaaca 8940
   gatgtccgag agaatgcata tcaggtgtat atactgatgc atatccacta tctcctgatg 9000
   cagtcaatgt tgctacaacc acactgtacg caaacacatc acgtgttaat cccaccataa 9060
   tgtactcaaa tacctcagaa attatcaaca tgctaagact caagaatgta caactagagg 9120
   cagcatacac tactacatca tgtatcactc atttcgggaa gggctactgc ttccacattg 9180
   ttgaaatcaa ccaagccagc cttaatacct tacaacctat gttgttcaag acaagtatcc 9240
   ctaaaatatg taaaatcaca tcttgagcag atcaagaccc aacactatat caattatgtg 9300
   aaaaccagat atgatgtata aaaatttaaa aacaaagcat gaatagacat ttatatgaca 9360
   aatagaataa gaaaaactta gggttaatgc ctgcctattt gtcaaatatg gataaacagg 9420
   agtcaactca gaattcctca gacatcttat atccagaatg tcacttgaac tctccgattg 9480
   taaaaagcaa gattgctcaa cttcacgttt tgctagatat caatcaaccc tatgatttaa 9540
   aagataacag tataataaat atcaccaaat acaaaatcag aaatggaggt ttatcgcccc 9600
   ggcagatcaa aatcagatcg ctaggcaaaa tccttaaaca agaaattaag gatattgatc 9660
   gttacacttt tgaaccttat ccgattttct cattagagtt actcagactg gatatcccag 9720
   aaatatgtga caaaataaga tccatttttt cagtctctga tagattaata agagaactat 9780
   catctggatt tcaagaattg tggttaaata ttcttagaca attaggctgt gttgaaggga 9840
   aagagggatt tgactcatta aaggatgtag atatcatccc agatataact gataaatata 9900
   ataaaaacac atggtatcgc ccattcttaa catggtttag catcaaatat gatatgagat 9960
   ggatgcaaaa gaataagtcg gggaaccatt tagatgtctc aaattctcac aattttcttg 10020
   actgtaaatc atatattttg attatatata gagatttagt gataataata aataaattaa 10080
   aattaaccgg ttatgtcctt acacctgaat tagtattaat gtattgtgat gttgtcgaag 10140
   gaagatggaa tatgtcttca gctggacgac tcgataaaag gtcatcaaaa ataacatgta 10200
   agggggaaga attatgggag cttatcgact ctttatttcc caatcttggt gaggatgtat 10260
   ataatattat atcactacta gaacctttat cacttgcttt aatacagttg gatgaccctg 10320
   taactaattt aaaaggagct ttcatgagac atgttttgac tgagctacat acaattttaa 10380
   taaaagataa tatatacaca gattcagaag cagacagcat aatggaatca ttgataaaga 10440
   ttttcagaga gacatcaatt gatgaaaaag cagaaatttt ctcctttttt agaacgtttg 10500
   gacatcctag cttagaagca ataactgctg ccgataaagt aaggacacat atgtattcct 10560
   ccaaaaaaat catactaaag acactatatg agtgtcatgc aatcttctgt gcaattataa 10620
   taaacggata tagagaaaga cacggtggtc aatggccgcc atgcgaattc cccaatcatg 10680
   tatgtcttga actcaagaat gcacaaggat ccaactctgc aatttcgtat gaatgtgccg 10740
   tagacaatta tagtagtttt ataggattta aatttttaaa atttattgag cctcaattag 10800
   atgaagattt gacaatttat atgaaggata aggctctatc acctaggaaa gcagcatggg 10860
   attcagtata tcccgacagt aatttatatt acaaagtccc tgaatcagaa gagactcgta 10920
   ggttaatcga ggtttttata aatgataata attttaaccc tgcggatatt attaattatg 10980
   tagagtcagg agaatggtta aatgacgata gcttcaacat atcttacagt ctcaaagaaa 11040
   aagaaattaa acaagagggt cgactctttg ccaagatgac atataagatg agagcagtcc 11100
   aggtattagc agaaacacta ctagcaaaag gagtaggtga gttattcagt gaaaatggga 11160
   tggtaaaggg agaaattgac ctactaaaga gactgactac attatctgtc tcaggtgttc 11220
   caagatccaa ctcagtttac aataatccca tattacatga gaaattgatc aaaaatatga 11280
   ataagtgcaa ttcaaatggg tattgggatg aaagaaagaa atctaaaaat gaattcaaag 11340
   ccgcagactc atcaaccgag gggtatgaga ctctgagctg ttttttaacc accgatttga 11400
   aaaaatactg tctcaactgg agatttgaaa gtacagcgtt gttcggtcaa agatgtaatg 11460
   agatattcgg gtttaaaact ttctttaact ggatgcaccc tattctagaa aaaagtacaa 11520
   tttatgtagg agatccttac tgtccagtac ctgatagaat gcacaaagaa ctccaagatc 11580
   atgatgatac cggaatcttt atccataatc caagaggggg aatagagggt tattgccaga 11640
   aattatggac actaatctct attagtgcaa tccatcttgc agctgttaaa gttggtgtca 11700
   gagtgtcagc aatggtacaa ggagacaatc aagctatagc agtgacatcc agagttcctg 11760
   tcacacaaac ctataagcaa aaaaagactc acgtctatga agaaatcaca agatatttcg 11820
   gtgccttgag agaagttatg tttgatattg gacatgaatt aaaattaaat gagaccatta 11880
   taagtagcaa aatgtttgta tacagcaaac ggatatatta tgatgggaaa atcctcccac 11940
   agtgcctcaa agctttaaca agatgtgtat tttggtcaga gactcttgta gatgaaaaca 12000
   ggtcagcatg ctcaaacatt gcaacatcta tagccaaagc tattgagaat ggatattcac 12060
   ctatcttagg ctattgtatt gctcttttta aaacttgcca acaggtatgt atatcattag 12120
   gaatgaccat taatcctact attacgtcaa ctatcaaaga tcaatatttt aaagggaaaa 12180
   attggttaag atgtgcaata ttgatcccag ctaacatagg agggttcaac tatatgtcta 12240
   cagctagatg ttttgtcaga aatataggtg atccagcagt tgcagctcta gcagacttaa 12300
   agagattcat caaagcaggt ctgttagata aacaggtatt atatcgtgtg atgaatcaag 12360
   aaccaggaga ctcaagcttc ttagattggg catcagaccc ttattcatgc aatctcccac 12420
   actcacaaag tataacaact ataatcaaaa atgtaacagc tagatcagta ttgcaggaat 12480
   cacctaatcc tctcctatca ggtctctttt cagaatcaag tagtgaagaa gatctcaact 12540
   tagcatcatt tttgatggat aggaaagcca tattgcccag agtagctcac gagatcttag 12600
   ataactcact tacaggtgta agagaagcta tagccgggat gcttgataca acgaaatctc 12660
   tagtaagagc tagtgtcagg agaggaggat tatcatatag tatcttaaga agacttataa 12720
   attatgatct attacaatat gagaccttaa caaggacact cagaaaaccg gttaaggata 12780
   atatagaata tgagtatatg tgttcagtag aattggcaat aggattgagg caaaaaatgt 12840
   ggtttcatct aacttatgga agaccaatcc acggtttaga aactccagac ccgttagaat  12900
   tattaagagg atcatttatt gaaggctcag aaatatgtaa attttgtaga tcagaaggga  12960
   ataaccctat gtatacttgg ttctatcttc ctgacaacat cgacttagat acacttagca  13020
   atggaagtcc tgccatacgt atcccttatt ttggttctgc tactgatgaa agatcagagg  13080
   ctcaactagg ttatgttaag aacttaagca agccggcaaa agcagcaata agaatcgcaa  13140
   tggtttacac ttgggcttat ggaactgatg aaatatcatg gatggaagca gcacttatag  13200
   ctcaaaccag ggctaactta agtttagaga atttgaagtt actcacccct gtatcgactt  13260
   ctacaaattt gtcccacaga ttgagagata ctgctacaca gatgaaattt tcaagtgcta  13320
   ctttagttcg agcgagtcga tttattacca tatctaatga taatatggca ttaaaagagg  13380
   caggagagtc taaagatact aatttagttt atcaacaaat tatgttaacc ggattgagct  13440
   tatttgaatt caatatgagg tataaacaag gatcattatc taaacctatg atattacact  13500
   tacatttgaa taataaatgc tgtatcatag aatctcctca agaattgaat attcctccta  13560
   gatctacatt ggacttagag atcactcagg aaaataacaa gttaatctat gatcctgatc  13620
   ctctcaagga catagatcta gagttattta gtaaggttag ggatgtagta cacacaattg  13680
   atatgaatta ttggtctgat gatgaaataa ttagagcaac tagtatatgt acagctatga  13740
   ctattgcaga cacaatgtct caattagata gagacaatct taaagaaatg atagcactga  13800
   taaatgatga tgatataaat agtttaatca ccgaatttat ggttattgat atacccttat  13860
   tttgttccac tttcgggggt attctaatca atcaatttgc atattcactt tacgggttaa  13920
   acgtcagagg gagggatgaa atatggggat atgtgatacg cataattaaa gacacatcac  13980
   atgcagtcct aaaagtactg tccaatgcat tatcacatcc taaaatattc aaacgattct  14040
   gggatgcagg agttgtagag cctgtttatg gacctaactt gtccaatcaa gacaagatac  14100
   tgttagccat ttcagtatgt gaatactctg ttgacctctt catgcgtgat tggcaagagg  14160
   gcataccgct tgaaatattt atttgtgata acgacccaaa tatagcagaa atgagaaaac  14220
   tttcattttt agctagacat ctagcatact tgtgtagttt ggcagagata gctaaagagg  14280
   gaccaaaatt ggaatctatg acatctctcg aacgactcga atcattgaaa gagtatctag  14340
   aacttacttt tttagacgat cctatattaa gatatagtca attgacaggc ttagttatta  14400
   agatattccc ttcaacgtta acttacatca ggaaatcttc aattaaggtg ttgagagtaa  14460
   gaggtatagg gataccagaa gtcttagagg actgggatcc tgatgccgat agtatgctac  14520
   tagataatat aactgctgag gttcaacaca atataccttt aaagaagaac gaaagaactc  14580
   ccttctgggg gttaagggta tcaaaatcac aagttctgcg acttagaggt tatgaagaga  14640
   taaaaaggga agaaagagga agatcaggtg taggattaac tctacctttt gatgggcgat  14700
   atttatcaca ccaattgaga cttttcggga ttaatagcac cagttgtttg aaagcattgg 14760
   aacttaccta tttactgaat cctctagtca ataaggataa agatagatta tatctcggag 14820
   aaggtgcagg tgcaatgctg tcttgttatg atgctacatt aggaccctgc atgaactatt 14880
   ataattcagg tgttaattct tgcgatctca acggacaaag agaattaaat atttatcctt 14940
   cagaagtggc actggtaggg aagaaattga ataatgtcac gagtttatgt caaagagtta 15000
   aggttttatt caatgggaat cctggatcaa cttggatagg gaatgatgaa tgtgaaacac 15060
   taatctggaa tgaattacag aataattcaa tagggtttat tcattgtgac atggaaggtg 15120
   gagaacacaa atgtgatcag gtggtcttac atgaacatta tagtgtgatc aggattgcat 15180
   accttgttgg ggataaggac gttatcttag taagcaaaat tgcaccaaga ttaggtacag 15240
   actggacaaa acaattaagt ttgtatttaa gatactggag agatgtcagc ttaatagtgt 15300
   tgaaaacatc taacccagcc tctacagaaa tgtatctgat atcaaaagat cctaaatctg 15360
   atattataga ggatagtaat acagtattgg caaaccttct tccattatct aaagaggata 15420
   gtattaagat agaaaaatgg attctagttg agaaagccaa agttcatgat tggatagtta 15480
   gagaattaaa ggaagggagt gcatcgtcag gtatgctaag accttaccat caagcattac 15540
   aaatcttcgg atttgagcct aatttaaaca aattatgtag agatttctta tctacactaa 15600
   atatagtaga cacaaaaaat tgtattatca catttgatag agtattaaga gatacaatct 15660
   ttgagtggac tcggataaaa gacgcagata agaagctaag acttacaggt aaatatgatc 15720
   tatatcctct tagagattca ggtaagttaa aagttatttc tagaaggctt gtaatatctt 15780
   ggatagcatt gtctatgtct acaagactag taacagggtc atttccagac attaaatttg 15840
   aatcaagact ccaattaggt atagtatcaa tatcctctcg tgaaatcaaa aatcttaggg 15900
   ttatatcaaa gattgtcatt gacaaatttg aagatattat acatagtgtg acctataggt 15960
   tcttgactaa agaaataaaa atattgatga aaattttggg agcagtcaaa ttatttgggg 16020
   caagacagag cacatctgct gatatcacta atatcgatac atcggactcc atacaatgat 16080
   cttatatctt ctcatcttta ttatctaatt tgtttaaaga gatgagttaa caagataaga 16140
   aatcccttta actgactcat aaaaacatag taagaaaaac ttacaacaga caagagtatt 16200
   aataatatat cgatatttct taaactcttg tctggt 16236
<210> 5
   <211> 16243
   <212> DNA
   <213> Human parainfluenza virus
<400> 5
   accaaacaag aggaaaaact tgtttggaat atataataat attaaatagt attttagggt 60
   taaagtaata ctttaaaggg acaagtcaca gacatttgat cttagtataa atacgcgtaa 120
   tggccgggct actaagtact tttgacacat ttagttccag gagaagtgag agcatcaata 180
   agtctggcgg aggagcaatt atacctggtc aaagaagtac cgtttctgtc ttcacattag 240
   gcccgagtgt gacagatgat gcagataaat tattaatagc aaccactttc ttagcccact 300
   cattggacac agataaacaa cactctcaaa gaggaggatt tttagtatca ctccttgcaa 360
   tggcctacag tagtccggag ttatatctca ctacaaacgg tgtcaatgct gatgtcaagt 420
   atgtgatata caatatagag agagatccta aaagaacaaa aacagatggg ttcattgtca 480
   aaacaagaga catggagtat gaaagaacaa cagagtggtt gtttggacct atgatcaaca 540
   agaacccatt gttccaaggg caaagagaga atgcggatct agaggcattg cttcagacat 600
   atggatatcc tgcatgtctc ggagctataa tagttcaagt ttggatagtg ttggttaaag 660
   ccataacaag tagtgctggt ctaagaaaag gattcttcaa tagattagaa gcattcagac 720
   aggatggaac cgttaaaagt gccctggtct tcacaggaga cacagttgaa ggtattggtg 780
   cagtgatgag gtcacaacaa agcttagtat ctcttatggt agaaactctg gtgactatga 840
   acacatccag gtcagattta actacattag agaagaacat tcagattgta ggaaattaca 900
   taagagatgc aggattagca tctttcatga acaccatcaa gtatggtgta gaaacgaaga 960
   tggccgccct gacactatca aatctgagac cagatataaa caaattgaga agccttgttg 1020
   atatctatct atcaaaggga gcccgagccc cttttatatg tatactcaga gacccagttc 1080
   atggagactt tgcccctgga aactatccag cactgtggag ctacgcaatg ggcgttgctg 1140
   tggtacaaaa caaagctatg caacagtatg taactggaag aacatatttg gacatggaaa 1200
   tgttcctact tggacaagct gtagctaaag atgctgattc caaaatcagc agtgctctgg 1260
   aggaagaact aggtgtgaca gatacagcaa aagagagact aagacaccat ctgacaaacc 1320
   tttcaggagg ggatggtgcg taccacaagc ctacaggtgg tggagctata gaagtggcaa 1380
   ttgatcatac agacataaca tttggagtcg aggacactgc tgatcgggac aacaagaact 1440
   ggacaaatga cagcaatgaa agatggatga atcactcgat cagcaaccac acaatcacga 1500
   ttcgtggtgc agaagaactt gaagaagaga caaatgatga agacatcact gatatagaaa 1560
   acaagattgc acgaaggctg gccgacagaa aacagagact aagccaggca aacaataaac 1620
   gagacaccag cagtgatgct gactatgaga atgatgatga tgctacagcg gctgcaggga 1680
   taggaggaat ttaacaggat acttggacaa tagaagccag atcaaaagta agaaaaactt 1740
   agggtgaatg acaattcaca gatcagctca accagggcgc gccagcatac acgaaaccaa 1800
   ccttcacata ggatacctca gcatccaaaa ctctccttcc cgagtggatt aggacgccta 1860
   gttttttgag agggatcctg aagccgaagg agaggcacca cgaaaacaag aatcactctc 1920
   agatgtgatc ggactccttg acgtagtctt atcctacaag cccacagaaa tagtgagaag 1980
   acagaagctg gctcctaggt atcatcgaca acccaaaaga aaacaagcca tcatgcaaag 2040
   ccgacgataa caacaaagac agagcaatct caacgtcgac ccaagatcat agatcaagtg 2100
   aggagagtgg aatctctagg agaacaagtg agtcaaaaac agagacacat gctagaatcc 2160
   ttgatcaaca aggtatacac agggcctcta ggcgagtaac tagtccaaac cctctacctg 2220
   agaattaggg catagaaaga aacaccagaa tcgacgaaga ttctccaaat gagagaagac 2280
   atcagagatc agtacttacg gatgaagaca gaaagatggc tgagaactct aataagaggg 2340
   aagaagacca agttgaggga tttccagtag aggtacgaag aagtacaccc ttatctgatg 2400
   atggagaggg ttgaacaaat aataatggaa gaagcatgga aactagcagc acacatagta 2460
   caagaataac tgatgtcatt accaacccaa gtttaattaa gaaaaactta gggttaaagc 2520
   ccaacatgga tcaggacgcc ttcttttttg agagggatcc tgaagccgaa ggagaggcac 2580
   cacgaaaaca agaatcactc tcagatgtga tcggactcct tgacgtagtc ttatcctaca 2640
   agcccacaga aataggagaa gacagaagct ggctccatgg tatcatcgac aacccaaaag 2700
   aaaacaagcc atcatgcaaa gccgacgata acaacaaaga cagagcaatc tcaacgtcga 2760
   cccaagatca tagatcaagt gaggagagtg gaatctctag gagaacaagt gagtcaaaaa 2820
   cagagacaca tgctagaatc cttgatcaac aaggtataca cagggcctct aggcgaggaa 2880
   ctagtccaaa ccctctacct gagaatatgg gcaatgaaag aaacaccaga atcgacgaag 2940
   attctccaaa tgagagaaga catcagagat cagtacttac ggatgaagac agaaagatgg 3000
   ctgagaactc taataagagg gaagaagacc aagttgaggg atttccagaa gaggtacgaa 3060
   gaagtacacc cttatctgat gatggagagg gtagaacaaa taataatgga agaagcatgg 3120
   aaactagcag cacacatagt acaagaataa ctgatgtcat taccaaccca agtccagagc 3180
   ttgaagatgc cgttctacaa aggaacaaaa gacggccgac gaccatcaag cgtaaccaaa 3240
   caagatcaga gagaacacag agttcagaac tccacaaatc aacaagtgaa aatagctcca 3300
   acctcgaaga ccacaacacc aaaaccagcc caaaagttcc accgtcaaag aacgaagagt 3360
   cagcagccac tccaaagaac aaccacaacc acagaaaaac aagatacaca acaaacaatg 3420
   caaacaacaa cacaaaaagt ccaccaactc ccgaacacga cgcaaccgca aatgaagagg 3480
   aaaccagcaa cacatcggtc gatgagatgg ccaagttatt agtaagtctt ggtgtaatga 3540
   aatcacaaca tgaatttgaa ttatctagga gtgcaagtca tgtatttgct aagcgcatgt 3600
   taaaatctgc aaattacaaa gaaatgacat ttaatctctg tggtatgctt atatcagttg 3660
   aaaaatcact tgagaataaa gtagaagaaa atagaacatt acttaaacaa attcaagagg 3720
   aaataaattc atccagggat cttcacaaac ggttctcgga ataccaaaaa gaacagaact 3780
   cactcatgat ggccaatcta tccacactcc atataattac agatagaggc gggaaaacgg 3840
   gaaatcccag tgatactaca aggtcaccat cagtcttcac aaaagggaaa gacaataagg 3900
   tcaaaaagac aaggtttgac ccctctatgg aagctctagg aggtcaagag ttcaagcctg 3960
   acttgataag agaggatgaa ctgagagatg acatcaaaaa tccggtacta gaagaaaaca 4020
   acaatgagcc tcaagcatcc aatgcatcac gcctgattcc gtccactgaa aaacacactc 4080
   tgcactcact caaactagtt atcgaaaaca gtcctctaag cagagtagag aagaaggctt 4140
   acatcaaatc cctttataag tgtcggacaa accaagaggt taaaaatgta atggagctat 4200
   tcgaggaaga catagattca ctaactaact aaacatgaat ctacagctag cgcggccgca 4260
   aaacaattaa gaaaaactta gggtcaaaga aattttgccc ggagaaagga aatggctgaa 4320
   acatacaggt tccccagatt ctcacacgaa gaaaatggga cagtagaacc tctccctctc 4380
   aaaacaggtc ctgacaaaaa agcaatccct cacatcagaa tagtcaaggt aggagatcct 4440
   ccaaaacatg gagtcaggta tcttgatgtg ctactattgg gattctttga aacacctaag 4500
   caaggacctc tatctggcag catatctgat ctcacagaat caaccagtta ttcaatctgt 4560
   ggatccggat ccttaccaat tggcatagcc aagtattacg gcacagatca agaattatta 4620
   aaagcctgca ttgacctcaa aataactgta cgaagaacag ttagatctgg agaaatgata 4680
   gtatacatgg tagattcgat ccatgctcct ctactaccat ggtccagccg actgagacaa 4740
   gggatgatat ataatgccaa taaagtagct ctagcacctc aatgtctccc agtcgacaaa 4800
   gacatcagat tcagggttgt atttgtcaat ggaacatcac taggtacaat tacaattgct 4860
   aaggtcccaa aaactcttgc agatcttgca ttaccgaact caatatcagt gaatctgctg 4920
   gttacactta gggcaggagt atcaacggaa caaaaaggaa tcctccccgt tctagacgat 4980
   gatggagaaa agaagctcaa cttcatggta cacctaggaa tcataagaag aaaagttggg 5040
   aagatatatt cagttgaata ctgcaaaaat aaaattgaga agatgaagct aatattctct 5100
   ctcgggcttg taggtggaat aagtttccat gtacatgcaa caggcacatt atccaaaact 5160
   ctaatgagcc aacttgcatg gaaaaaagca gtttgctatc ctttaatgga tgtaaatcca 5220
   catatgaatc tagtcatctg ggcagcttca gtagaaatca caagtgtaga tgctgtgttc 5280
   caacctgcaa ttccgaaaga atttcgctat tacccaaatg ttgttgcaaa aagcatcggg 5340
   aaaatcagga ggatataagt ctacactcct caataatgac acccattagc tctaaatcgt 5400
   accattaatc aaatacagat caattcgata caatcagttc aaataagaaa aacgtaggga 5460
   caaagtcctc taccaacatc aaggaagaca agagtctcaa aaagctcagc ctaagcagag 5520
   agaaaaacaa caacacaaag aaagaaaagg acaagatcac aaacaagaac aaaagcaaaa 5580
   acaaaaacaa gaacaaaaaa gggaagaaaa acaaaagtat acacaaaaac caaaaaagaa 5640
   aaaaggccag agacaaaaac ggaggcaaga acaaaaattt aaacaaaaac agaatttaaa 5700
   ttcataataa acaccaagat agagacaaaa atgcaaaaat cagagatcct cttcttagta 5760
   tactcaagct tgctattatc ttcatcatta tgtcaaattc cggtagaaaa actttcaaat 5820
   gtaggggtta taatcaatga gggcaaatta cttaaaatag caggatctta tgaatctaga 5880
   tacatagtgt taagcttggt accttcaatt gacctacaag atggatgtgg aacaactcaa 5940
   attattcaat acaagaattt attaaataga cttctaattc ctctgaagga tgccttggat 6000
   cttcaggaat ccctgataac aataactaat gacaccactg tgacaaatga taatccacaa 6060
   actagattct ttggtgctgt cattggtacc atagcactag gagtagccac agctgctcaa 6120
   ataactgcgg gcattgcatt agctgaagca cgagaagcca ggaaggacat agcactaata 6180
   aaagattcca tagtcaagac acacaattct gtagaactca ttcaaagagg tataggagaa 6240
   cagataattg cattaaagac attacaagat tttgtaaatg acgagataag acctgcaata 6300
   ggagaactaa ggtgtgagac tacggcattg aaactaggga tcaagctcac ccaacactac 6360
   tctgaattag caacagcatt cagctccaat cttgggacta taggagaaaa aagtcttacc 6420
   ttgcaggcat tatcatctct ctactctgct aatataacag aaattctaag tacaactaaa 6480
   aaggataaat cagatatata tgacatcatt tacactgaac aggttaaggg aactgtgata 6540
   gatgttgatt tggaaaaata catggttacc ctcttagtta aaataccaat tttatcagaa 6600
   ataccaggcg tgttgatata cagagcttca tctatatctt ataatattga aggagaagaa 6660
   tggcatgtcg caatcccaaa ttacataatc aataaggcat catccttagg aggtgcagat 6720
   gtcacaaact gtatagaatc aaaattggca tatatatgtc ctagagatcc tacacaatta 6780
   atacctgata accaacagaa gtgtatactc ggggatgtat caaagtgccc tgtgactaaa 6840
   gtaataaaca atctagtacc aaagttcgca ttcatcaatg gtggtgtagt ggctaattgc 6900
   attgcatcca catgtacatg cgggacaaac agaataccag tgaatcaaga tcgctcaaga 6960
   ggagttacat tcttgaccta taccaattgc ggtttaatag gtataaatgg aatagaacta 7020
   tatgccaata aaaggggacg agacactact tgggggaatc aaatcatcaa agtgggtcca 7080
   gcagtctcca ttagacctgt agacatttct ttaaatcttg catctgccac aaatttccta 7140
   gaggaatcca agacagagct catgaaggca agggcaatca tatcagcagt tggaggatgg 7200
   cacaacacag agagtactca gataatcatg ataataattg tgtgcatact tataataatc 7260
   atatgtggta tattatacta tctatacagg gttagaagac tattagtaat gattaattca 7320
   actcataatt cacctgttaa tgcttatact ctggagtcaa gaatgagaaa tccctacatg 7380
   ggtaacaact ccaattaaaa aatcagatca agtacattgt agcatacata caacaatcaa 7440
   atctatccac aacttcacca atcaggtgta caacaagtaa gaaaaactta gggttaaaga 7500
   caatccagtc aacctataag gcaacagcat ccgattatac aaacgatggc tgaaaaaggg 7560
   aaaacaaata gttcatattg gtctacaacc cgaaatgaca attccacggt aaacacacac 7620
   attaatacac cagcaggaag gacacacatc tggctactga ttgcaacaac aatgcataca 7680
   gtattgtcct tcattatcat gatcctatgc attgacctaa ttataaaaca agacacttgt 7740
   atgaagacaa acatcatgac agtatcctcc atgaacgaaa gtgccaaaat aatcaaagag 7800
   acaatcacag aattaatcag acaagaagta atatcaagga ccataaacat acaaagttca 7860
   gtacaaagcg ggatcccaat attgttaaac aagcaaagca gagatctcac acaattaata 7920
   gagaagtcat gcaacagaca ggaattggct cagatatgcg aaaacaccat tgctattcac 7980
   catgcagacg gcatatctcc tctggaccca cacgatttct ggagatgtcc cgtaggggaa 8040
   cccctactga gcaacaaccc caatatctca ttattacctg gaccaagtct actttctgga 8100
   tccaccacaa tttcaggatg tgttagacta ccttcattat caattggtga tgcaatatat 8160
   gcgtattcat caaacttaat cactcaagga tgtgcagata tagggaagtc atatcaggtt 8220
   ttacaattag gttacatatc cttaaattca gatatgtatc ctgatttaaa cccggtaatt 8280
   tctcatacct atgacatcaa cgacaacagg aaatcatgtt ctgtaatagc tgcaggaaca 8340
   aggggttatc agttatgctc cttgcccact gtgaatgaga ctacagacta ctcgagtgaa 8400
   ggtatagaag atttagtatt tgacatatta gatctcaagg gaaagaccaa atctcatcga 8460
   tacaaaaatg aagatataac ttttgaccat cctttttctg caatgtatcc gagtgtagga 8520
   agtgggataa aaattgaaaa tacactcatt ttcctagggt acggtggctt aacaactccg 8580
   ctccaaggcg acactaagtg tgtgataaac agatgtacca atgttaatca gagtgtttgc 8640
   aatgatgctc ttaagataac ttggctaaag aaaagacaag ttgtcaatgt cttaattcgt 8700
   atcaataatt atttatctga taggccaaag attgttgtcg agacaattcc aataactcaa 8760
   aattacttag gtgccgaagg taggctactt aaactaggta aaaagatcta catatatact 8820
   agatcttcag gttggcactc caacctgcaa ataggatcat tagatatcaa caaccccatg 8880
   accattaaat gggcgcctca tgaagtcctg tctcgaccag gaaaccaaga ctgcaactgg 8940
   tacaacagat gtccgagaga atgcatatca ggtgtatata ctgatgcata tccactatct 9000
   cctgatgcag tcaatgttgc tacaaccaca ctgtacgcaa acacatcacg tgttaatccc 9060
   accataatgt actcaaatac ctcagaaatt atcaacatgc taagactcaa gaatgtacaa 9120
   ctagaggcag catacactac tacatcatgt atcactcatt tcgggaaggg ctactgcttc 9180
   cacattgttg aaatcaacca agccagcctt aataccttac aacctatgtt gttcaagaca 9240
   agtatcccta aaatatgtaa aatcacatct tgagcagatc aagacccaac actatatcaa 9300
   ttatgtgaaa accagatatg atgtataaaa atttaaaaac aaagcatgaa tagacattta 9360
   tatgacaaat agaataagaa aaacttaggg ttaatgcctg cctatttgtc aaatatggat 9420
   aaacaggagt caactcagaa ttcctcagac atcttatatc cagaatgtca cttgaactct 9480
   ccgattgtaa aaagcaagat tgctcaactt cacgttttgc tagatatcaa tcaaccctat 9540
   gatttaaaag ataacagtat aataaatatc accaaataca aaatcagaaa tggaggttta 9600
   tcgccccggc agatcaaaat cagatcgcta ggcaaaatcc ttaaacaaga aattaaggat 9660
   attgatcgtt acacttttga accttatccg attttctcat tagagttact cagactggat 9720
   atcccagaaa tatgtgacaa aataagatcc attttttcag tctctgatag attaataaga 9780
   gaactatcat ctggatttca agaattgtgg ttaaatattc ttagacaatt aggctgtgtt 9840
   gaagggaaag agggatttga ctcattaaag gatgtagata tcatcccaga tataactgat 9900
   aaatataata aaaacacatg gtatcgccca ttcttaacat ggtttagcat caaatatgat 9960
   atgagatgga tgcaaaagaa taagtcgggg aaccatttag atgtctcaaa ttctcacaat 10020
   tttcttgact gtaaatcata tattttgatt atatatagag atttagtgat aataataaat 10080
   aaattaaaat taaccggtta tgtccttaca cctgaattag tattaatgta ttgtgatgtt 10140
   gtcgaaggaa gatggaatat gtcttcagct ggacgactcg ataaaaggtc atcaaaaata 10200
   acatgtaagg gggaagaatt atgggagctt atcgactctt tatttcccaa tcttggtgag 10260
   gatgtatata atattatatc actactagaa cctttatcac ttgctttaat acagttggat 10320
   gaccctgtaa ctaatttaaa aggagctttc atgagacatg ttttgactga gctacataca 10380
   attttaataa aagataatat atacacagat tcagaagcag acagcataat ggaatcattg 10440
   ataaagattt tcagagagac atcaattgat gaaaaagcag aaattttctc cttttttaga 10500
   acgtttggac atcctagctt agaagcaata actgctgccg ataaagtaag gacacatatg 10560
   tattcctcca aaaaaatcat actaaagaca ctatatgagt gtcatgcaat cttctgtgca 10620
   attataataa acggatatag agaaagacac ggtggtcaat ggccgccatg cgaattcccc 10680
   aatcatgtat gtcttgaact caagaatgca caaggatcca actctgcaat ttcgtatgaa 10740
   tgtgccgtag acaattatag tagttttata ggatttaaat ttttaaaatt tattgagcct 10800
   caattagatg aagatttgac aatttatatg aaggataagg ctctatcacc taggaaagca 10860
   gcatgggatt cagtatatcc cgacagtaat ttatattaca aagtccctga atcagaagag 10920
   actcgtaggt taatcgaggt ttttataaat gataataatt ttaaccctgc ggatattatt 10980
   aattatgtag agtcaggaga atggttaaat gacgatagct tcaacatatc ttacagtctc 11040
   aaagaaaaag aaattaaaca agagggtcga ctctttgcca agatgacata taagatgaga 11100
   gcagtccagg tattagcaga aacactacta gcaaaaggag taggtgagtt attcagtgaa 11160
   aatgggatgg taaagggaga aattgaccta ctaaagagac tgactacatt atctgtctca 11220
   ggtgttccaa gatccaactc agtttacaat aatcccatat tacatgagaa attgatcaaa 11280
   aatatgaata agtgcaattc aaatgggtat tgggatgaaa gaaagaaatc taaaaatgaa 11340
   ttcaaagctg cagactcatc aaccgagggg tatgagactc tgagctgttt tttaaccacc 11400
   gatttgaaaa aatactgtct caactggaga tttgaaagta cagcgttgtt cggtcaaaga 11460
   tgtaatgaga tattcgggtt taaaactttc tttaactgga tgcaccctat tctagaaaaa 11520
   agtacaattt atgtaggaga tccttactgt ccagtacctg atagaatgca caaagaactc 11580
   caagatcatg atgataccgg aatctttatc cataatccaa gagggggaat agagggttat 11640
   tgccagaaat tatggacact aatctctatt agtgcaatcc atcttgcagc tgttaaagtt 11700
   ggtgtcagag tgtcagcaat ggtacaagga gacaatcaag ctatagcagt gacatccaga 11760
   gttcctgtca cacaaaccta taagcaaaaa aagactcacg tctatgaaga aatcacaaga 11820
   tatttcggtg ccttgagaga agttatgttt gatattggac atgaattaaa attaaatgag 11880
   accattataa gtagcaaaat gtttgtatac agcaaacgga tatattatga tgggaaaatc 11940
   ctcccacagt gcctcaaagc tttaacaaga tgtgtatttt ggtcagagac tcttgtagat 12000
   gaaaacaggt cagcatgctc aaacattgca acatctatag ccaaagctat tgagaatgga 12060
   tattcaccta tcttaggcta ttgtattgct ctttttaaaa cttgccaaca ggtatgtata 12120
   tcattaggaa tgaccattaa tcctactatt acgtcaacta tcaaagatca atattttaaa 12180
   gggaaaaatt ggttaagatg tgcaatattg atcccagcta acataggagg gttcaactat 12240
   atgtctacag ctagatgttt tgtcagaaat ataggtgatc cagcagttgc agctctagca 12300
   gacttaaaga gattcatcaa agcaggtctg ttagataaac aggtattata tcgtgtgatg 12360
   aatcaagaac caggagactc aagcttctta gattgggcat cagaccctta ttcatgcaat 12420
   ctcccacact cacaaagtat aacaactata atcaaaaatg taacagctag atcagtattg 12480
   caggaatcac ctaatcctct cctatcaggt ctcttttcag aatcaagtag tgaagaagat 12540
   ctcaacttag catcattttt gatggatagg aaagccatat tgcccagagt agctcacgag 12600
   atcttagata actcacttac aggtgtaaga gaagctatag ccgggatgct tgatacaacg 12660
   aaatctctag taagagctag tgtcaggaga ggaggattat catatagtat cttaagaaga 12720
   cttataaatt atgatctatt acaatatgag accttaacaa ggacactcag aaaaccggtt 12780
   aaggataata tagaatatga gtatatgtgt tcagtagaat tggcaatagg attgaggcaa 12840
   aaaatgtggt ttcatctaac ttatggaaga ccaatccacg gtttagaaac tccagacccg 12900
   ttagaattat taagaggatc atttattgaa ggctcagaaa tatgtaaatt ttgtagatca 12960
   gaagggaata accctatgta tacttggttc tatcttcctg acaacatcga cttagataca 13020
   cttagcaatg gaagtcctgc catacgtatc ccttattttg gttctgctac tgatgaaaga 13080
   tcagaggctc aactaggtta tgttaagaac ttaagcaagc cggcaaaagc agcaataaga 13140
   atcgcaatgg tttacacttg ggcttatgga actgatgaaa tatcatggat ggaagcagca 13200
   cttatagctc aaaccagggc taacttaagt ttagagaatt tgaagttact cacccctgta 13260
   tcgacttcta caaatttgtc ccacagattg agagatactg ctacacagat gaaattttca 13320
   agtgctactt tagttcgagc gagtcgattt attaccatat ctaatgataa tatggcatta 13380
   aaagaggcag gagagtctaa agatactaat ttagtttatc aacaaattat gttaaccgga 13440
   ttgagcttat ttgaattcaa tatgaggtat aaacaaggat cattatctaa acctatgata 13500
   ttacacttac atttgaataa taaatgctgt atcatagaat ctcctcaaga attgaatatt 13560
   cctcctagat ctacattgga cttagagatc actcaggaaa ataacaagtt aatctatgat 13620
   cctgatcctc tcaaggacat agatctagag ttatttagta aggttaggga tgtagtacac 13680
   acaattgata tgaattattg gtctgatgat gaaataatta gagcaactag tatatgtaca 13740
   gctatgacta ttgcagacac aatgtctcaa ttagatagag acaatcttaa agaaatgata 13800
   gcactgataa atgatgatga tataaatagt ttaatcaccg aatttatggt tattgatata 13860
   cccttatttt gttccacttt cgggggtatt ctaatcaatc aatttgcata ttcactttac 13920
   gggttaaacg tcagagggag ggatgaaata tggggatatg tgatacgcat aattaaagac 13980
   acatcacatg cagtcctaaa agtactgtcc aatgcattat cacatcctaa aatattcaaa 14040
   cgattctggg atgcaggagt tgtagagcct gtttatggac ctaacttgtc caatcaagac 14100
   aagatactgt tagccatttc agtatgtgaa tactctgttg acctcttcat gcgtgattgg 14160
   caagagggca taccgcttga aatatttatt tgtgataacg acccaaatat agcagaaatg 14220
   agaaaacttt catttttagc tagacatcta gcatacttgt gtagtttggc agagatagct 14280
   aaagagggac caaaattgga atctatgaca tctctcgaac gactcgaatc attgaaagag 14340
   tatctagaac ttactttttt agacgatcct atattaagat atagtcaatt gacaggctta 14400
   gttattaaga tattcccttc aacgttaact tacatcagga aatcttcaat taaggtgttg 14460
   agagtaagag gtatagggat accagaagtc ttagaggact gggatcctga tgccgatagt 14520
   atgctactag ataatataac tgctgaggtt caacacaata tacctttaaa gaagaacgaa 14580
   agaactccct tctgggggtt aagggtatca aaatcacaag ttctgcgact tagaggttat 14640
   gaagagataa aaagggaaga aagaggaaga tcaggtgtag gattaactct accttttgat 14700
   gggcgatatt tatcacacca attgagactt ttcgggatta atagcaccag ttgtttgaaa 14760
   gcattggaac ttacctattt actgaatcct ctagtcaata aggataaaga tagattatat 14820
   ctcggagaag gtgcaggtgc aatgctgtct tgttatgatg ctacattagg accctgcatg 14880
   aactattata attcaggtgt taattcttgt gatctcaacg gacaaagaga attaaatatt 14940
   tatccttcag aagtggcact ggtagggaag aaattgaata atgtcacgag tttatgtcaa 15000
   agagttaagg ttttattcaa tgggaatcct ggatcaactt ggatagggaa tgatgaatgt 15060
   gaaacactaa tctggaatga attacagaat aattcaatag ggtttattca ttgtgacatg 15120
   gaaggtggag aacacaaatg tgatcaggtg gtcttacatg aacattatag tgtgatcagg 15180
   attgcatacc ttgttgggga taaggacgtt atcttagtaa gcaaaattgc accaagatta 15240
   ggtacagact ggacaaaaca attaagtttg tatttaagat actggagaga tgtcagctta 15300
   atagtgttga aaacatctaa cccagcctct acagaaatgt atctgatatc aaaagatcct 15360
   aaatctgata ttatagagga tagtaataca gtattggcaa accttcttcc attatctaaa 15420
   gaggatagta ttaagataga aaaatggatt ctagttgaga aagccaaagt tcatgattgg 15480
   atagttagag aattaaagga agggagtgca tcgtcaggta tgctaagacc ttaccatcaa 15540
   gcattacaaa tcttcggatt tgagcctaat ttaaacaaat tatgtagaga tttcttatct 15600
   acactaaata tagtagacac aaaaaattgt attatcacat ttgatagagt attaagagat 15660
   acaatctttg agtggactcg gataaaagac gcagataaga agctaagact tacaggtaaa 15720
   tatgatctat atcctcttag agattcaggt aagttaaaag ttatttctag aaggcttgta 15780
   atatcttgga tagcattgtc tatgtctaca agactagtaa cagggtcatt tccagacatt 15840
   aaatttgaat caagactcca attaggtata gtatcaatat cctctcgtga aatcaaaaat 15900
   cttagggtta tatcaaagat tgtcattgac aaatttgaag atattataca tagtgtgacc 15960
   tataggttct tgactaaaga aataaaaata ttgatgaaaa ttttgggagc agtcaaatta 16020
   tttggggcaa gacagagcac atctgctgat atcactaata tcgatacatc ggactccata 16080
   caatgatctt atatcttctc atctttatta tctaatttgt ttaaagagat gagttaacaa 16140
   gataagaaat ccctttaact gactcataaa aacatagtaa gaaaaactta caacagacaa 16200
   gagtattaat aatatatcga tatttcttaa actcttgtct ggt 16243
<210> 6
   <211> 15474
   <212> DNA
   <213> Human parainfluenza virus
<400> 6
   accaaacaag aggaaaaact tgtttggaat atataataat attaaatagt attttagggt 60
   taaagtaata ctttaaaggg acaagtcaca gacatttgat cttagtataa atacgcgtaa 120
   tggccgggct actaagtact tttgacacat ttagttccag gagaagtgag agcatcaata 180
   agtctggcgg aggagcaatt atacctggtc aaagaagtac cgtttctgtc ttcacattag 240
   gcccgagtgt gacagatgat gcagataaat tattaatagc aaccactttc ttagcccact 300
   cattggacac agataaacaa cactctcaaa gaggaggatt tttagtatca ctccttgcaa 360
   tggcctacag tagtccggag ttatatctca ctacaaacgg tgtcaatgct gatgtcaagt 420
   atgtgatata caatatagag agagatccta aaagaacaaa aacagatggg ttcattgtca 480
   aaacaagaga catggagtat gaaagaacaa cagagtggtt gtttggacct atgatcaaca 540
   agaacccatt gttccaaggg caaagagaga atgcggatct agaggcattg cttcagacat 600
   atggatatcc tgcatgtctc ggagctataa tagttcaagt ttggatagtg ttggttaaag 660
   ccataacaag tagtgctggt ctaagaaaag gattcttcaa tagattagaa gcattcagac 720
   aggatggaac cgttaaaagt gccctggtct tcacaggaga cacagttgaa ggtattggtg 780
   cagtgatgag gtcacaacaa agcttagtat ctcttatggt agaaactctg gtgactatga 840
   acacatccag gtcagattta actacattag agaagaacat tcagattgta ggaaattaca 900
   taagagatgc aggattagca tctttcatga acaccatcaa gtatggtgta gaaacgaaga 960
   tggccgccct gacactatca aatctgagac cagatataaa caaattgaga agccttgttg 1020
   atatctatct atcaaaggga gcccgagccc cttttatatg tatactcaga gacccagttc 1080
   atggagactt tgcccctgga aactatccag cactgtggag ctacgcaatg ggcgttgctg 1140
   tggtacaaaa caaagctatg caacagtatg taactggaag aacatatttg gacatggaaa 1200
   tgttcctact tggacaagct gtagctaaag atgctgattc caaaatcagc agtgctctgg 1260
   aggaagaact aggtgtgaca gatacagcaa aagagagact aagacaccat ctgacaaacc 1320
   tttcaggagg ggatggtgcg taccacaagc ctacaggtgg tggagctata gaagtggcaa 1380
   ttgatcatac agacataaca tttggagtcg aggacactgc tgatcgggac aacaagaact 1440
   ggacaaatga cagcaatgaa agatggatga atcactcgat cagcaaccac acaatcacga 1500
   ttcgtggtgc agaagaactt gaagaagaga caaatgatga agacatcact gatatagaaa 1560
   acaagattgc acgaaggctg gccgacagaa aacagagact aagccaggca aacaataaac 1620
   gagacaccag cagtgatgct gactatgaga atgatgatga tgctacagcg gctgcaggga 1680
   taggaggaat ttaacaggat acttggacaa tagaagccag atcaaaagta agaaaaactt 1740
   agggttaaag cccaacatgg atcaggacgc cttctttttt gagagggatc ctgaagccga 1800
   aggagaggca ccacgaaaac aagaatcact ctcagatgtg atcggactcc ttgacgtagt 1860
   cttatcctac aagcccacag aaataggaga agacagaagc tggctccatg gtatcatcga 1920
   caacccaaaa gaaaacaagc catcatgcaa agccgacgat aacaacaaag acagagcaat 1980
   ctcaacgtcg acccaagatc atagatcaag tgaggagagt ggaatctcta ggagaacaag 2040
   tgagtcaaaa acagagacac atgctagaat ccttgatcaa caaggtatac acagggcctc 2100
   taggcgagga actagtccaa accctctacc tgagaatatg ggcaatgaaa gaaacaccag 2160
   aatcgacgaa gattctccaa atgagagaag acatcagaga tcagtactta cggatgaaga 2220
   cagaaagatg gctgagaact ctaataagag ggaagaagac caagttgagg gatttccaga 2280
   agaggtacga agaagtacac ccttatctga tgatggagag ggtagaacaa ataataatgg 2340
   aagaagcatg gaaactagca gcacacatag tacaagaata actgatgtca ttaccaaccc 2400
   aagtccagag cttgaagatg ccgttctaca aaggaacaaa agacggccga cgaccatcaa 2460
   gcgtaaccaa acaagatcag agagaacaca gagttcagaa ctccacaaat caacaagtga 2520
   aaatagctcc aacctcgaag accacaacac caaaaccagc ccaaaagttc caccgtcaaa 2580
   gaacgaagag tcagcagcca ctccaaagaa caaccacaac cacagaaaaa caagatacac 2640
   aacaaacaat gcaaacaaca acacaaaaag tccaccaact cccgaacacg acgcaaccgc 2700
   aaatgaagag gaaaccagca acacatcggt cgatgagatg gccaagttat tagtaagtct 2760
   tggtgtaatg aaatcacaac atgaatttga attatctagg agtgcaagtc atgtatttgc 2820
   taagcgcatg ttaaaatctg caaattacaa agaaatgaca tttaatctct gtggtatgct 2880
   tatatcagtt gaaaaatcac ttgagaataa agtagaagaa aatagaacat tacttaaaca 2940
   aattcaagag gaaataaatt catccaggga tcttcacaaa cggttctcgg aataccaaaa 3000
   agaacagaac tcactcatga tggccaatct atccacactc catataatta cagatagagg 3060
   cgggaaaacg ggaaatccca gtgatactac aaggtcacca tcagtcttca caaaagggaa 3120
   agacaataag gtcaaaaaga caaggtttga cccctctatg gaagctctag gaggtcaaga 3180
   gttcaagcct gacttgataa gagaggatga actgagagat gacatcaaaa atccggtact 3240
   agaagaaaac aacaatgagc ctcaagcatc caatgcatca cgcctgattc cgtccactga 3300
   aaaacacact ctgcactcac tcaaactagt tatcgaaaac agtcctctaa gcagagtaga 3360
   gaagaaggct tacatcaaat ccctttataa gtgtcggaca aaccaagagg ttaaaaatgt 3420
   aatggagcta ttcgaggaag acatagattc actaactaac taaacatgaa tctacagcta 3480
   gcgcggccgc aaaacaatta agaaaaactt agggtcaaag aaattttgcc cggagaaagg 3540
   aaatggctga aacatacagg ttccccagat tctcacacga agaaaatggg acagtagaac 3600
   ctctccctct caaaacaggt cctgacaaaa aagcaatccc tcacatcaga atagtcaagg 3660
   taggagatcc tccaaaacat ggagtcaggt atcttgatgt gctactattg ggattctttg 3720
   aaacacctaa gcaaggacct ctatctggca gcatatctga tctcacagaa tcaaccagtt 3780
   attcaatctg tggatccgga tccttaccaa ttggcatagc caagtattac ggcacagatc 3840
   aagaattatt aaaagcctgc attgacctca aaataactgt acgaagaaca gttagatctg 3900
   gagaaatgat agtatacatg gtagattcga tccatgctcc tctactacca tggtccagcc 3960
   gactgagaca agggatgata tataatgcca ataaagtagc tctagcacct caatgtctcc 4020
   cagtcgacaa agacatcaga ttcagggttg tatttgtcaa tggaacatca ctaggtacaa 4080
   ttacaattgc taaggtccca aaaactcttg cagatcttgc attaccgaac tcaatatcag 4140
   tgaatctgct ggttacactt agggcaggag tatcaacgga acaaaaagga atcctccccg 4200
   ttctagacga tgatggagaa aagaagctca acttcatggt acacctagga atcataagaa 4260
   gaaaagttgg gaagatatat tcagttgaat actgcaaaaa taaaattgag aagatgaagc 4320
   taatattctc tctcgggctt gtaggtggaa taagtttcca tgtacatgca acaggcacat 4380
   tatccaaaac tctaatgagc caacttgcat ggaaaaaagc agtttgctat cctttaatgg 4440
   atgtaaatcc acatatgaat ctagtcatct gggcagcttc agtagaaatc acaagtgtag 4500
   atgctgtgtt ccaacctgca attccgaaag aatttcgcta ttacccaaat gttgttgcaa 4560
   aaagcatcgg gaaaatcagg aggatataag tctacactcc tcaataatga cacccattag 4620
   ctctaaatcg taccattaat caaatacaga tcaattcgat acaatcagtt caaataagaa 4680
   aaacgtaggg acaaagtcct ctaccaacat caaggaagac aagagtctca aaaagctcag 4740
   cctaagcaga gagaaaaaca acaacacaaa gaaagaaaag gacaagatca caaacaagaa 4800
   caaaagcaaa aacaaaaaca agaacaaaaa agggaagaaa aacaaaagta tacacaaaaa 4860
   ccaaaaaaga aaaaaggcca gagacaaaaa cggaggcaag aacaaaaatt taaacaaaaa 4920
   cagaatttaa attcataata aacaccaaga tagagacaaa aatgcaaaaa tcagagatcc 4980
   tcttcttagt atactcaagc ttgctattat cttcatcatt atgtcaaatt ccggtagaaa 5040
   aactttcaaa tgtaggggtt ataatcaatg agggcaaatt acttaaaata gcaggatctt 5100
   atgaatctag atacatagtg ttaagcttgg taccttcaat tgacctacaa gatggatgtg 5160
   gaacaactca aattattcaa tacaagaatt tattaaatag acttctaatt cctctgaagg 5220
   atgccttgga tcttcaggaa tccctgataa caataactaa tgacaccact gtgacaaatg 5280
   ataatccaca aactagattc tttggtgctg tcattggtac catagcacta ggagtagcca 5340
   cagctgctca aataactgcg ggcattgcat tagctgaagc acgagaagcc aggaaggaca 5400
   tagcactaat aaaagattcc atagtcaaga cacacaattc tgtagaactc attcaaagag 5460
   gtataggaga acagataatt gcattaaaga cattacaaga ttttgtaaat gacgagataa 5520
   gacctgcaat aggagaacta aggtgtgaga ctacggcatt gaaactaggg atcaagctca 5580
   cccaacacta ctctgaatta gcaacagcat tcagctccaa tcttgggact ataggagaaa 5640
   aaagtcttac cttgcaggca ttatcatctc tctactctgc taatataaca gaaattctaa 5700
   gtacaactaa aaaggataaa tcagatatat atgacatcat ttacactgaa caggttaagg 5760
   gaactgtgat agatgttgat ttggaaaaat acatggttac cctcttagtt aaaataccaa 5820
   ttttatcaga aataccaggc gtgttgatat acagagcttc atctatatct tataatattg 5880
   aaggagaaga atggcatgtc gcaatcccaa attacataat caataaggca tcatccttag 5940
   gaggtgcaga tgtcacaaac tgtatagaat caaaattggc atatatatgt cctagagatc 6000
   ctacacaatt aatacctgat aaccaacaga agtgtatact cggggatgta tcaaagtgcc 6060
   ctgtgactaa agtaataaac aatctagtac caaagttcgc attcatcaat ggtggtgtag 6120
   tggctaattg cattgcatcc acatgtacat gcgggacaaa cagaatacca gtgaatcaag 6180
   atcgctcaag aggagttaca ttcttgacct ataccaattg cggtttaata ggtataaatg 6240
   gaatagaact atatgccaat aaaaggggac gagacactac ttgggggaat caaatcatca 6300
   aagtgggtcc agcagtctcc attagacctg tagacatttc tttaaatctt gcatctgcca 6360
   caaatttcct agaggaatcc aagacagagc tcatgaaggc aagggcaatc atatcagcag 6420
   ttggaggatg gcacaacaca gagagtactc agataatcat gataataatt gtgtgcatac 6480
   ttataataat catatgtggt atattatact atctatacag ggttagaaga ctattagtaa 6540
   tgattaattc aactcataat tcacctgtta atgcttatac tctggagtca agaatgagaa 6600
   atccctacat gggtaacaac tccaattaaa aaatcagatc aagtacattg tagcatacat 6660
   acaacaatca aatctatcca caacttcacc aatcaggtgt acaacaagta agaaaaactt 6720
   agggttaaag acaatccagt caacctataa ggcaacagca tccgattata caaacgatgg 6780
   ctgaaaaagg gaaaacaaat agttcatatt ggtctacaac ccgaaatgac aattccacgg 6840
   taaacacaca cattaataca ccagcaggaa ggacacacat ctggctactg attgcaacaa 6900
   caatgcatac agtattgtcc ttcattatca tgatcctatg cattgaccta attataaaac 6960
   aagacacttg tatgaagaca aacatcatga cagtatcctc catgaacgaa agtgccaaaa 7020
   taatcaaaga gacaatcaca gaattaatca gacaagaagt aatatcaagg accataaaca 7080
   tacaaagttc agtacaaagc gggatcccaa tattgttaaa caagcaaagc agagatctca 7140
   cacaattaat agagaagtca tgcaacagac aggaattggc tcagatatgc gaaaacacca 7200
   ttgctattca ccatgcagac ggcatatctc ctctggaccc acacgatttc tggagatgtc 7260
   ccgtagggga acccctactg agcaacaacc ccaatatctc attattacct ggaccaagtc 7320
   tactttctgg atccaccaca atttcaggat gtgttagact accttcatta tcaattggtg 7380
   atgcaatata tgcgtattca tcaaacttaa tcactcaagg atgtgcagat atagggaagt 7440
   catatcaggt tttacaatta ggttacatat ccttaaattc agatatgtat cctgatttaa 7500
   acccggtaat ttctcatacc tatgacatca acgacaacag gaaatcatgt tctgtaatag 7560
   ctgcaggaac aaggggttat cagttatgct ccttgcccac tgtgaatgag actacagact 7620
   actcgagtga aggtatagaa gatttagtat ttgacatatt agatctcaag ggaaagacca 7680
   aatctcatcg atacaaaaat gaagatataa cttttgacca tcctttttct gcaatgtatc 7740
   cgagtgtagg aagtgggata aaaattgaaa atacactcat tttcctaggg tacggtggct 7800
   taacaactcc gctccaaggc gacactaagt gtgtgataaa cagatgtacc aatgttaatc 7860
   agagtgtttg caatgatgct cttaagataa cttggctaaa gaaaagacaa gttgtcaatg 7920
   tcttaattcg tatcaataat tatttatctg ataggccaaa gattgttgtc gagacaattc 7980
   caataactca aaattactta ggtgccgaag gtaggctact taaactaggt aaaaagatct 8040
   acatatatac tagatcttca ggttggcact ccaacctgca aataggatca ttagatatca 8100
   acaaccccat gaccattaaa tgggcgcctc atgaagtcct gtctcgacca ggaaaccaag 8160
   actgcaactg gtacaacaga tgtccgagag aatgcatatc aggtgtatat actgatgcat 8220
   atccactatc tcctgatgca gtcaatgttg ctacaaccac actgtacgca aacacatcac 8280
   gtgttaatcc caccataatg tactcaaata cctcagaaat tatcaacatg ctaagactca 8340
   agaatgtaca actagaggca gcatacacta ctacatcatg tatcactcat ttcgggaagg 8400
   gctactgctt ccacattgtt gaaatcaacc aagccagcct taatacctta caacctatgt 8460
   tgttcaagac aagtatccct aaaatatgta aaatcacatc ttgagcagat caagacccaa 8520
   cactatatca attatgtgaa aaccagatat gatgtataaa aatttaaaaa caaagcatga 8580
   atagacattt atatgacaaa tagaataaga aaaacttagg gttaatgcct gcctatttgt 8640
   caaatatgga taaacaggag tcaactcaga attcctcaga catcttatat ccagaatgtc 8700
   acttgaactc tccgattgta aaaagcaaga ttgctcaact tcacgttttg ctagatatca 8760
   atcaacccta tgatttaaaa gataacagta taataaatat caccaaatac aaaatcagaa 8820
   atggaggttt atcgccccgg cagatcaaaa tcagatcgct aggcaaaatc cttaaacaag 8880
   aaattaagga tattgatcgt tacacttttg aaccttatcc gattttctca ttagagttac 8940
   tcagactgga tatcccagaa atatgtgaca aaataagatc cattttttca gtctctgata 9000
   gattaataag agaactatca tctggatttc aagaattgtg gttaaatatt cttagacaat 9060
   taggctgtgt tgaagggaaa gagggatttg actcattaaa ggatgtagat atcatcccag 9120
   atataactga taaatataat aaaaacacat ggtatcgccc attcttaaca tggtttagca 9180
   tcaaatatga tatgagatgg atgcaaaaga ataagtcggg gaaccattta gatgtctcaa 9240
   attctcacaa ttttcttgac tgtaaatcat atattttgat tatatataga gatttagtga 9300
   taataataaa taaattaaaa ttaaccggtt atgtccttac acctgaatta gtattaatgt 9360
   attgtgatgt tgtcgaagga agatggaata tgtcttcagc tggacgactc gataaaaggt 9420
   catcaaaaat aacatgtaag ggggaagaat tatgggagct tatcgactct ttatttccca 9480
   atcttggtga ggatgtatat aatattatat cactactaga acctttatca cttgctttaa 9540
   tacagttgga tgaccctgta actaatttaa aaggagcttt catgagacat gttttgactg 9600
   agctacatac aattttaata aaagataata tatacacaga ttcagaagca gacagcataa 9660
   tggaatcatt gataaagatt ttcagagaga catcaattga tgaaaaagca gaaattttct 9720
   ccttttttag aacgtttgga catcctagct tagaagcaat aactgctgcc gataaagtaa 9780
   ggacacatat gtattcctcc aaaaaaatca tactaaagac actatatgag tgtcatgcaa 9840
   tcttctgtgc aattataata aacggatata gagaaagaca cggtggtcaa tggccgccat 9900
   gcgaattccc caatcatgta tgtcttgaac tcaagaatgc acaaggatcc aactctgcaa 9960
   tttcgtatga atgtgccgta gacaattata gtagttttat aggatttaaa tttttaaaat 10020
   ttattgagcc tcaattagat gaagatttga caatttatat gaaggataag gctctatcac 10080
   ctaggaaagc agcatgggat tcagtatatc ccgacagtaa tttatattac aaagtccctg 10140
   aatcagaaga gactcgtagg ttaatcgagg tttttataaa tgataataat tttaaccctg 10200
   cggatattat taattatgta gagtcaggag aatggttaaa tgacgatagc ttcaacatat 10260
   cttacagtct caaagaaaaa gaaattaaac aagagggtcg actctttgcc aagatgacat 10320
   ataagatgag agcagtccag gtattagcag aaacactact agcaaaagga gtaggtgagt 10380
   tattcagtga aaatgggatg gtaaagggag aaattgacct actaaagaga ctgactacat 10440
   tatctgtctc aggtgttcca agatccaact cagtttacaa taatcccata ttacatgaga 10500
   aattgatcaa aaatatgaat aagtgcaatt caaatgggta ttgggatgaa agaaagaaat 10560
   ctaaaaatga attcaaagct gcagactcat caaccgaggg gtatgagact ctgagctgtt 10620
   ttttaaccac cgatttgaaa aaatactgtc tcaactggag atttgaaagt acagcgttgt 10680
   tcggtcaaag atgtaatgag atattcgggt ttaaaacttt ctttaactgg atgcacccta 10740
   ttctagaaaa aagtacaatt tatgtaggag atccttactg tccagtacct gatagaatgc 10800
   acaaagaact ccaagatcat gatgataccg gaatctttat ccataatcca agagggggaa 10860
   tagagggtta ttgccagaaa ttatggacac taatctctat tagtgcaatc catcttgcag 10920
   ctgttaaagt tggtgtcaga gtgtcagcaa tggtacaagg agacaatcaa gctatagcag 10980
   tgacatccag agttcctgtc acacaaacct ataagcaaaa aaagactcac gtctatgaag 11040
   aaatcacaag atatttcggt gccttgagag aagttatgtt tgatattgga catgaattaa 11100
   aattaaatga gaccattata agtagcaaaa tgtttgtata cagcaaacgg atatattatg 11160
   atgggaaaat cctcccacag tgcctcaaag ctttaacaag atgtgtattt tggtcagaga 11220
   ctcttgtaga tgaaaacagg tcagcatgct caaacattgc aacatctata gccaaagcta 11280
   ttgagaatgg atattcacct atcttaggct attgtattgc tctttttaaa acttgccaac 11340
   aggtatgtat atcattagga atgaccatta atcctactat tacgtcaact atcaaagatc 11400
   aatattttaa agggaaaaat tggttaagat gtgcaatatt gatcccagct aacataggag 11460
   ggttcaacta tatgtctaca gctagatgtt ttgtcagaaa tataggtgat ccagcagttg 11520
   cagctctagc agacttaaag agattcatca aagcaggtct gttagataaa caggtattat 11580
   atcgtgtgat gaatcaagaa ccaggagact caagcttctt agattgggca tcagaccctt 11640
   attcatgcaa tctcccacac tcacaaagta taacaactat aatcaaaaat gtaacagcta 11700
   gatcagtatt gcaggaatca cctaatcctc tcctatcagg tctcttttca gaatcaagta 11760
   gtgaagaaga tctcaactta gcatcatttt tgatggatag gaaagccata ttgcccagag 11820
   tagctcacga gatcttagat aactcactta caggtgtaag agaagctata gccgggatgc 11880
   ttgatacaac gaaatctcta gtaagagcta gtgtcaggag aggaggatta tcatatagta 11940
   tcttaagaag acttataaat tatgatctat tacaatatga gaccttaaca aggacactca 12000
   gaaaaccggt taaggataat atagaatatg agtatatgtg ttcagtagaa ttggcaatag 12060
   gattgaggca aaaaatgtgg tttcatctaa cttatggaag accaatccac ggtttagaaa 12120
   ctccagaccc gttagaatta ttaagaggat catttattga aggctcagaa atatgtaaat 12180
   tttgtagatc agaagggaat aaccctatgt atacttggtt ctatcttcct gacaacatcg 12240
   acttagatac acttagcaat ggaagtcctg ccatacgtat cccttatttt ggttctgcta 12300
   ctgatgaaag atcagaggct caactaggtt atgttaagaa cttaagcaag ccggcaaaag 12360
   cagcaataag aatcgcaatg gtttacactt gggcttatgg aactgatgaa atatcatgga 12420
   tggaagcagc acttatagct caaaccaggg ctaacttaag tttagagaat ttgaagttac 12480
   tcacccctgt atcgacttct acaaatttgt cccacagatt gagagatact gctacacaga 12540
   tgaaattttc aagtgctact ttagttcgag cgagtcgatt tattaccata tctaatgata 12600
   atatggcatt aaaagaggca ggagagtcta aagatactaa tttagtttat caacaaatta 12660
   tgttaaccgg attgagctta tttgaattca atatgaggta taaacaagga tcattatcta 12720
   aacctatgat attacactta catttgaata ataaatgctg tatcatagaa tctcctcaag 12780
   aattgaatat tcctcctaga tctacattgg acttagagat cactcaggaa aataacaagt 12840
   taatctatga tcctgatcct ctcaaggaca tagatctaga gttatttagt aaggttaggg 12900
   atgtagtaca cacaattgat atgaattatt ggtctgatga tgaaataatt agagcaacta 12960
   gtatatgtac agctatgact attgcagaca caatgtctca attagataga gacaatctta 13020
   aagaaatgat agcactgata aatgatgatg atataaatag tttaatcacc gaatttatgg 13080
   ttattgatat acccttattt tgttccactt tcgggggtat tctaatcaat caatttgcat 13140
   attcacttta cgggttaaac gtcagaggga gggatgaaat atggggatat gtgatacgca 13200
   taattaaaga cacatcacat gcagtcctaa aagtactgtc caatgcatta tcacatccta 13260
   aaatattcaa acgattctgg gatgcaggag ttgtagagcc tgtttatgga cctaacttgt 13320
   ccaatcaaga caagatactg ttagccattt cagtatgtga atactctgtt gacctcttca 13380
   tgcgtgattg gcaagagggc ataccgcttg aaatatttat ttgtgataac gacccaaata 13440
   tagcagaaat gagaaaactt tcatttttag ctagacatct agcatacttg tgtagtttgg 13500
   cagagatagc taaagaggga ccaaaattgg aatctatgac atctctcgaa cgactcgaat 13560
   cattgaaaga gtatctagaa cttacttttt tagacgatcc tatattaaga tatagtcaat 13620
   tgacaggctt agttattaag atattccctt caacgttaac ttacatcagg aaatcttcaa 13680
   ttaaggtgtt gagagtaaga ggtataggga taccagaagt cttagaggac tgggatcctg 13740
   atgccgatag tatgctacta gataatataa ctgctgaggt tcaacacaat atacctttaa 13800
   agaagaacga aagaactccc ttctgggggt taagggtatc aaaatcacaa gttctgcgac 13860
   ttagaggtta tgaagagata aaaagggaag aaagaggaag atcaggtgta ggattaactc 13920
   taccttttga tgggcgatat ttatcacacc aattgagact tttcgggatt aatagcacca 13980
   gttgtttgaa agcattggaa cttacctatt tactgaatcc tctagtcaat aaggataaag 14040
   atagattata tctcggagaa ggtgcaggtg caatgctgtc ttgttatgat gctacattag 14100
   gaccctgcat gaactattat aattcaggtg ttaattcttg tgatctcaac ggacaaagag 14160
   aattaaatat ttatccttca gaagtggcac tggtagggaa gaaattgaat aatgtcacga 14220
   gtttatgtca aagagttaag gttttattca atgggaatcc tggatcaact tggataggga 14280
   atgatgaatg tgaaacacta atctggaatg aattacagaa taattcaata gggtttattc 14340
   attgtgacat ggaaggtgga gaacacaaat gtgatcaggt ggtcttacat gaacattata 14400
   gtgtgatcag gattgcatac cttgttgggg ataaggacgt tatcttagta agcaaaattg 14460
   caccaagatt aggtacagac tggacaaaac aattaagttt gtatttaaga tactggagag 14520
   atgtcagctt aatagtgttg aaaacatcta acccagcctc tacagaaatg tatctgatat 14580
   caaaagatcc taaatctgat attatagagg atagtaatac agtattggca aaccttcttc 14640
   cattatctaa agaggatagt attaagatag aaaaatggat tctagttgag aaagccaaag 14700
   ttcatgattg gatagttaga gaattaaagg aagggagtgc atcgtcaggt atgctaagac 14760
   cttaccatca agcattacaa atcttcggat ttgagcctaa tttaaacaaa ttatgtagag 14820
   atttcttatc tacactaaat atagtagaca caaaaaattg tattatcaca tttgatagag 14880
   tattaagaga tacaatcttt gagtggactc ggataaaaga cgcagataag aagctaagac 14940
   ttacaggtaa atatgatcta tatcctctta gagattcagg taagttaaaa gttatttcta 15000
   gaaggcttgt aatatcttgg atagcattgt ctatgtctac aagactagta acagggtcat 15060
   ttccagacat taaatttgaa tcaagactcc aattaggtat agtatcaata tcctctcgtg 15120
   aaatcaaaaa tcttagggtt atatcaaaga ttgtcattga caaatttgaa gatattatac 15180
   atagtgtgac ctataggttc ttgactaaag aaataaaaat attgatgaaa attttgggag 15240
   cagtcaaatt atttggggca agacagagca catctgctga tatcactaat atcgatacat 15300
   cggactccat acaatgatct tatatcttct catctttatt atctaatttg tttaaagaga 15360
   tgagttaaca agataagaaa tccctttaac tgactcataa aaacatagta agaaaaactt 15420
   acaacagaca agagtattaa taatatatcg atatttctta aactcttgtc tggt 15474
<210> 7
   <211> 49
   <212> DNA
   <213> Human parainfluenza virus
<400> 7
   aagaatcact ctcagatgtc atcggactcc ttgacgtcgt cttatccta 49
<210> 8
   <211> 49
   <212> DNA
   <213> Human parainfluenza virus
<400> 8
   taggataaga cgacgtcaag gagtccgatg acatctgaga gtgattctt 49
<210> 9
   <211> 49
   <212> DNA
   <213> Artificial Sequence
<220>
   <223> Description of Artificial Sequence: Synthetic oligonucleotide primer derived from Human parainfluenza virus
<400> 9
   aagaatcact ctcagctgtc atcggactcc ttgtcgtcgt cttatccta 49
<210> 10
   <211> 49
   <212> DNA
   <213> Artificial Sequence
<220>
   <223> Description of Artificial Sequence: Synthetic oligonucleotide primer derived from Human parainfluenza virus
<400> 10
   taggataaga cgacgacaag gagtccgatg acagctgaga gtgattctt 49
<210> 11
   <211> 40
   <212> DNA
   <213> Artificial Sequence
<220>
   <223> Description of Artificial Sequence: Synthetic oligonucleotide primer derived from Human parainfluenza virus
<400> 11
   aacttagggt gaatgacaat tcacagatca gctcaaccag 40
<210> 12
   <211> 40
   <212> DNA
   <213> Artificial Sequence
<220>
   <223> Description of Artificial Sequence: Synthetic oligonucleotide primer derived from Human parainfluenza virus
<400> 12
   ttaattcttt ttgaatccca atttcgggtt gtacctagtc 40
<210> 13
   <211> 39
   <212> DNA
   <213> Human parainfluenza virus
<400> 13
   ccttcccgag tggattagga tgcctagttt tttgagagg 39
<210> 14
   <211> 39
   <212> DNA
   <213> Human parainfluenza virus
<400> 14
   cctctcaaaa aactaggcat cctaatccac tcgggaagg 39
<210> 15
   <211> 39
   <212> DNA
   <213> Artificial Sequence
<220>
   <223> Description of Artificial Sequence: Synthetic oligonucleotide primer derived from Human parainfluenza virus
<400> 15
   ccttcccgag tggattaggc tgcctagttt tttgagagg 39
<210> 16
   <211> 39
   <212> DNA
   <213> Artificial Sequence
<220>
   <223> Description of Artificial Sequence: Synthetic oligonucleotide primer derived from Human parainfluenza virus
<400> 16
   cctctcaaaa aactaggcag cctaatccac tcgggaagg 39
<210> 17
   <211> 40
   <212> DNA
   <213> Artificial Sequence
<220>
   <223> Description of Artificial Sequence: Synthetic oligonucleotide primer derived from Human parainfluenza virus
<400> 17
   aacttagggt gaatgacaat tcacagatca gctcaaccag 40
<210> 18
   <211> 40
   <212> DNA
   <213> Artificial Sequence
<220>
   <223> Description of Artificial Sequence: Synthetic oligonucleotide primer derived from Human parainfluenza virus
<400> 18
   ttaattcttt ttgaatccca atttcgggtt gtacctagtc 40
<210> 19
   <211> 96
   <212> DNA
   <213> Human parainfluenza virus
<400> 19
   ctcaaccagg gcgcgccagc atacacgaaa ccaaccttca cagtggatac ctcagcatcc     60
   aaaactctcc ttcccgagtg gattaggatg cctagt                               96
<210> 20
   <211> 33
   <212> DNA
   <213> Human parainfluenza virus
<400> 20
   accaaagcac atacgaccgc gcgggaccaa ctc 33
<210> 21
   <211> 15
   <212> DNA
   <213> Human parainfluenza virus
<400> 21
   tgatccgtag gatta 15
<210> 22
   <211> 48
   <212> DNA
   <213> Artificial Sequence
<220>
   <223> Description of Artificial Sequence: Synthetic oligonucleotide primer derived from Human parainfluenza virus
<400> 22
   ctcaaccagg gcgcgccagc atacacgaaa ccaattagga tgcctagt 48
<210> 23
   <211> 40
   <212> DNA
   <213> Artificial Sequence
<220>
   <223> Description of Artificial Sequence: Synthetic oligonucleotide primer derived from Human parainfluenza virus
<400> 23
   ttaattcttt ttgaatccca atttcgggtt gtacctagtc 40
<210> 24
   <211> 54
   <212> DNA
   <213> Human parainfluenza virus
<400> 24
   acgaaaccaa ccttcacagt ggatacctca gcatccaaaa ctctccttcc cgag 54
<210> 25
   <211> 18
   <212> DNA
   <213> Human parainfluenza virus
<400> 25
   acacttccaa ccaaagca 18
<210> 26
   <211> 24
   <212> DNA
   <213> Human parainfluenza virus
<400> 26
   gagcccttcc tctcaaaacc tacg 24
<210> 27
   <211> 42
   <212> DNA
   <213> Artificial Sequence
<220>
   <223> Description of Artificial Sequence: Synthetic oligonucleotide primer derived from Human parainfluenza virus
<400> 27
   acgaaaccaa ccttcacagc atccaaaact ctccttcccg ag 42
<210> 28
   <211> 42
   <212> DNA
   <213> Artificial Sequence
<220>
   <223> Description of Artificial Sequence: Synthetic oligonucleotide primer derived from Human parainfluenza virus
<400> 28
   tgctttggtt ggaagtgtcg taggttttga gaggaagggc tc 42
<210> 29
   <211> 40
   <212> DNA
   <213> Artificial Sequence
<220>
   <223> Description of Artificial Sequence: Synthetic oligonucleotide primer derived from Human parainfluenza virus
<400> 29
   aacttagggt gaatgacaat tcacagatca gctcaaccag 40
<210> 30
   <211> 40
   <212> DNA
   <213> Artificial Sequence
<220>
   <223> Description of Artificial Sequence: Synthetic oligonucleotide primer derived from Human parainfluenza virus
<400> 30
   ttaattcttt ttgaatccca atttcgggtt gtacctagtc 40
<210> 31
   <211> 170
   <212> DNA
   <213> Human parainfluenza virus
<400> 31
   ggcgcgccag catacacgaa accaaccttc acagtggata cctcagcatc caaaactctc     60
   cttcccgagt ggattaggat gcctagtttt ttgagaggga tcctgaagcc gaaggagagg    120
   caccacgaaa acaagaatca ctctcagatg tcatcggact ccttgacgtc               170
<210> 32
   <211> 30
   <212> DNA
   <213> Human parainfluenza virus
<400> 32
   ctgcagttcc tcaggctact gtagactctc 30
<210> 33
   <211> 38
   <212> DNA
   <213> Artificial Sequence
<220>
   <223> Description of Artificial Sequence: Synthetic oligonucleotide primer derived from Human parainfluenza virus
<400> 33
   ggcgcgccct ctcagatgtc atcggactcc ttgacgtc 38
<210> 34
   <211> 38
   <212> DNA
   <213> Artificial Sequence
<220>
   <223> Description of Artificial Sequence: Synthetic oligonucleotide primer derived from Human parainfluenza virus
<400> 34
   gacgtcaagg agtccgatga catctgagag ggcgcgcc 38
<210> 35
   <211> 36
   <212> DNA
   <213> Artificial Sequence
<220>
   <223> Description of Artificial Sequence: Synthetic oligonucleotide primer derived from Human parainfluenza virus
<400> 35
   gcgcgccctc tcagatgtca tcggactcct tgacgt 36
<210> 36
   <211> 27
   <212> DNA
   <213> Artificial Sequence
<220>
   <223> Description of Artificial Sequence: Synthetic oligonucleotide primer derived from Human parainfluenza virus
<400> 36
   caaggagtcc gatgacatct gagaggg 27
<210> 37
   <211> 48
   <212> DNA
   <213> Human parainfluenza virus
<400> 37
   agggcgcgcc ctctcagatg tcatcggact ccttgacgtc gtcttatc 48
<210> 38
   <211> 48
   <212> DNA
   <213> Human parainfluenza virus
<400> 38
   gataagacga cgtcaaggag tccgatgaca tctgagaggg cgcgccct 48
<210> 39
   <211> 48
   <212> DNA
   <213> Artificial Sequence
<220>
   <223> Description of Artificial Sequence: Synthetic oligonucleotide primer derived from Human parainfluenza virus
<400> 39
   agggcgcgcc ctctaagatg tcatcggact ccttgtcgtc gtcttatc 48
<210> 40
   <211> 48
   <212> DNA
   <213> Artificial Sequence
<220>
   <223> Description of Artificial Sequence: Synthetic oligonucleotide primer derived from Human parainfluenza virus
<400> 40
   gataagacga cgacaaggag tccgatgaca tcttagaggg cgcgccct 48
<210> 41
   <211> 40
   <212> DNA
   <213> Artificial Sequence
<220>
   <223> Description of Artificial Sequence: Synthetic oligonucleotide primer derived from Human parainfluenza virus
<400> 41
   aacttagggt gaatgacaat tcacagatca gctcaaccag 40
<210> 42
   <211> 40
   <212> DNA
   <213> Artificial Sequence
<220>
   <223> Description of Artificial Sequence: Synthetic oligonucleotide primer derived from Human parainfluenza virus
<400> 42
   ttaattcttt ttgaatccca atttcgggtt gtacctagtc 40
<210> 43
   <211> 46
   <212> DNA
   <213> Human parainfluenza virus
<400> 43
   cgcgccctct cagatgtcat cggactcctt gacgtcgtct tatcct 46
<210> 44
   <211> 46
   <212> DNA
   <213> Human parainfluenza virus
<400> 44
   aggataagac gacgtcaagg agtccgatga catctgagag ggcgcg 46
<210> 45
   <211> 46
   <212> DNA
   <213> Artificial Sequence
<220>
   <223> Description of Artificial Sequence: Synthetic oligonucleotide primer derived from Human parainfluenza virus
<400> 45
   cgcgccctct cagctgtcat cggactccgt gatgtcgtct tatcct 46
<210> 46
   <211> 46
   <212> DNA
   <213> Artificial Sequence
<220>
   <223> Description of Artificial Sequence: Synthetic oligonucleotide primer derived from Human parainfluenza virus
<400> 46
   aggataagac gacatcacgg agtccgatga cagctgagag ggcgcg 46
<210> 47
   <211> 40
   <212> DNA
   <213> Artificial Sequence
<220>
   <223> Description of Artificial Sequence: Synthetic oligonucleotide primer derived from Human parainfluenza virus
<400> 47
   aacttagggt gaatgacaat tcacagatca gctcaaccag 40
<210> 48
   <211> 40
   <212> DNA
   <213> Artificial Sequence
<220>
   <223> Description of Artificial Sequence: Synthetic oligonucleotide primer derived from Human parainfluenza virus
<400> 48
   ttaattcttt ttgaatccca atttcgggtt gtacctagtc 40
<210> 49
   <211> 46
   <212> DNA
   <213> Human parainfluenza virus
<400> 49
   cgcgccctct cagatgtcat cggactcctt gacgtcgtct tatcct 46
<210> 50
   <211> 46
   <212> DNA
   <213> Human parainfluenza virus
<400> 50
   aggataagac gacgtcaagg agtccgatga catctgagag ggcgcg 46
<210> 51
   <211> 46
   <212> DNA
   <213> Artificial Sequence
<220>
   <223> Description of Artificial Sequence: Synthetic oligonucleotide primer derived from Human parainfluenza virus
<400> 51
   cgcgccctct cagctgtcat cggactccgt gacgtcgtct tatcct 46
<210> 52
   <211> 46
   <212> DNA
   <213> Artificial Sequence
<220>
   <223> Description of Artificial Sequence: Synthetic oligonucleotide primer derived from Human parainfluenza virus
<400> 52
   aggataagac gacgtcacgg agtccgatga cagctgagag ggcgcg 46
<210> 53
   <211> 40
   <212> DNA
   <213> Artificial Sequence
<220>
   <223> Description of Artificial Sequence: Synthetic oligonucleotide primer derived from Human parainfluenza virus
<400> 53
   aacttagggt gaatgacaat tcacagatca gctcaaccag 40
<210> 54
   <211> 40
   <212> DNA
   <213> Artificial Sequence
<220>
   <223> Description of Artificial Sequence: Synthetic oligonucleotide primer derived from Human parainfluenza virus
<400> 54
   ctgatccatg ttgggcttta accctaagtt tttcttaatt 40
<210> 55
   <211> 41
   <212> DNA
   <213> Artificial Sequence
<220>
   <223> Description of Artificial Sequence: Synthetic oligonucleotide primer derived from Human parainfluenza virus
<400> 55
   accttcacag tggatacccc agcatccaaa actctccttc c 41
<210> 56
   <211> 41
   <212> DNA
   <213> Artificial Sequence
<220>
   <223> Description of Artificial Sequence: Synthetic oligonucleotide primer derived from Human parainfluenza virus
<400> 56
   ggaaggagag ttttggatgc tggggtatcc actgtgaagg t 41
<210> 57
   <211> 25
   <212> DNA
   <213> Artificial Sequence
<220>
   <223> Description of Artificial Sequence: Synthetic oligonucleotide primer derived from Human parainfluenza virus
<400> 57
   ctgctgatcg ggacaacaag aactg 25
<210> 58
   <211> 40
   <212> DNA
   <213> Artificial Sequence
<220>
   <223> Description of Artificial Sequence: Synthetic oligonucleotide primer derived from Human parainfluenza virus
<400> 58
   ttaattcttt ttgaatccca atttcgggtt gtacctagtc 40